# EUROPEAN PATENT APPLICATION

(11) **EP 4 817 642 A1**
(43) Date of publication of application: **07.10.2026**
(21) Application number: 25168591.3
(22) Date of filing: 04.04.2025
(51) Int. Cl.: A61L 15/32, A61L 15/42, A61L 27/22, A61L 27/24, A61L 27/52

(54) **NUCLEIC ACID OR POLYPEPTIDE-BASED COMPOSITION**

(71) Applicant: Danmarks Tekniske Universitet, 2800 Kongens Lyngby (DK)
(72) Inventor: GHARIBI, Hamidreza, 2800 Kongens Lyngby (DK); DOLATSHAHI-PIROUZ, Alireza, 2800 Kongens Lyngby (DK)
(74) Representative: AWA Denmark A/S

(57) **Abstract**

A composition comprising at least two materials of materials A, B, and C; wherein
A is selected from the group consisting of nucleic acid, peptide nucleic acid, single-stranded deoxyribonucleic acid (ssDNA), double-stranded deoxyribonucleic acid (dsDNA), peptide nucleic acid (PNA), and ribonucleic acid (RNA);
B is selected from the group consisting of metalloprotein, ferritin, serum albumin such as bovine serum albumin (BSA), histone, lysozyme, fibrinogen, fibronectin, silk protein, transferrin, peptides comprising basic amino acids, chlorophyll-protein complexes, peridinin-chlorophyll-protein complex (PCP), chlorophyll a/b binding proteins, Rubisco (Ribulose-1,5-bisphosphate carboxylase/oxygenase), ATP Synthase, NADP+ Reductase, cytochrome, ferredoxin, plastocyanin (PC), photosystem proteins, ribosomal proteins, mitochondrial proteins, NADH Dehydrogenase, humanin, and MOTS-c; and
C is selected from the group consisting of collagen, aminated collagen, gelatin, aminated gelatin, poly-I-lysine, poly-histidine, poly-arginine, chitosan, aminated pectin, aminated polythylene glycol, polyethylenimine (PEI), polyacrylamide, and peptide nucleic acid (PNA);
wherein the composition comprises a complex comprising at least two of the materials of the at least two materials.

## Description

### Technical field

The present invention relates to a composition, a method of denaturing a composition, and use of the composition.

### Background Art

Pathophysiological conditions are complex and involve many abnormal physiological states. Therefore, we need to create new systems that can respond to multiple stimuli. These systems must exhibit adaptability in terms of altering their electrical, photochemical properties, rheomechanical behaviors, or molecular conformation, responding to diverse external or physiological stimuli such as reactive oxygen species (ROS), temperatures, pH, magnetic fields, light, ionic strength, and mechanical frequency. Addressing this need, our research attempts to pioneer the development of a novel class of self-healable hydrogels. These hydrogels, rooted in the synergistic combination of bioactive (macro)molecules such as hemoproteins or hemin and dynamic macromolecules such as DNA, demonstrate biocompatibility, self-healing capability, and responsiveness to a spectrum of physiological and chemical stimuli. The overarching goal is to establish a foundation for smart, biocompatible hybrid active hydrogels (HAH) that not only navigate the complexities of pathophysiological conditions but also possess injectability and flexibility, thereby opening avenues for diverse biomedical and bioelectronics manipulation with their rooted collective properties and balanced intelligence.

### Summary of the invention

It is an object of the disclosure to develop a new class of smart biocompatible hybrid hydrogels based on DNA and/ or polypeptides, for delivering dynamic hydrogel systems with self-healing ability, injectability and biosensing capacity.

It is a further object to provide a composition, such as a hydrogel, that is injectable and conductive.

It is a further object to provide a transparent composition with enhanced mechanorheologogical properties.

It is a further object to provide a composition with an increased homogeneity and isotropy in particle size and pore size.

One or more of these objects, and other objects that are apparent to the skilled person from reading the entire disclosure, are met by the various aspects disclosed.

Thus, in a first aspect, there is provided a composition comprising at least two materials of materials A, B, and C; wherein
A is selected from the group consisting of nucleic acid, peptide nucleic acid, single-stranded deoxyribonucleic acid (ssDNA), double-stranded deoxyribonucleic acid (dsDNA), peptide nucleic acid (PNA), and ribonucleic acid (RNA);
B is selected from the group consisting of metalloprotein, ferritin, serum albumin such as bovine serum albumin (BSA), histone, lysozyme, fibrinogen, fibronectin, silk protein, transferrin, peptides containing basic amino acids, chlorophyll-protein complexes, peridinin-chlorophyll-protein complex (PCP), chlorophyll a/b binding proteins, Rubisco (Ribulose-1,5-bisphosphate carboxylase/oxygenase), ATP Synthase, NADP+ Reductase, cytochrome, ferredoxin, plastocyanin (PC), photosystem proteins, ribosomal proteins, mitochondrial proteins, NADH Dehydrogenase, humanin, and MOTS-c; and
C is selected from the group consisting of collagen, aminated collagen, gelatin, aminated gelatin, poly-l-lysine, poly-histidine, poly-arginine, chitosan, aminated pectin, aminated polythylene glycol, aminated chitosan, poly(N-isopropylacryalamide (NIPAM), Polyaniline (PANI), amidine doped PEDOT:PSS, polyethylenimine (PEI), polyacrylamide, and peptide nucleic acid (PNA). The composition comprises a complex comprising at least two of the materials of the at least two materials. In other words, the complex comprises a material A and a material B; a material A and a material C; a material B and a material C; or a material A, a material B and a material C.

As disclosed herein, the group A materials comprise various purine and pyrimidine bases. In one embodiment, A comprise or consist of nucleic acid. In one embodiment, A comprise or consist of peptide nucleic acid. In one embodiment, A comprise or consist of single-stranded deoxyribonucleic acid (ssDNA) . In one embodiment, A comprise or consist of double-stranded deoxyribonucleic acid (dsDNA) . In one embodiment, A comprise or consist of peptide nucleic acid (PNA) . In one embodiment, A comprise or consist of ribonucleic acid (RNA).
As disclosed herein, the group B materials comprise proteins and peptides, each composed of various amino acids. In one embodiment, B is serum albumin, such as bovine serum albumin (BSA) . Most proteins carry an electrostatic charge and are rich in amines, enabling them to readily form intricate complexes with DNA. BSA is a 65 kDa molecule that exhibits a negative charge at physiological pH but becomes positively charged in acidic conditions, allowing it to electrostatically interact with the negatively charged phosphate groups of DNA at low pH. Since lysine, a primary amino acid in BSA, is an amine-rich molecule, lysine enables hydrogen bonding with the numerous bases of DNA and engages in electrostatic interactions with the phosphate groups in DNA. Thus it may be advantageous that material B is an amine-rich molecule, such as a lysine rich molecule. With amine-rich it is intended that B is a protein with a content of from 3 % of lysine.
BSA features a hydrophobic core that becomes accessible in its denatured form, thereby paving the way for its interaction with other proteins to form protein-protein complexes.

In one embodiment, B comprise or consist of metalloprotein. In one embodiment, B comprise or consist of ferritin. In one embodiment, B comprise or consist of histone. In one embodiment, B comprise or consist of lysozyme. In one embodiment, B comprise or consist of fibrinogen. In one embodiment, B comprise or consist of fibronectin. In one embodiment, B comprise or consist of silk protein. In one embodiment, B comprise or consist of transferrin. In one embodiment, B comprise or consist of peptides containing basic amino acids. In one embodiment, B comprise or consist of chlorophyll-protein complexes. In one embodiment, B comprise or consist of peridinin-chlorophyll-protein complex (PCP) . In one embodiment, B comprise or consist of chlorophyll a/b binding proteins. In one embodiment, B comprise or consist of Rubisco (Ribulose-1,5-bcomprise or consist of phosphate carboxylase/oxygenase) . In one embodiment, B comprise or consist of ATP Synthase. In one embodiment, B comprise or consist of NADP+ Reductase. In one embodiment, B comprise or consist of cytochrome. In one embodiment, B comprise or consist of ferredoxin. In one embodiment, B comprise or consist of plastocyanin (PC) . In one embodiment, B comprise or consist of photosystem proteins. In one embodiment, B comprise or consist of ribosomal proteins. In one embodiment, B comprise or consist of mitochondrial proteins. In one embodiment, B comprise or consist of NADH Dehydrogenase. In one embodiment, B comprise or consist of humanin. In one embodiment, B comprise or consist of MOTS-c.

As disclosed herein, the group C materials comprise functional groups that can confer positive or negative charges to the (macro)molecule at different pH levels. These (macro)molecules also possess groups capable of forming multiple hydrogen bonds, such as those found in polyelectrolyte polymers, amide backbones, or basic side chains like amino groups, guanidino groups, or imidazole groups. In one embodiment, C comprise or consist of collagen. In one embodiment, C comprise or consist of aminated collagen. In one embodiment, C comprise or consist of gelatin. In one embodiment, C comprise or consist of aminated gelatin. In one embodiment, C comprise or consist of poly-l-lysine. In one embodiment, C comprise or consist of poly-histidine. In one embodiment, C comprise or consist of poly-arginine. In one embodiment, C comprise or consist of chitosan. In one embodiment, C comprise or consist of aminated pectin. In one embodiment, C comprise or consist of aminated polythylene glycol. In one embodiment, C comprise or consist of aminated chitosan. In one embodiment, C comprise or consist of poly(N-comprise or consist of poly(N-isopropylacryalamide (NIPAM). In one embodiment, C comprise or consist of Polyaniline (PANI). In one embodiment, C comprise or consist of amidine doped PEDOT:PSS. In one embodiment, C comprise or consist of polyethylenimine (PEI) . In one embodiment, C comprise or consist of polyacrylamide. In one embodiment, C comprise or consist of peptide nucleic acid (PNA).

In one embodiment, the complex is a condensate, an aggregate, a cluster, a supramolecular assembly, a coordination compound or a network. In one embodiment, the complex is a condensate. In one embodiment, the complex is an aggregate.. In one embodiment, the complex is a cluster. In one embodiment, the complex is a supramolecular assembly. In one embodiment, the complex is a coordination compound or a network.

Various factors can influence the interactions, conformational changes, electronic structures, and the formation of complexes such as condensates and aggregates in biomolecular systems. For example, DNA-protein interactions are driven by electrostatic forces between the negatively charged phosphate backbone of DNA and positively charged amino acid residues in proteins. Hydrogen bonding and hydrophobic interactions may also play significant roles in stabilizing these interactions. These forces contribute to the formation of specific and non-specific complexes which impact processes like binding sites, catalytic and electroactivity, and their dynamicity.

Protein-protein interactions involve electrostatic forces, hydrogen bonding, and hydrophobic effects, which are crucial for the specificity and stability of these interactions. Proteins may undergo conformational changes upon binding to other proteins which affect their activity and function such as their conductivity and binding sites. These interactions can lead to the formation of condensates through phase separation which plays roles in signaling. Under stress conditions and denaturation stimuli, protein aggregation can occur that lead to the formation of insoluble fibrils or complexes which can be useful for making film.

Protein-polyelectrolyte interactions are primarily driven by electrostatic forces between charged residues and polyelectrolyte chains. Binding polyelectrolytes can alter the electronic structure of proteins which affect their reactivity and interaction dynamics. The formation of protein-polyelectrolyte complexes can lead to changes in the overall conformation of both the protein and the polyelectrolyte. These interactions can result in condensates through electrostatic interactions which impact cellular processes and material properties. Under certain conditions, protein-polyelectrolyte interactions can lead to aggregation that form larger complexes or gel networks.

When DNA and BSA are combined at pH 6, they form a complex primarily due to electrostatic interactions between the negatively charged phosphate groups of DNA and the positively charged amino acid residues of BSA in the presence of secondary interactions. If the pH is increased to 9, changes in the charge of amino acid residues or exposure of new amino acids diminish the electrostatic interactions. Consequently, secondary interactions such as hydrogen bonds and van der Waals forces become more significant. pH changes also affect the DNA double bonds, expose new nucleobase sides, and alter the dynamicity of DNA which impacts the final product interactions. For instance, the exposure of nucleobases can lead to new types of hydrogen bonding and π-π stacking that significantly alters the properties of the resulting product compared to undenatured product.

Various analytical techniques such as FTIR, DSC, TGA, XRD, UV-Vis and fluorescence spectroscopy, as well as measurements of conductivity and sensitivity, mechanical, rheological, swelling and degradation, and optical properties, are used to study the effects of environmental changes on polymers and their interactions. For example, changes in pH can significantly alter the properties of DNA-BSA systems. In the case of BSA-DNA complexes, an opaque and viscous material is formed at a certain pH, but increasing the pH transforms it into a transparent and elastic hydrogel.

In one embodiment, the at least two materials are in a denatured state. As used herein, denaturation refers to a process of partial or total alteration of the native secondary, and/or tertiary, and/or quaternary structures of proteins or nucleic acids resulting in a loss of (bio)activity. A person of skill in the art, appreciates that denaturation is a process in which proteins or nucleic acids lose the folded structure present in their native state due to various factors, including application of some external stress or compound, such as a strong acid or base, a concentrated inorganic salt, an organic solvent, agitation, radiation, or heat.

According to the present disclosure, upon denaturation, material B undergoes unfolding which exposes new amino acid residues. This exposure alters its wettability, electronic structures, binding sites, and interactions. The α-helices and β-sheets that constitute the secondary and tertiary structures are altered which lead to changes in solubility, dynamicity, mechanical, rheological properties, and biological activities. If denaturation exceeds a certain threshold, it may cause aggregation which forms larger complexes or gel networks, and crosslinking of active residues.

Denaturation of material A results in the separation of double bonds and the breaking of hydrogen bonds between base pairs which causes the double helix to unwind and separate into single strands which the extent of changes depending on the intensity of denaturation. The helical structure is altered that leads to changes in reactivity and binding sites, which subsequently affect the physical, chemical, mechanical, electrical, optical, and biological properties.

In one embodiment the composition is heat treated. The composition may be heat treated at a temperature above 50°C, such as above 60 °C, or such as above 70°C, or such as above 80°C or such as above 90 °C.

The composition may be heat treated at a temperature in the range of 50 °C to 100 °C, such as in the range of 50 to 90 °C, or such as in the range of 50 to 80 °C, or such as in the range of 50 to 70 °C, or such as in the range of 50 to 60 °C, or such as in the range of 60 to 90 °C, or such as in the range of 70 to 90 °C, or such as in the range of 80 to 90°C, or such as in the range of 60 to 80 °C.

In some embodiments, the denatured state is provided by heat treatment of the composition. Alternatively or additionally, the denatured state is provided by UV treatment of the composition. Alternatively or additionally, the denatured state is provided by elevating the pH-level of the composition.

In one embodiment, the composition has a pH of at least 6, such as a pH of at least 7, such as a pH of at least 8, such as a pH of at least 10.In one embodiment, the composition has a pH of at least 10. In one embodiment, the composition has a pH of at least 11, such as a pH of at least 12, such as a pH of at least 13, such as a pH of at least 14.

In one embodiment, the composition is in the form of a hydrogel. In one embodiment, the composition is in the form of a film. In one embodiment, the composition is in the form of a nanoparticle. In one embodiment, the composition is in the form of an aggregate. In one embodiment, the composition is in the form of a condensate. In one embodiment, the composition is in the form of a paste. In one embodiment, the composition is in the form of a nanoparticle. In one embodiment, the composition is in the form of a microparticle. In one embodiment, the composition is in the form of a film.

Hydrogels are composed of water-swollen polymer networks, that can retain up to 90% water. They are therefore highly attractive candidates for recapitulating the soft Extracellular matrix of native tissues. Since they contain water, they dissipate energy and can thus mimic the viscoelastic properties of native tissues. Recent innovations in hydrogel design, such as dynamic crosslinking, and the incorporation of bioactive motifs have significantly enhanced their ability to support complex tissue regeneration.

Water itself plays a crucial role in biological processes due to its high polarity, hydrogen bonding capability, and other unique behavior. It for instance serves as a solvent, reactant, and medium for transporting nutrients and waste products, facilitating biochemical reactions essential for life, such as hydrolysis and condensation in metabolic processes like photosynthesis and cellular respiration. Hydrogels, therefore represent a more native milieu for cells to thrive and grow within. Indeed, they can be loaded with cells, growth factors, and bioactive molecules to promote tissue repair, such as regenerating cartilage and bone tissues. And in wound dressings hydrogels can assist in maintaining a moist environment that is conducive to healing - along these lines they have also been loaded with antimicrobial agents to promote the wound healing process.

In one embodiment, the metalloprotein is selected from the group consisting of hemoglobin, myoglobin, cytochrome C, carbonic anhydrase, and superoxide dismutase. In one embodiment, the metalloprotein is hemoglobin. In one embodiment, the metalloprotein is myoglobin. In one embodiment, the metalloprotein is cytochrome C. In one embodiment, the metalloprotein is carbonic anhydrase. In one embodiment, the metalloprotein is superoxide dismutase.

In one embodiment, the peptide comprising basic amino acid comprises amino acids selected from the group consisting of Lysine, Arginine, and Histidine. The peptide may be considered rich in these amino acids. Advantages described above for amino-rich proteins are equally applicable to the present peptides.

In one embodiment, the composition comprises dsDNA and serum albumin, such as BSA. In one embodiment, the dsDNA is from fish sperm and BSA.

In one embodiment, the composition comprises dsDNA and hemoglobin. In one embodiment, the dsDNA is from fish sperm.

In one embodiment, the composition comprises material A in the range of from about 3 to about 30% w/v, and material B in the range of from about 3 to about 30% w/v. In one embodiment, the composition comprises material A in the range of from about 4 to about 25% w/v, and material B in the range of from about 4 to about 25% w/v. In one embodiment, the composition comprises material A in the range of from about 6 to about 20 % w/v, and B in the range of 6 to 20% w/v.

In one embodiment, the composition comprises PEI and serum albumin such as bovine serum albumin (BSA), and/or hemoglobin.

In one embodiment, the composition comprises PEI in the range of 1 to 10% w/v, serum albumin in the range of 3 to 30% w/v and/or hemoglobin in the range of 4 to 30% w/v. In one embodiment, the composition comprises PEI in the range of 2% w/v, serum albumin in the range of 4 to 25% w/v and/or hemoglobin in the range of 5 to 25% w/v.

In one embodiment, the complex is of uniform size distribution, such as a size distribution of from about 10 nm to about 800 nm, such as from about 10 nm to about 700 nm, such as from about 10 nm to about 70 nm, such as from about 15 nm to about 60 nm, or such as from about 200 nm to about 700 nm.

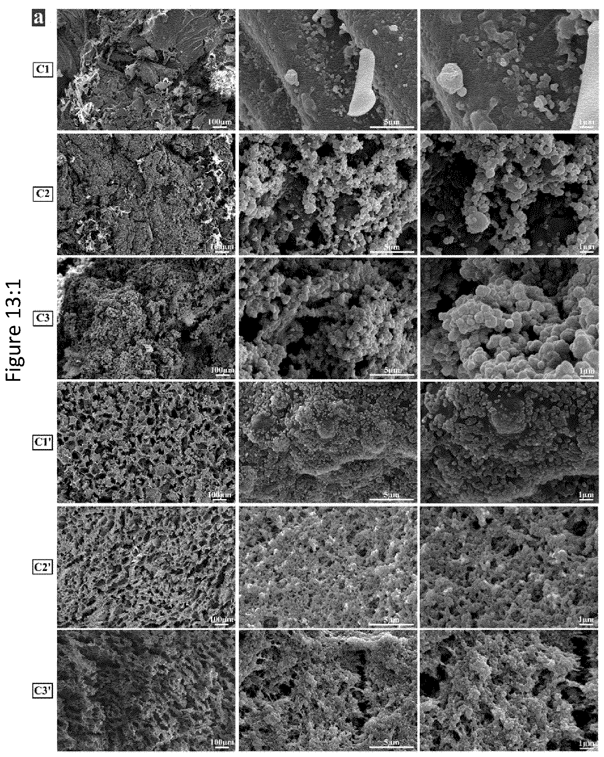

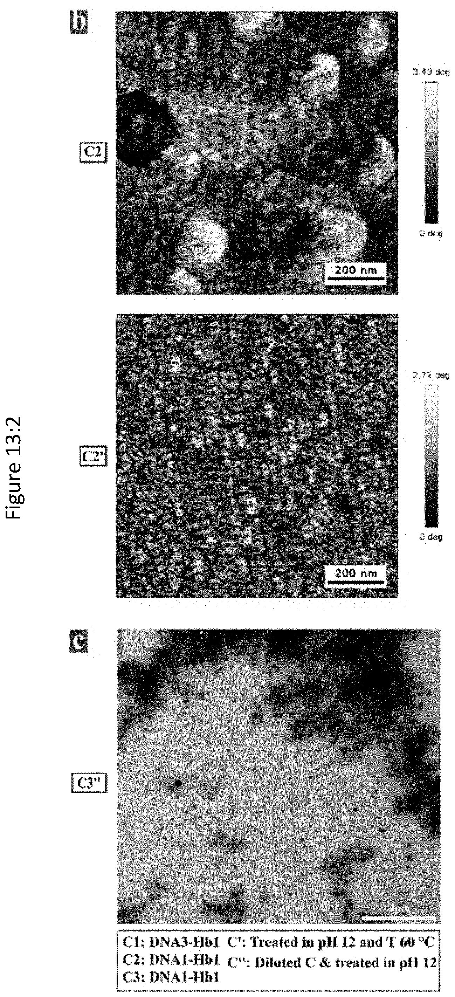

The size distribution may be measured through DLS, and also TEM, AFM and SEM shows ranges of particle sizes. Examples of ranges can be found in figure 13.

Denaturation and polymer concentration not only alter the size distribution but also affect the sizes of complexes and condensates. Additionally, the ratio between the components plays a crucial role in determining the distribution and size of the complexes and condensates.

Up to a certain extent, denaturation can result in a more uniform size distribution of condensates (e.g., at pH 9). However, if denaturation exceeds a certain limit, such as under conditions of high ionic strength or elevated pH, it disrupts the uniform size distribution. Other factors influencing the size distribution of condensates include the concentration of prepolymers in the stock solution. Diluted concentrations reduce interactions which lead to a decreased size of complexes more uniform size distribution (e.g. C3" samples (denatured and diluted solution) form 15-60 nm while 200-700 nm for C3 (undenatured and concentrated solution)).

In one embodiment, the composition is further comprising a filler selected from the group consisting of porphyrin and a porphyrin derivative, graphene, chlorophyll, tannic acid, carbon nanotube, nanozymes, metal nanoparticle, Mxene's, metal-organic framworks, metal and metal-free chromophores, covalent organic framework, metal-organic cages, nanosilicate, Laponite, Hallosite, Sumecton, Montmorillonite, and calcium orthophosphate family such as Hydroxyapatite; such as porphyrin and a porphyrin derivative, graphene, carbon nanotube, metal nanoparticle, Mxene's, metal-organic framworks, metal and metal-free chromophores, covalent organic framework, metal-organic cages, and calcium orthophosphate family such as Hydroxyapatite. The filler may comprise or essentially consist of one or more of porphyrin, porphyrin derivative, graphene, chlorophyll, tannic acid, carbon nanotube, nanozymes, metal nanoparticle, Mxene's, metal-organic framworks, metal and metal-free chromophores, covalent organic framework, metal-organic cages, nanosilicate, Laponite, Hallosite, Sumecton, Montmorillonite, or calcium orthophosphate family such as Hydroxyapatite.

In a second aspect there is provided a method of denaturing a composition according to the first aspect. The method comprising the steps of:
- providing a material A according to the first aspect and adding a base, such as a NaOH solution, to reach a pH level in the range of 10 to 14,
- adding a material B according to the first aspect,
- curing the composition at a temperature in the range of 40 to 80 °C, such as 50 to 70 °C for at least 30 minutes.

In a third aspect there is provided a method of denaturing a composition according to the first aspect, comprising the steps of:
- providing a composition according to the first aspect,
- exposing the composition to UV light in the range of 100 to 400 nm, such as in the range of 150 - 250 nm, or 250 - 350 nm, or 240 nm, or 380nm, for at least 5 minutes, such as for at least 15 minutes or at least 30 minutes.

In a fourth aspect there is provided a use of a composition according to the first aspect in tissue engineering, in stem cell differentiation, in a flexible electronic device, in a biosensor or in a woundcare patch. Tissue engineering may be of the musculoskeletal system.

### Brief description of the drawings

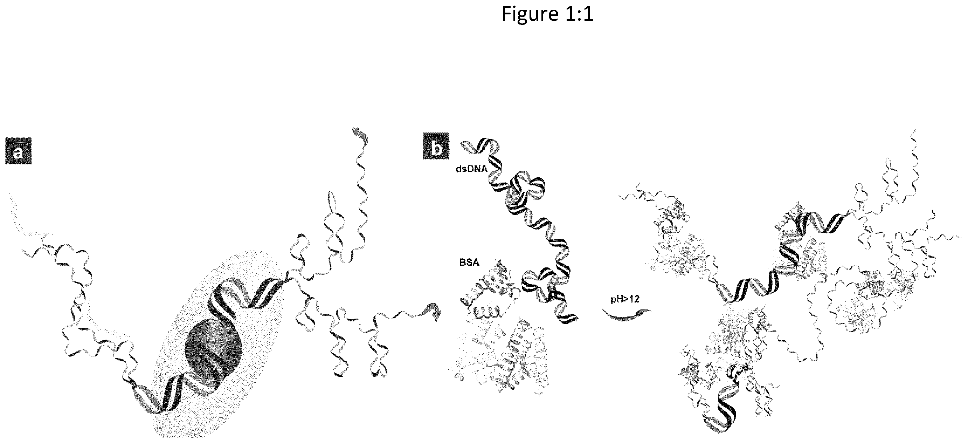

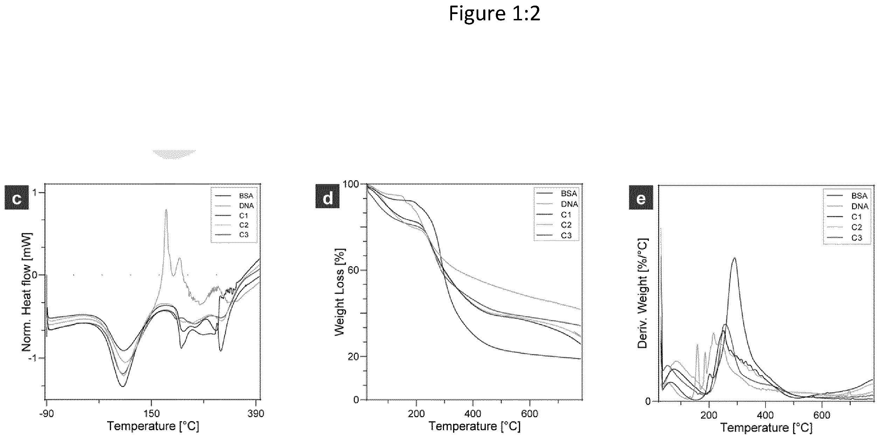

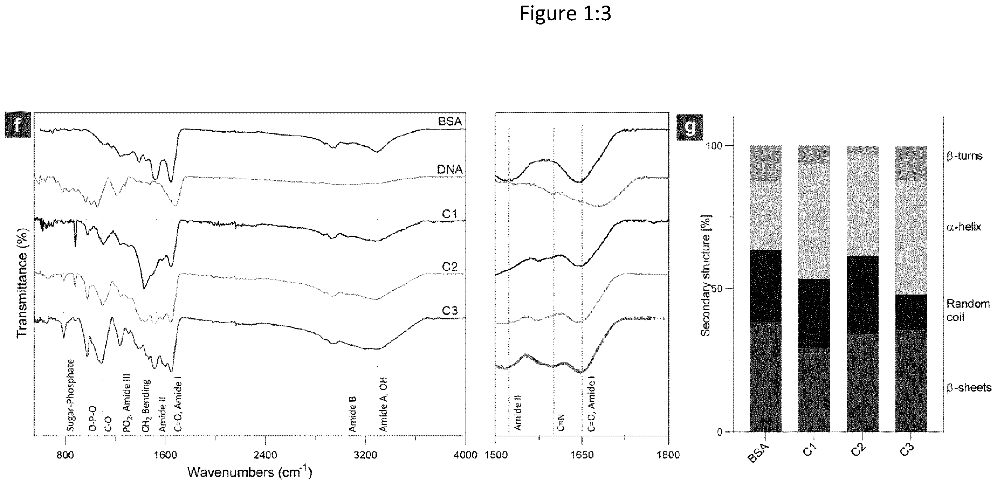

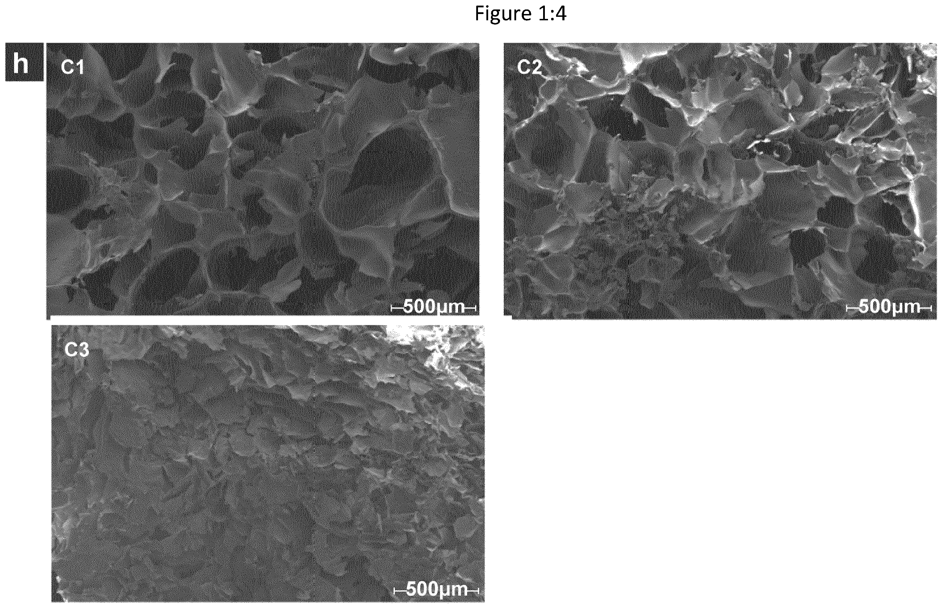

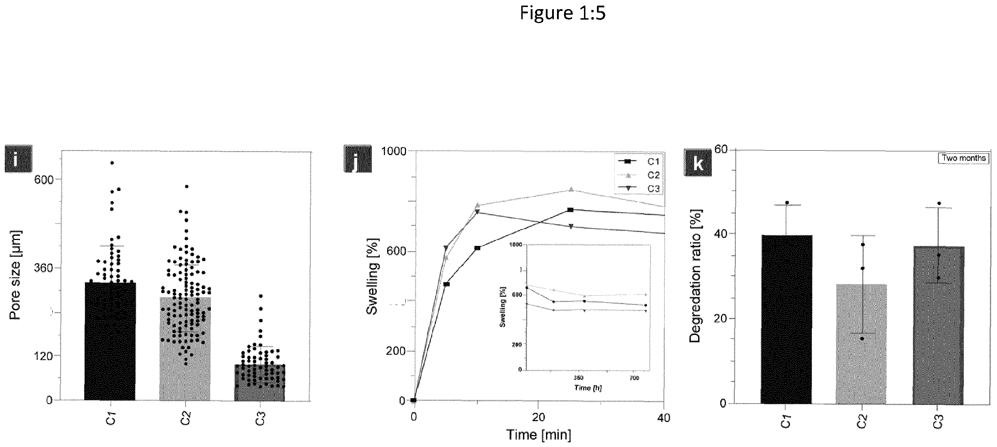

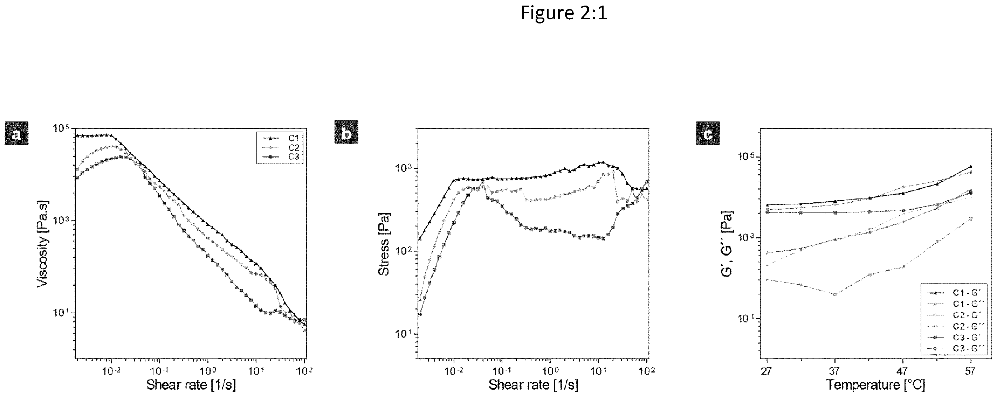

Figure 1 shows: (a) A brief depiction of the concepts behind the structure and its relation to the application, (b) The fabrication process behind the bioadaptable hydrogels systems are highlighted here, (c) Differential scanning calorimetry (DSC) analysis, (d) Thermogravimetric analysis (TGA), (e) Derivative thermogravimetry (DTG) analysis of the pure DNA and BSA as well as different combinations of hydrogels (C1: 25DNA-75BSA, C2: 35DNA-65BSA, and C3: 50DNA-50BSA), (f) FTIR characterization, (g) FTIR secondary structure characterization, (h) SEM images of different DNA-BSA ratios after swelling in PBS, (i) Pore size of the different combinations after swelling, (j) Swelling kinetics of the different combination of DNA-BSA in PBS pH 7.4 at 37 °C, and (k) Degradation ratio after 8 weeks in PBS pH 7.4 at 37 °.

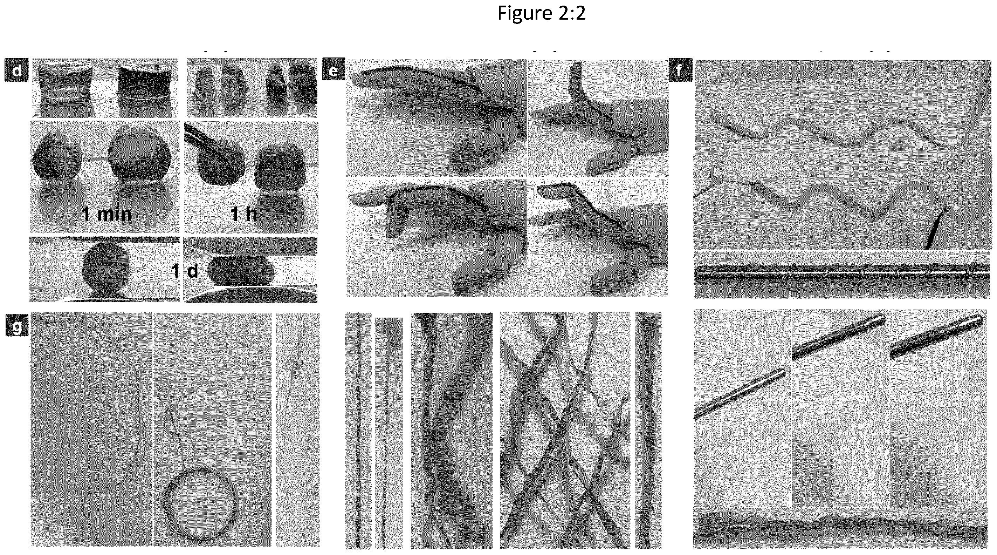

Figure 2 shows Rheology and printability demonstration. (a-c) Rheological properties: Including viscosity (a), shear stress (b) and modulus temperature sensitivity (c) as function of shear rate, (d) Self-healing and recovering images of the of 35DNA-65BSA hydrogel after cutting-reassembling, and subjecting to compression, (e) Conformability of hydrogels towards movement, (f) Injectability, (g) Conformability of hydrogel fibers towards stretching, twisting, and weaving.

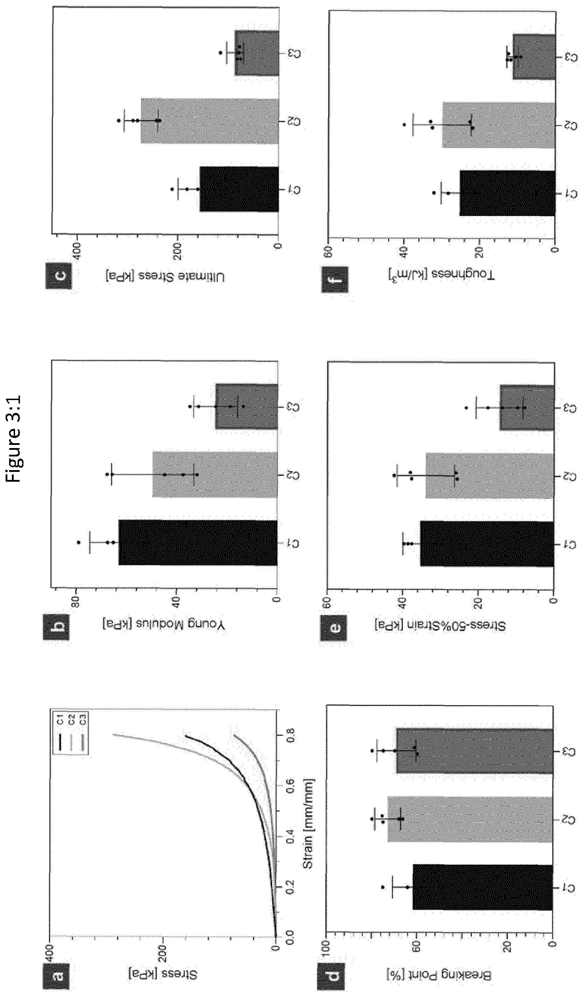

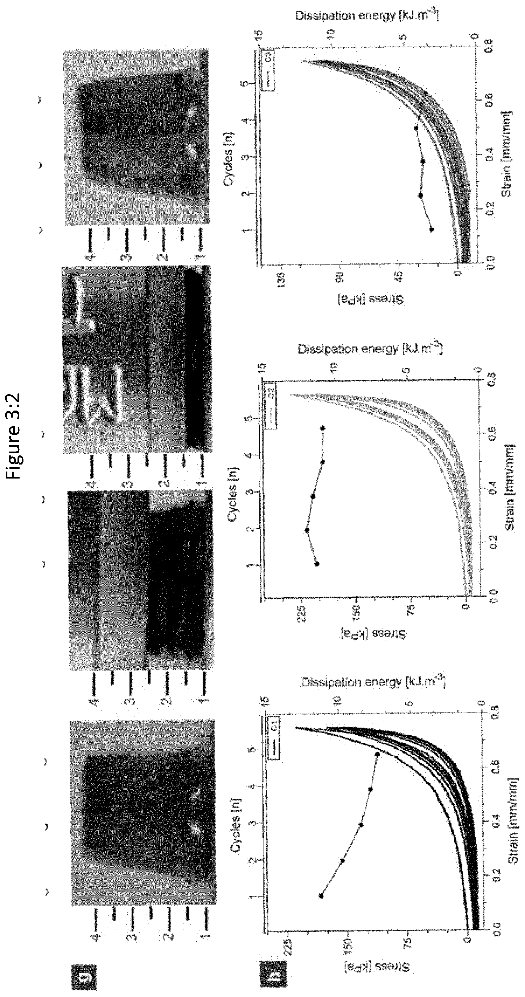

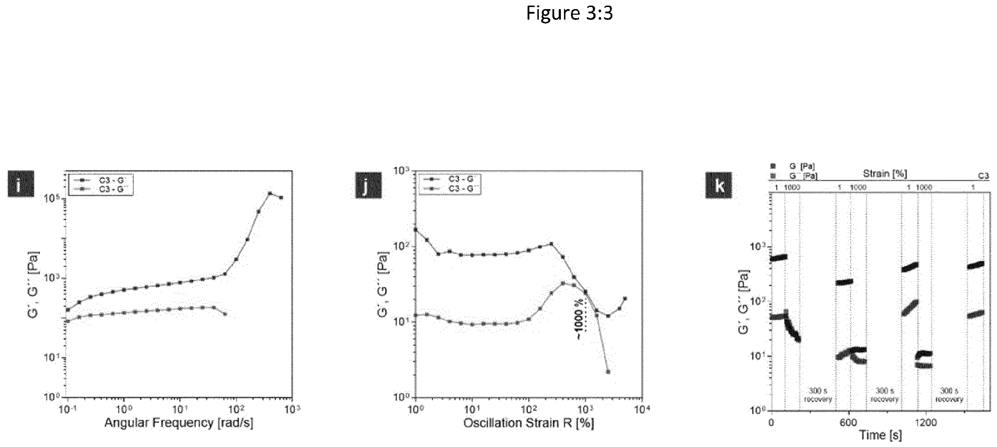

Figure 3 shows Mechanical and rheological characterization: (a) Compressive stress-strain curves for the different composites. Calculated (b) Young modulus, (c) Compressive strength, (d) Strain at break, (e) Stress at 50% strain and (f) Toughness values. (g) Images demonstrating the compressibility and recovery of 35DNA-65BSA hydrogel, (h) Cyclic mechanical test and calculated dissipated energy of the hydrogels for each cycle, (i) Dynamic frequency sweep and (j) Amplitude sweep data (storage modulus G' and loss modulus G" vs. shear strain), and (k) Dynamic cyclic strain sweep measurement for shear strain recovery properties of the 50DNA-50BSA hydrogel.

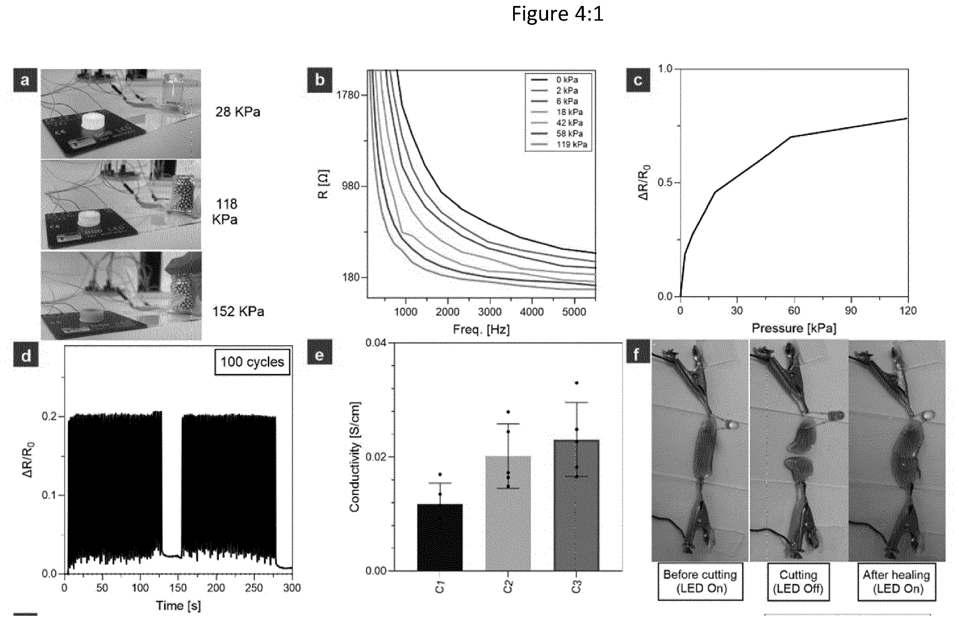

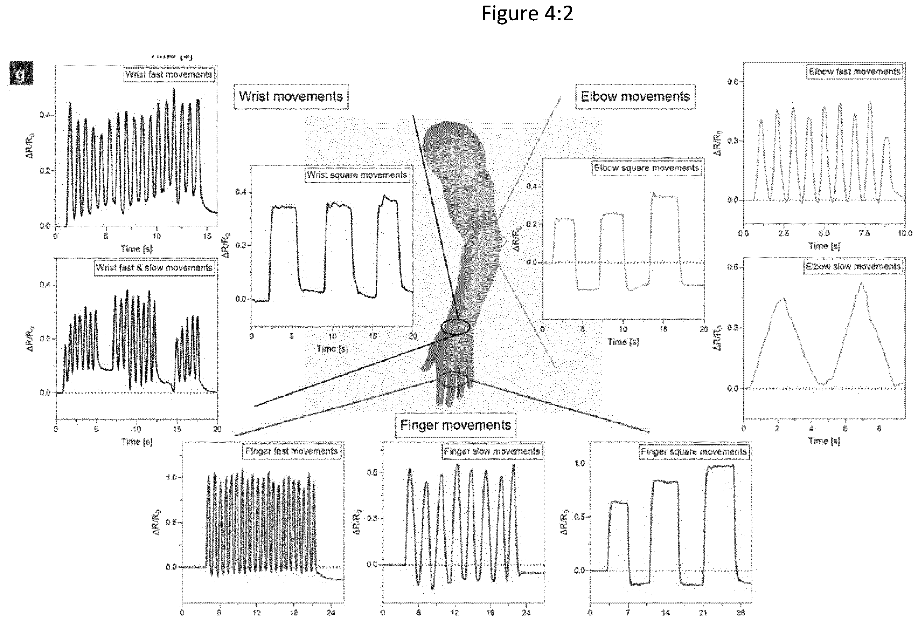

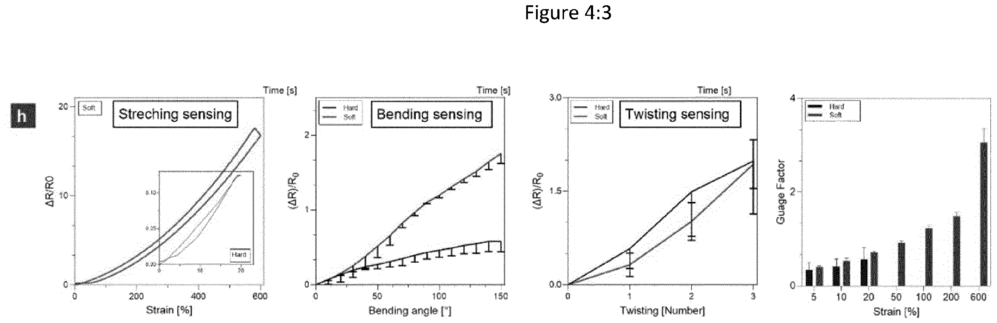

Figure 4 shows: (a) Illustration of pressure sensitivity test, (b) Impedance changes based on the compressive stress of the 35DNA-65BSA hydrogel, (c) Impedance changes based on the compressive stress in 200 kHz of the 35DNA-65BSA hydrogel, (d) Stability of the Cyclic compressive pressure sensitivity of 35DNA-65BSA hydrogel and cyclic changes of the impedance in high number of cycles, (e) Conductivities of the different combination of DNA and BSA hydrogels, (f) Electrical self-healing of the hydrogel, (g) human motion sensing, and (h) Impedance sensitivities towards stretching, bending and twisting.

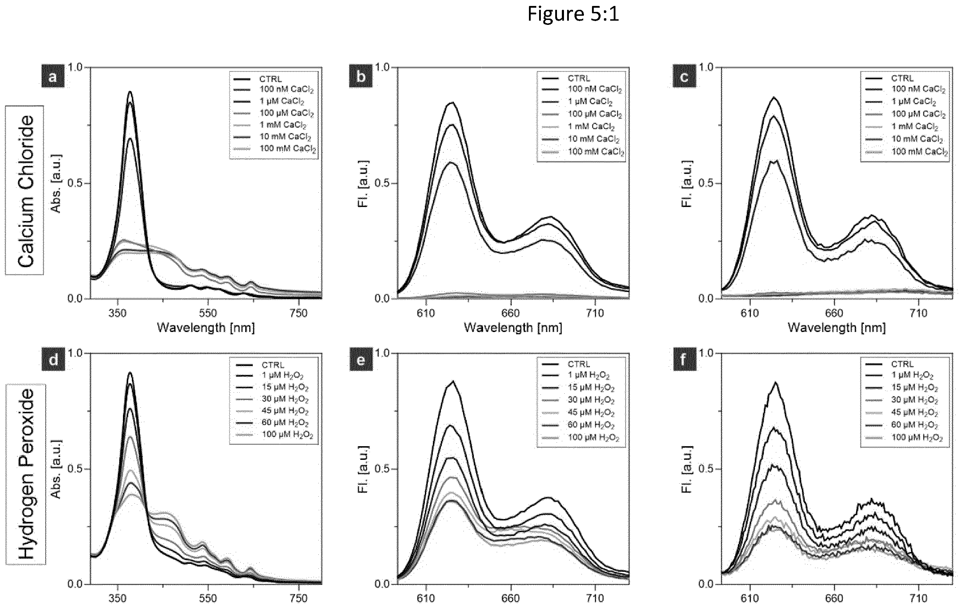

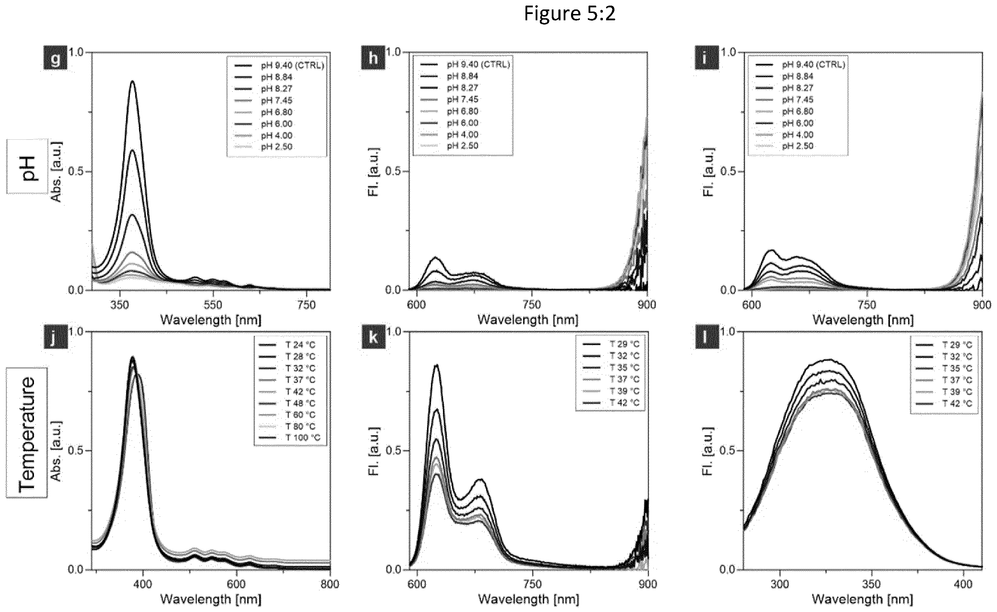

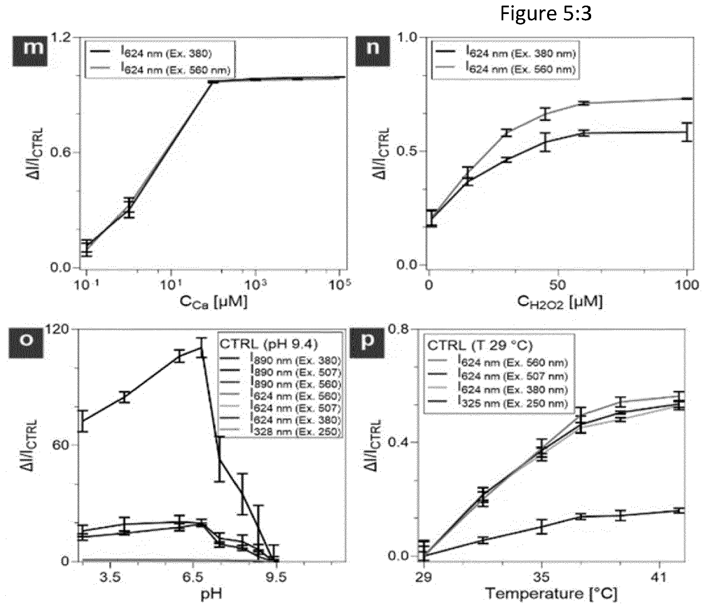

Figure 5 shows Hemin-based calcium sensing (a) UV-Vis. Spectroscopy sensitivity of the Demetallized Hemin (DHem) to Ca2+, (b) Fluorescence spectroscopy sensitivity of DHem to Ca2+ (ex. 380 nm), (c) Fluorescence spectroscopy sensitivity of DHem to Ca2+ (ex. 560 nm), (d) UV-Vis. Spectroscopy sensitivity of the DHem to H2O2, (e) Fluorescence spectroscopy sensitivity of DHem to H2O2 (ex. 380 nm), (f) Fluorescence spectroscopy sensitivity of DHem to H2O2 (ex. 560 nm), (d) UV-Vis. Spectroscopy sensitivity of the DHem to pH, (e) Fluorescence spectroscopy sensitivity of DHem to pH (ex. 380 nm), (f) Fluorescence spectroscopy sensitivity of DHem to pH (ex. 560 nm), (g) UV-Vis. Spectroscopy sensitivity of the DHem to H2O2, (h) Fluorescence spectroscopy sensitivity of DHem to H2O2 (ex. 380 nm), (i) Fluorescence spectroscopy sensitivity of DHem to H2O2 (ex. 560 nm), (j) UV-Vis. Spectroscopy sensitivity of the Demetallized Hemin (DHem) to temperature, (k) Fluorescence spectroscopy sensitivity of DHem to temperature (ex. 380 nm), (l) Fluorescence spectroscopy sensitivity of DHem to temperature (ex. 560 nm), (m) Calibration curve of UV-Vis. and Fluorescence Spectroscopy sensitivity of the DHem to Ca2+, (n) Calibration curve of UV-Vis. and Fluorescence Spectroscopy sensitivity of the DHem to H2O2, (o) Calibration curve of UV-Vis. and Fluorescence Spectroscopy sensitivity of the DHem to pH and (p) temperature.

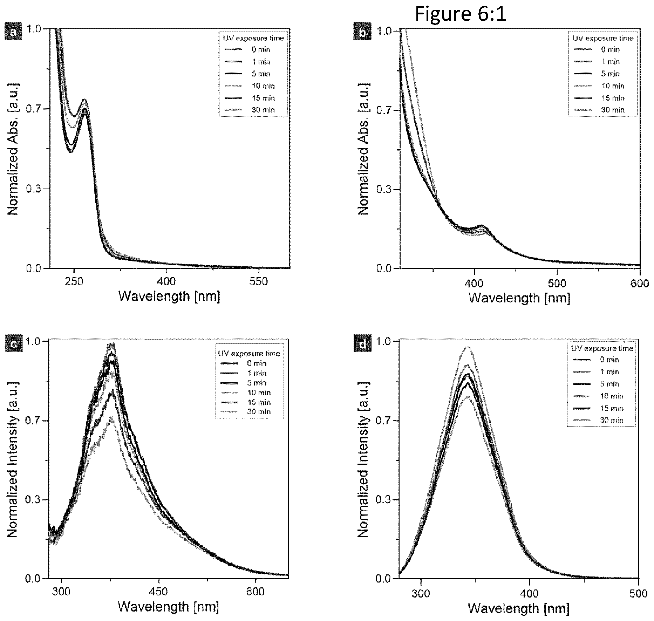

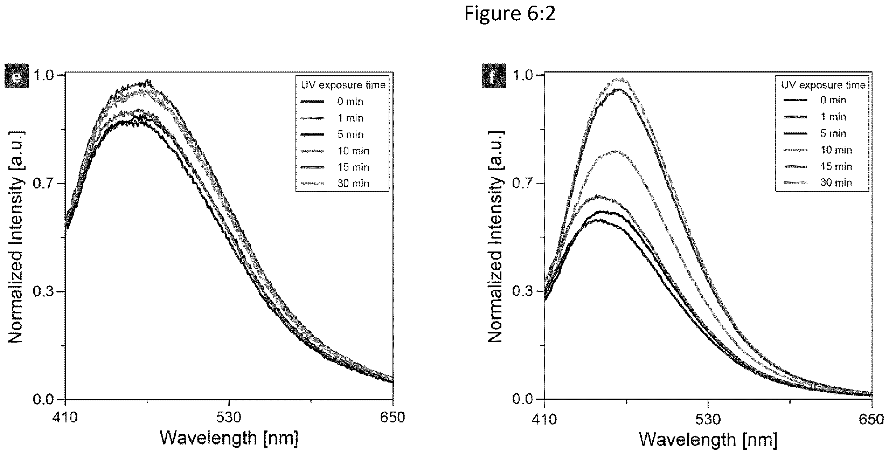

Figure 6 shows Photochemical properties upon exposure of UV: (a) UV-Vis spectroscopy of DNA in different UV exposure time, (b) UV-Vis spectroscopy of BSA in different UV exposure time, (c) Fluorescence spectroscopy of DNA in different UV exposure time (ex. 240 nm), (d) Fluorescence spectroscopy of BSA in different UV exposure time (ex. 240 nm), (e) Fluorescence spectroscopy of DNA in different UV exposure time (ex. 380 nm), (f) Fluorescence spectroscopy of BSA in different UV exposure time (ex. 380 nm).

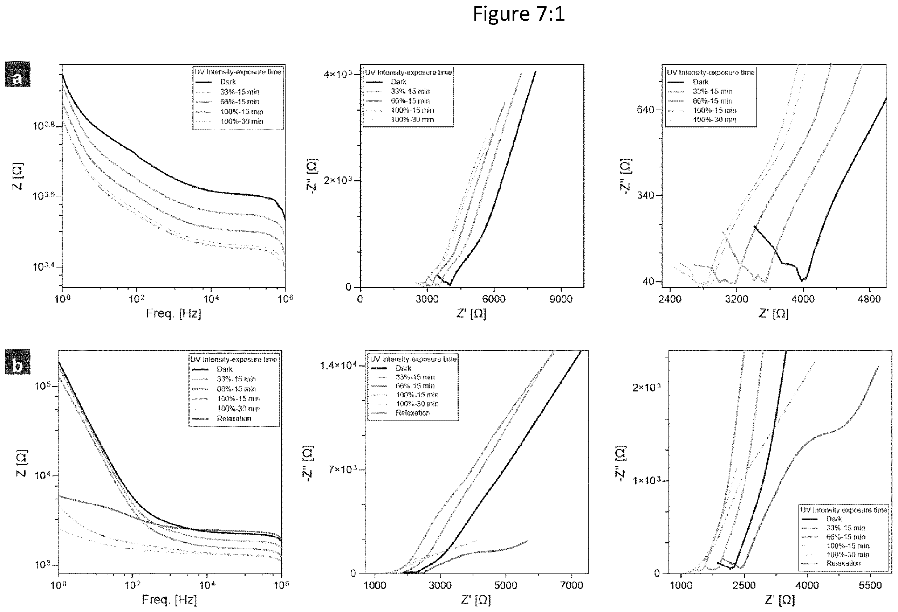

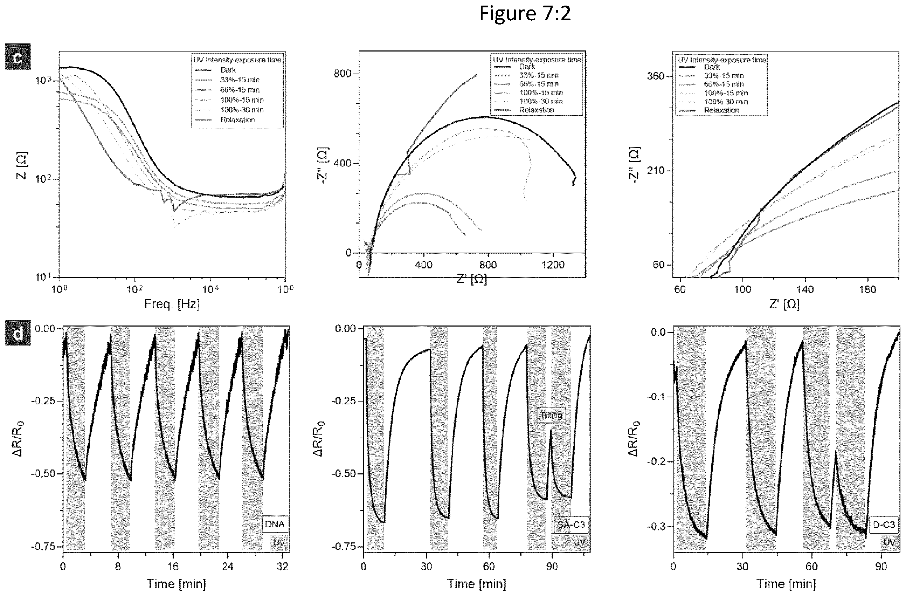

Figure 7 shows Impact of UV illumination on: (a) Impedance-Frequency and impedance Nyquist plot (Z, consisting of real, Z', and imaginary part, Z") spectra measured on DNA gel in different UV intensity percentages and UV exposure time, (b) Impedance-Frequency and impedance Nyquist plot (Z, consisting of real, Z', and imaginary part, Z") spectra measured on self-assembled DNA-BSA (SA-C3) hydrogel (1:1 ratio) in different UV intensity percentages and UV exposure time, (c) Impedance-Frequency and impedance Nyquist plot (Z, consisting of real, Z', and imaginary part, Z") spectra measured on denatured DNA-BSA (D-C3) hydrogel (1:1 ratio) in different UV intensity percentages and UV exposure time, (d) the in situ cyclic impedance measurement with on-off UV light exposure.

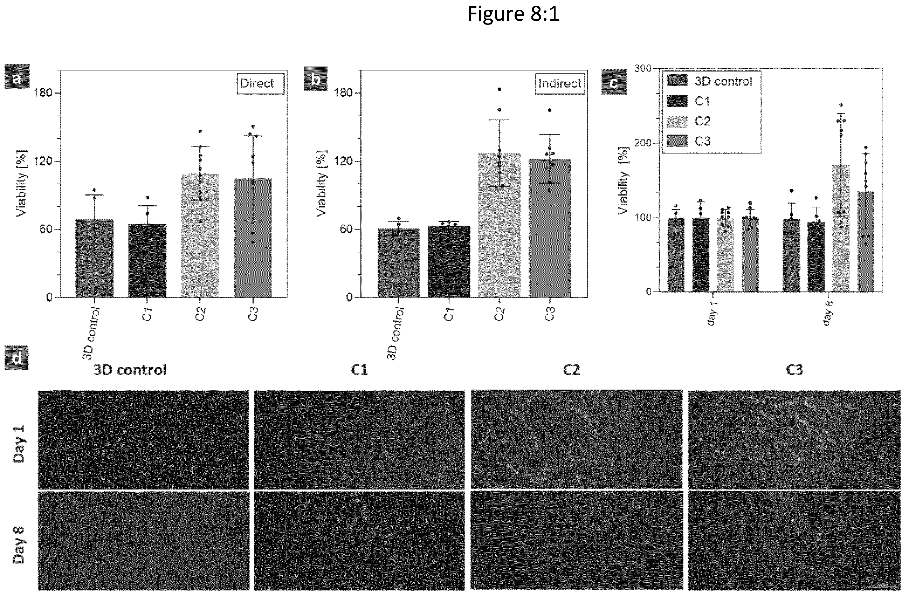

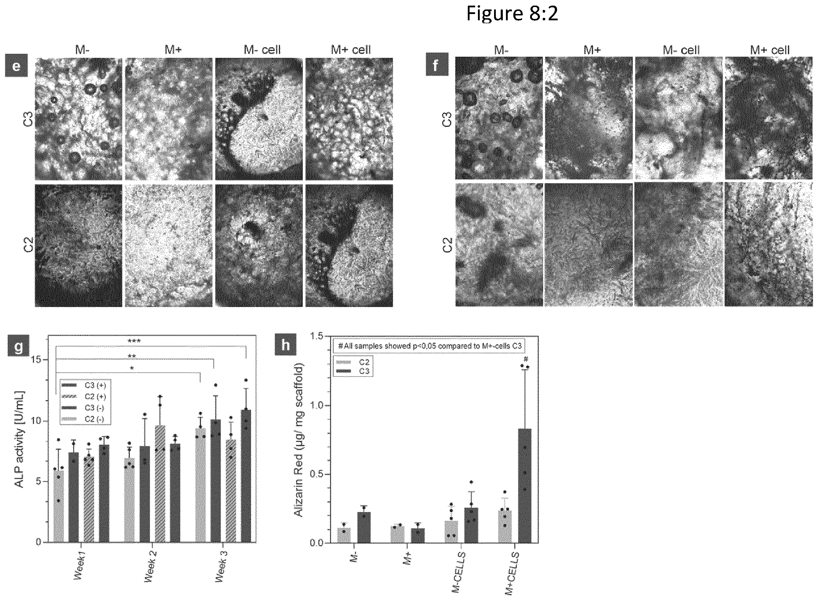

Figure 8. Cytocompatibility/biocompatibility and tissue engineering; In vitro studies: Biocompatibility towards L-929 Fibroblasts: (a) Direct and (b) Indirect; Biocompatibility towards bone marrow mesenchymal stem cells: (c) Cell viability percentage after 1 and 8 days in contact, (d) Microscope image of cell viability test after 8 days and; Osteogenic ability: (e) Quantitative results of early marker (ALP activity), (f) Microscopic images of early marker (ALP activity), (g) Microscopic images of mineralization (Alizarin Red), (h) Quantitative results of mineralization (Alizarin Red).

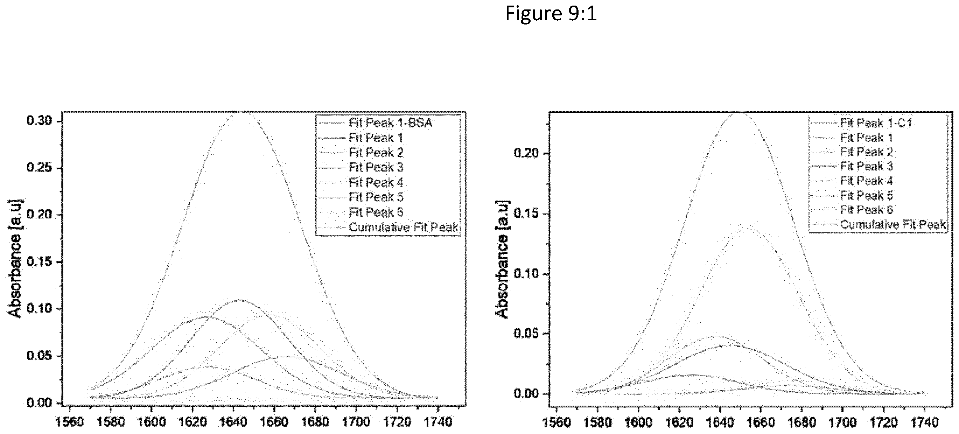

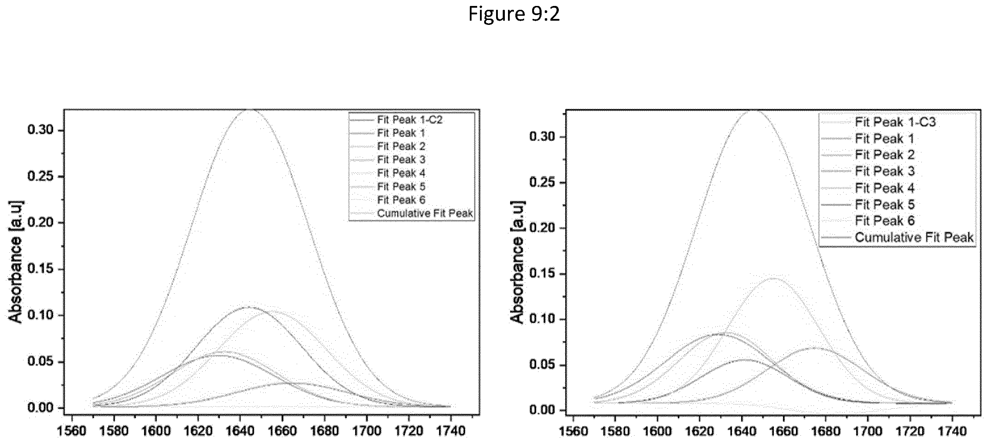

Figure 9 shows FTIR secondary structure characterization of BSA and different combinations of hydrogels (C1: 25DNA-75BSA, C2: 35DNA-65BSA, and C3: 50DNA-50BSA).

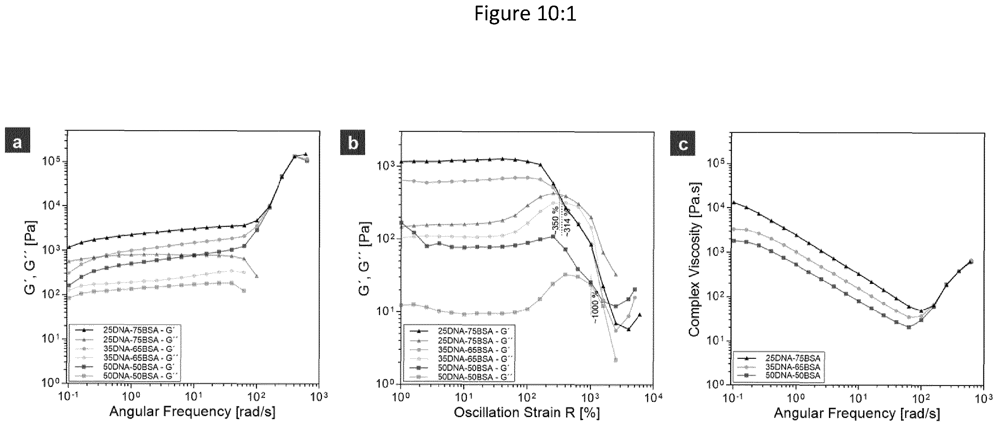

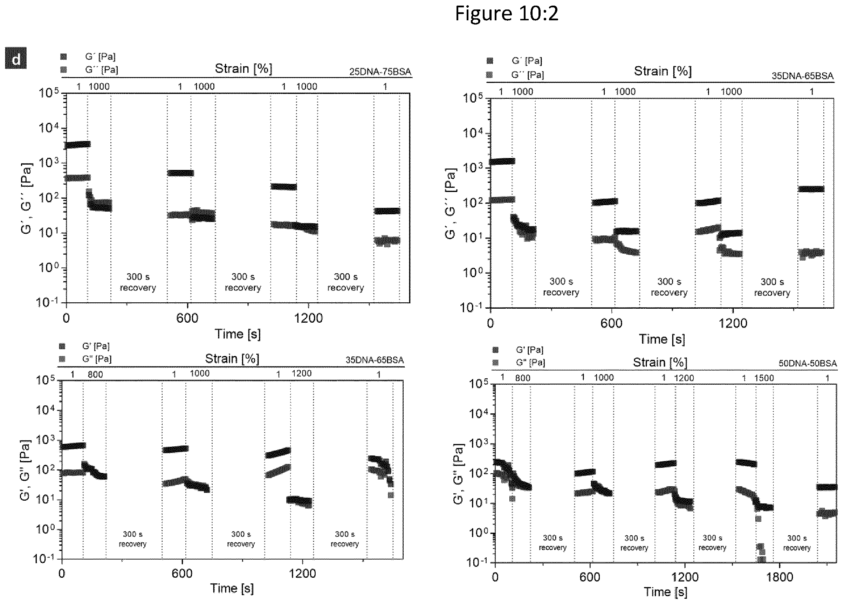

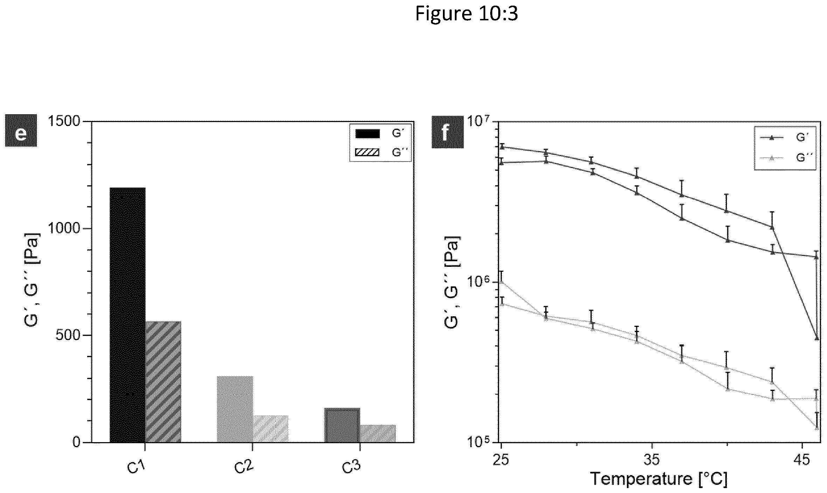

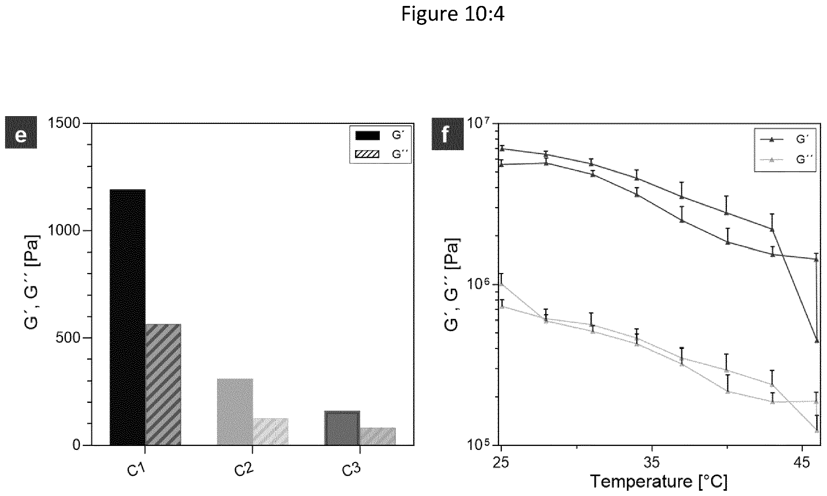

Figure 10 shows: (a) Dynamic frequency sweep, (b) Amplitude sweep data (storage modulus G' and loss modulus G" vs. shear strain), (c) Complex viscosity, (d) Dynamic cyclic strain sweep measurement for shear strain recovery properties of the hydrogels at high shear strains in differe ratios, (e) Starting point modulus, and (f) Reversible modulus changes in various temperatures of C3 dried hydrogel.

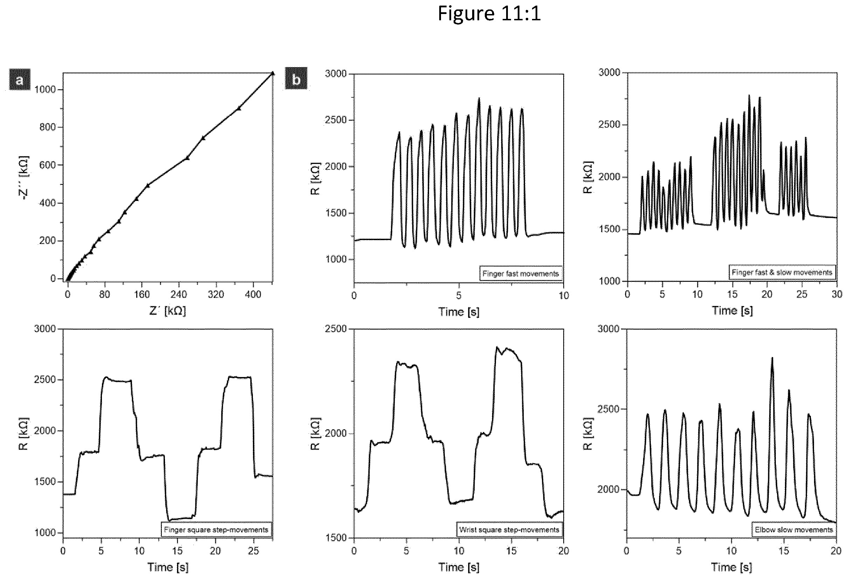

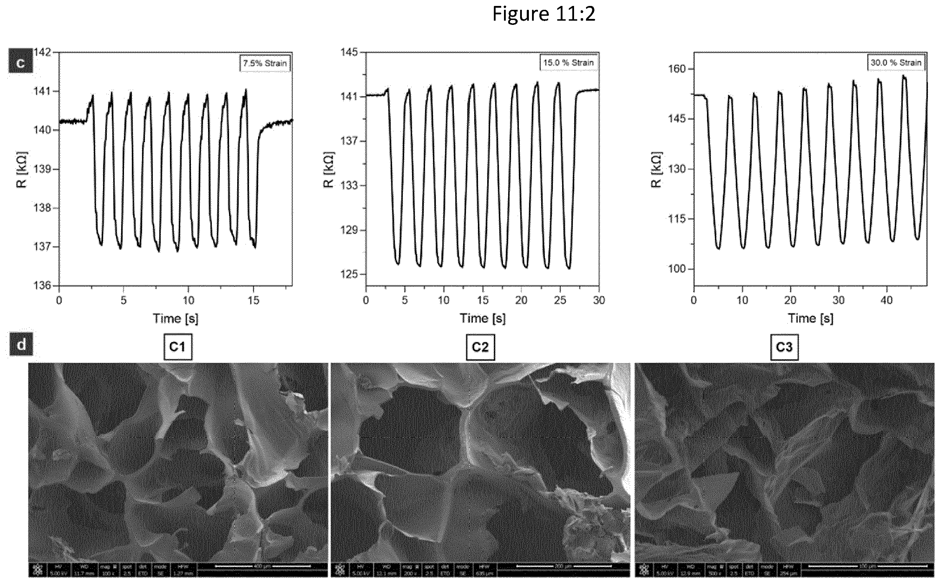

Figure 11 shows: (a) Impedance spectroscopy of DNA-BSA sample hydrogel (Imaginary vs. real part of the impedance spec.), (b) human motion sensing, (c) Cyclic compressive pressure sensitivity of DNA-BSA sample hydrogel and cyclic changes of the impedance in different strain ratio, (d) SEM images in higher magnification of different DNA-BSA ratios after swelling in PBS.

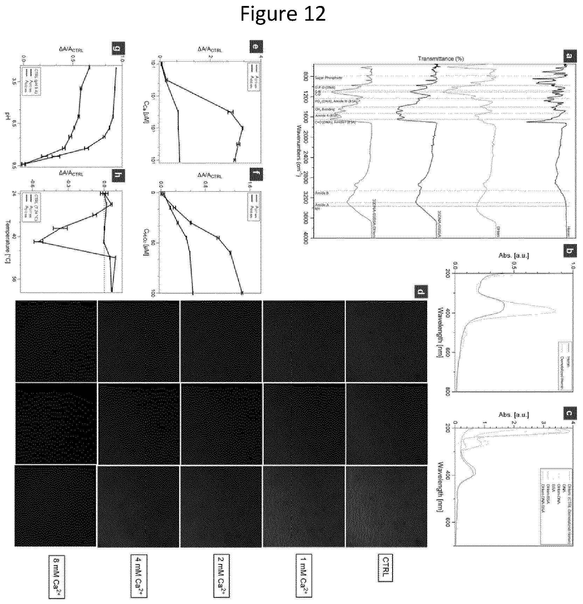

Figure 12 shows Modification of the hydrogels with demetallized Hemin: (a) FTIR characterization of Hemin, Demetallized Hemin (DHem), and DHem-BSA-DNA hydrogel, (b) UV-Vis. Spectroscopy of Hemin and Demetallized Hemin, (c) UV-Vis. Spectroscopy of different combinations of the DNA-BSA-Demetallized Hemin, (d) Fluorescence Images sensitivity of the hydrogels to H2O2, (e) Calibration curve of UV-Vis. Spectroscopy sensitivity of the DHem to Ca2+, (f) Calibration curve of UV-Vis. Spectroscopy sensitivity of the DHem to H2O2, (g) Calibration curve of UV-Vis. Spectroscopy sensitivity of the DHem to pH and (h) temperature.
Figure 13 shows (a) SEM images of different DNA-Hb weight ratios (C1: 3DNA-1Hb, C2: 1DNA-1BSA, and C3: 1DNA-3BSA) after swelling in PBS pH 7.4 and freeze-drying, (b) AFM images of C2 and C2' (fabricated in pH 12 and T 60 °C) after drying at room temperature, (c) TEM image of C2" (fabricated in pH 12 under dilution with DPBS pH 7.4).

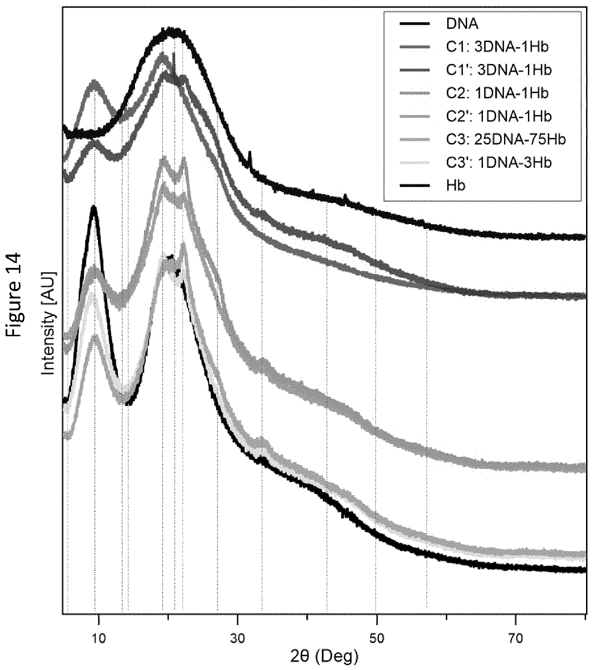

Figure 14 shows XRD of different samples after swelling and subsequent freeze-drying.

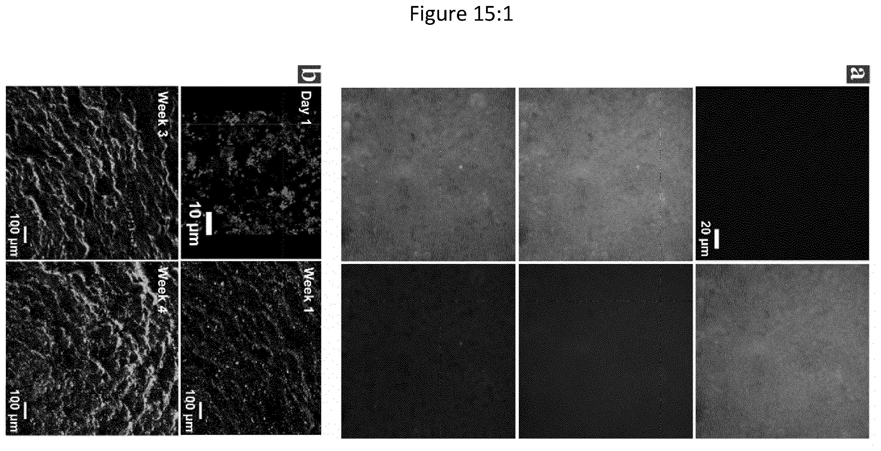

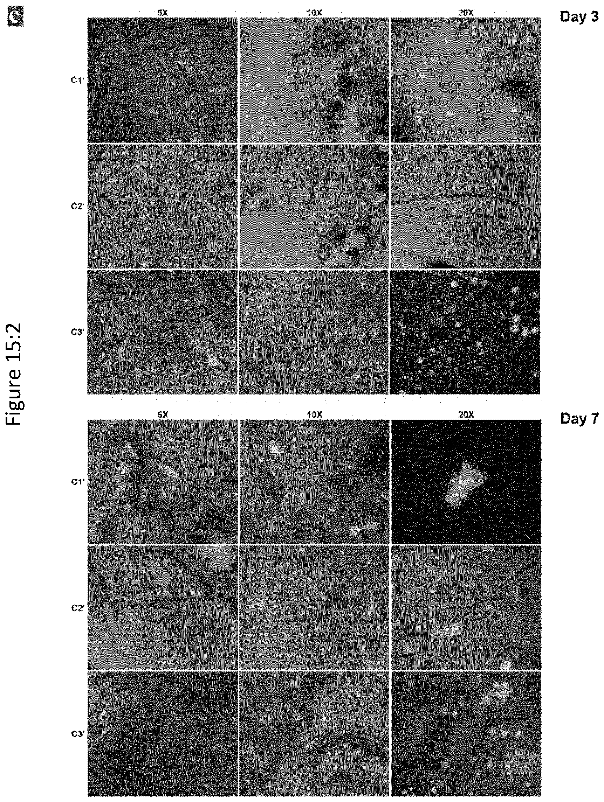

Figure 15 shows: (a) Fluorescence microscopy images of C3' in different channels (Alexa Fluor 405 (Ex. 401 nm, Em. 421 nm) blue, Calcein (Ex. 493 nm, Em. 514 nm) pale green, Alexa Fluor 514 (Ex. 514 nm, Em. 543 nm) green, Cy 3 (Ex. 549 nm, Em. 562 nm) orange,Alexa Fluor 594 (Ex. 590 nm, Em. 619 nm) pale red, FM 4-64 (Ex. 508 nm, Em. 751 nm) red; (b) Proliferation of engineered Lactococcus lactis with optogenetic gene expression sytem monitored with Superfolder GFP (Ex. 488 nm, Em. 510 nm) channel; (c) Viability test of human mesenchymal stem cells (hMSCs).

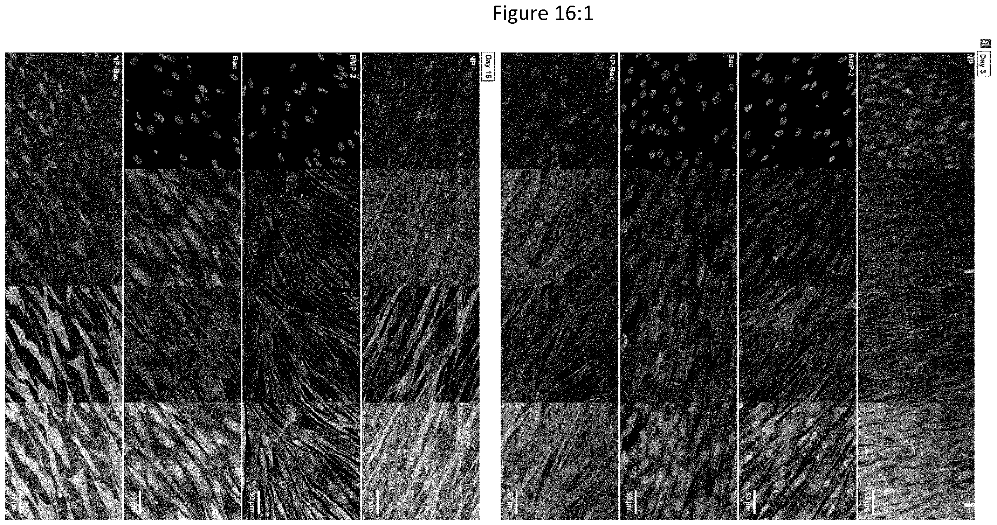

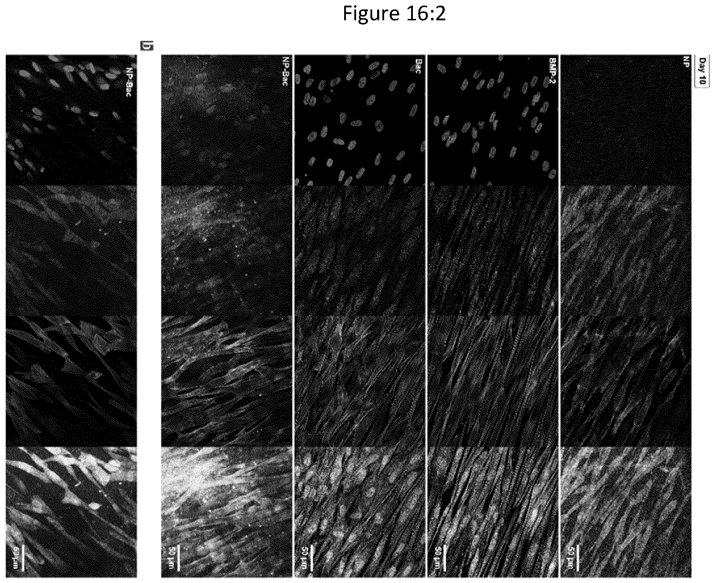

Figure 16 shows: (a) Immunostaining microscopy images and cell morphology of hMSCs in different conditions: NP: in vicinity of nanoparticles, BMP-2: with adding BMP-2 in media, Bac: contain BMP-2 releasing engineered bacteria, NP-Bac: having both NP and Bac. Images from Left to right: DAPI for nuclei (blue), Cy 3 for OPN (orange) and Alexa Fluor 488 (green) for actin and the merged images; (b) z-stack projection of hMSCs in day 16.

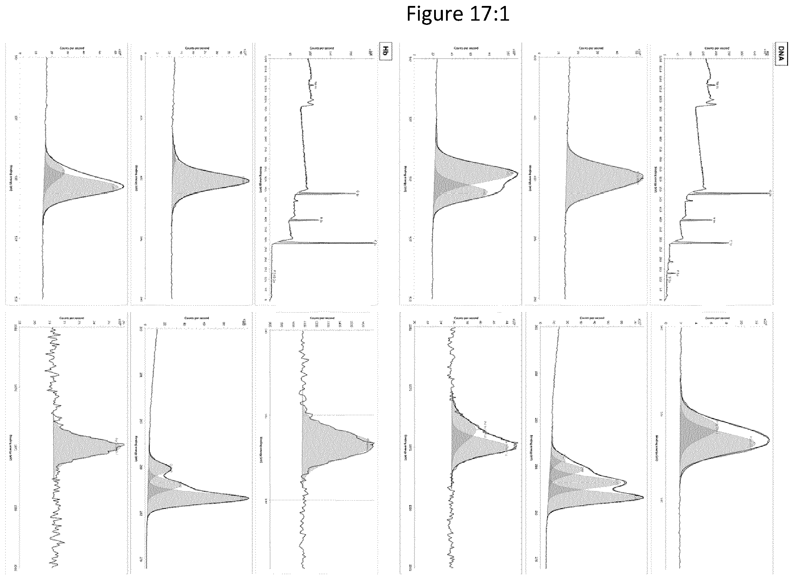

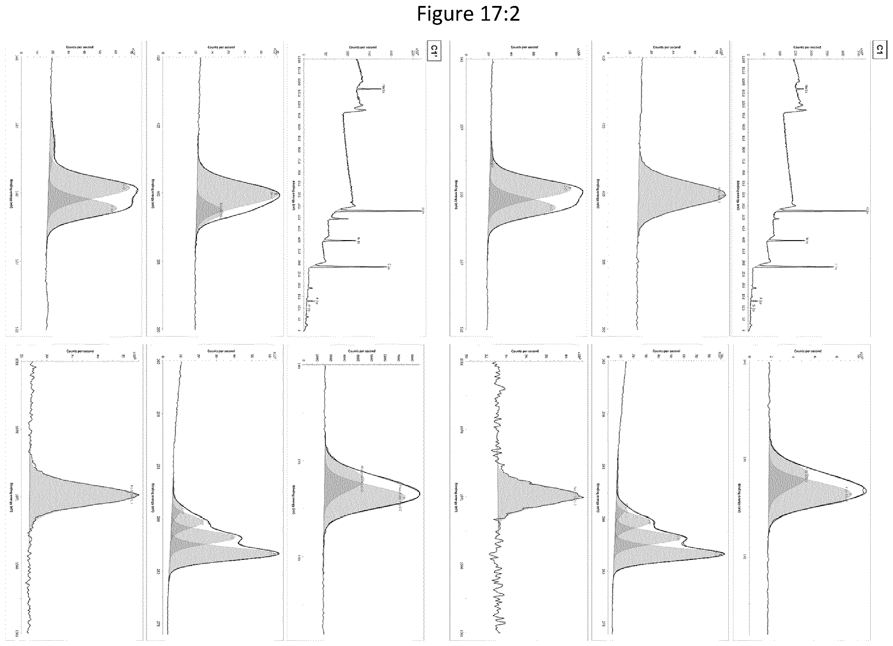

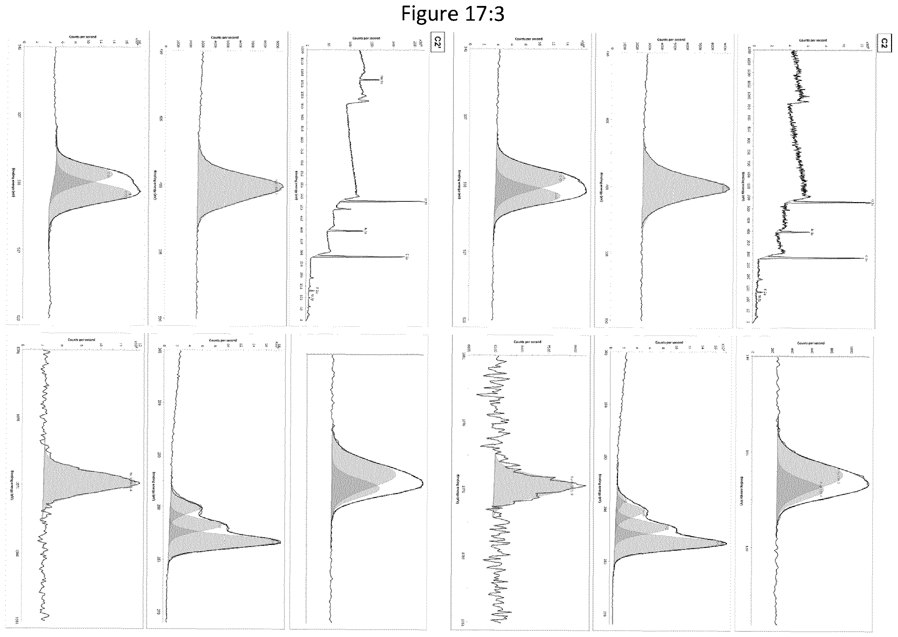

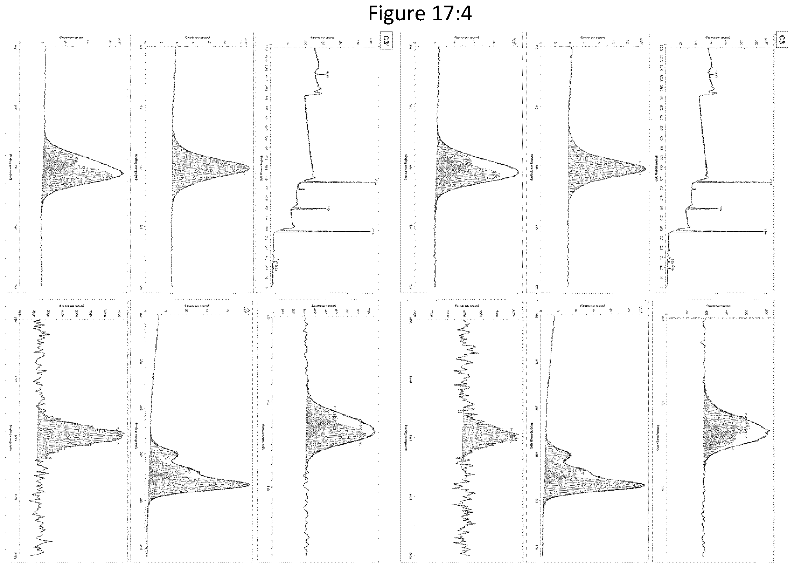

Figure 17 shows Wide XPS spectra and core level of modelled orbitals in region scan

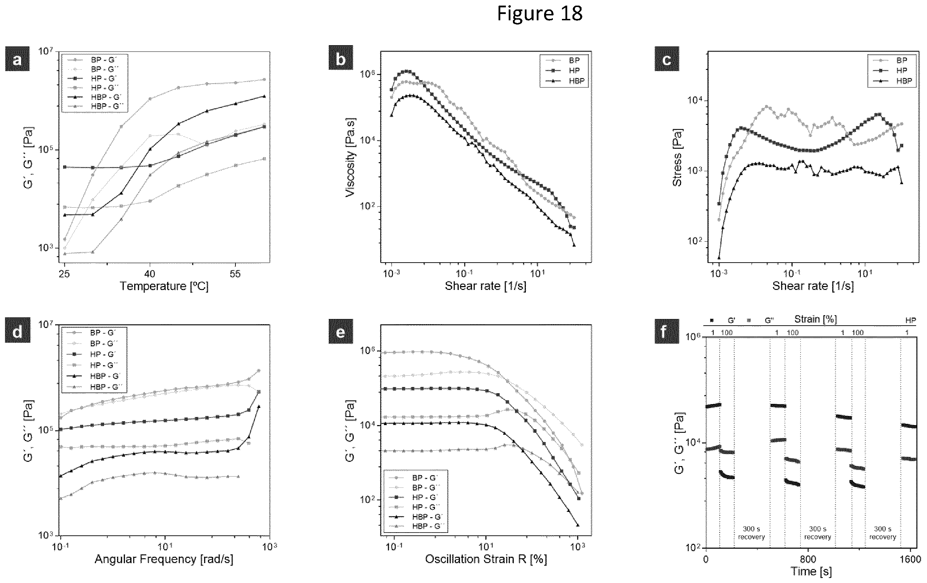

Figure 18 shows Rheology and printability demonstration. (a-f) Rheological properties: Including storage modulus G' and loss modulus G" vs. temperature (a), viscosity (25 °C) vs. shear rate (b) and stress (25 °C) vs. shear rate (c), G' and G" as function of angular frequency (d), G' and G" as function of oscillation strain (e), dynamic cyclic strain sweep measurement for modulus recovery properties in 1 and 100% shear strain of the hydrogels (HP: 16.66% Hb - 4.16% PEI, BP: 16.66% BSA - 4.16% PEI, and HBP: 8.33% Hb - 8.33% BSA - 4.16% PEI) (f).

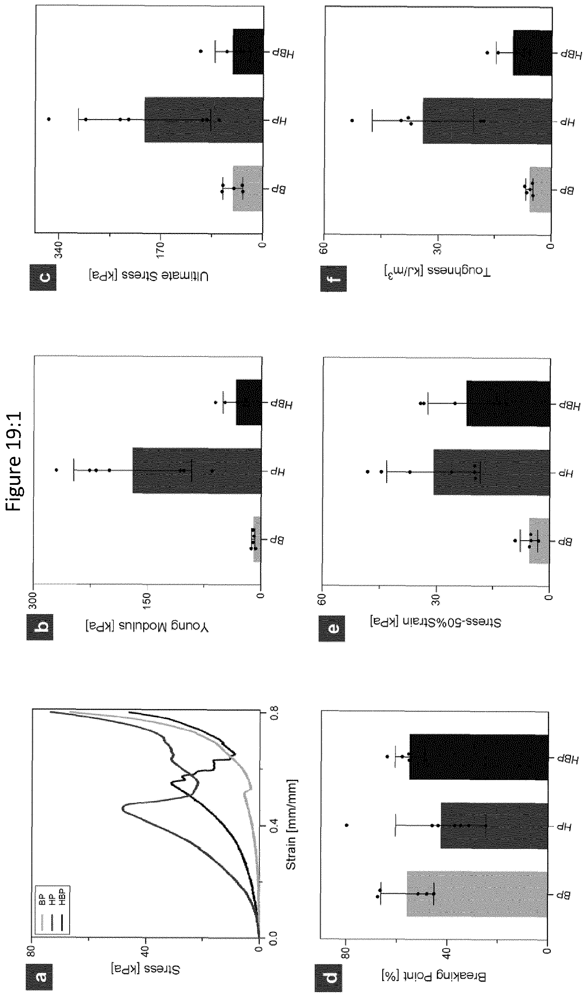

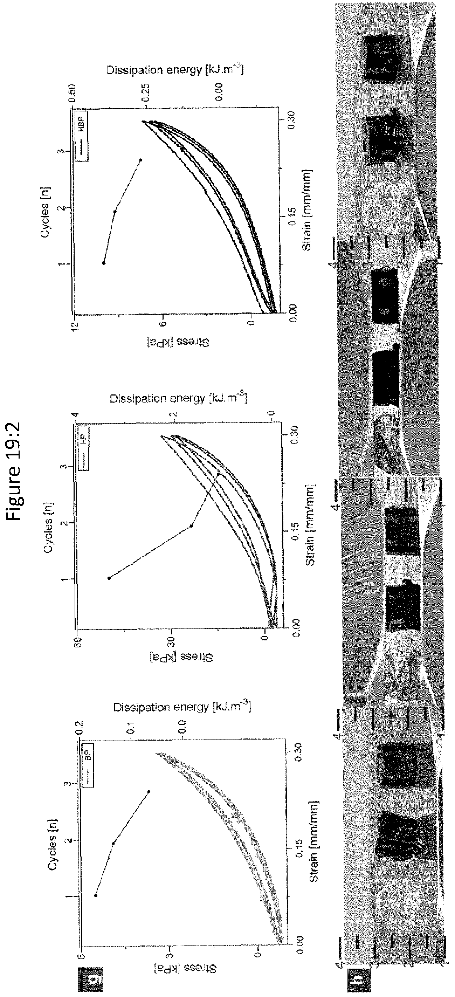

Figure 19 shows Mechanical characterization: (a) Compressive stress-strain curves for the different composites (HP: 16.66% Hb - 4.16% PEI, BP: 16.66% BSA - 4.16% PEI, and HBP: 8.33% Hb - 8.33% BSA - 4.16% PEI). Calculated (b) Young modulus, (c) Compressive strength, (d) Strain at break, (e) Stress at 50% strain and (f) Toughness values. (g) Cyclic mechanical test and calculated dissipated energy of the hydrogels for each cycle, (h) Images demonstrating the compressibility and recovery of HP, BP, HBP hydrogels.

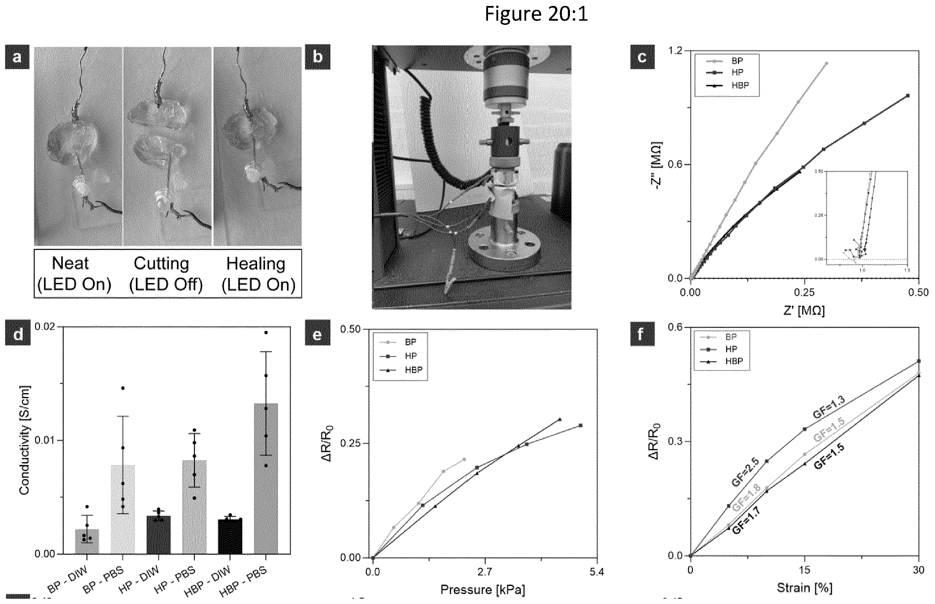

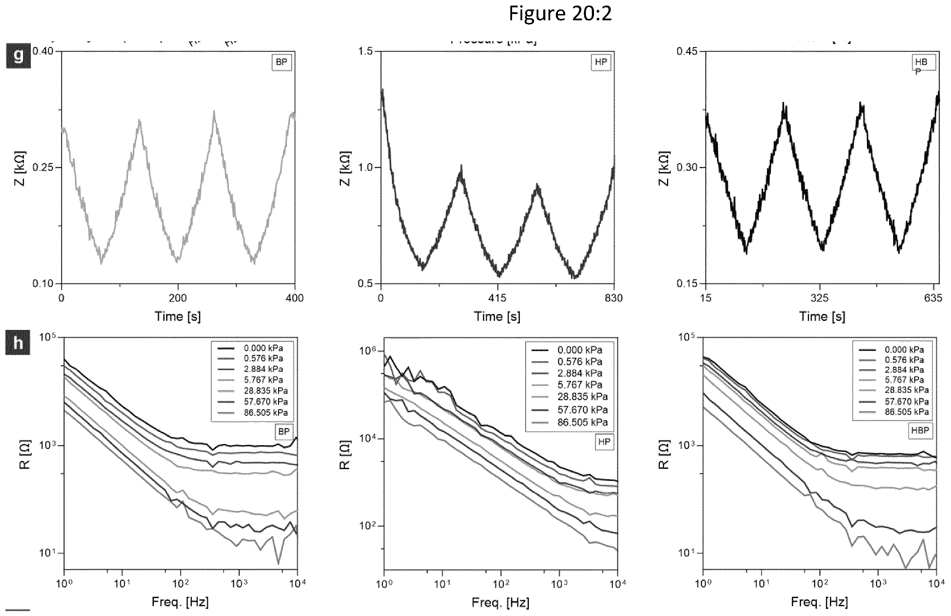

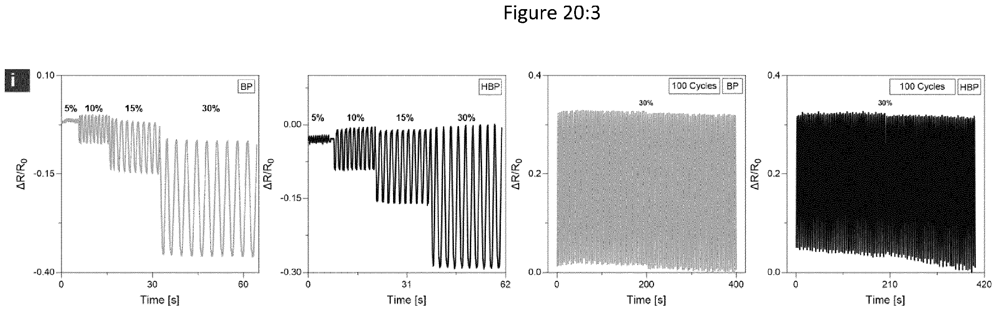

Figure 20 shows: (a) Electrical self-healing of the BP hydrogel, (b) Illustration of pressure sensitivity test, (c) Impedance spectroscopy of Hb.-BSA-PEI sample hydrogels (Imaginary vs. real part of the impedance spec.), (d) Conductivities of the different combination of HP, BP, HBP hydrogels in DIW and PBS (pH 7.4), (e) Impedance changes based on the compressive stress in 200 kHz of the HP, BP, HBP hydrogels, (f) Impedance changes based on the various strain percentages of the HP, BP, HBP hydrogels, (g) Cyclic compressive pressure sensitivity of HP, BP, HBP hydrogels and cyclic changes of the impedance, (h) Impedance changes based on the compressive stress in different frequencies of the HP, BP, HBP hydrogels, (i) Cyclic impedance changes based on the 5%, 10%, 15% and 30% strain percentages of the BP and HBP hydrogels, and stability of the cyclic compressive pressure sensitivity of BP and HBP hydrogels in 100 cycles. (HP: 16.66% Hb - 4.16% PEI, BP: 16.66% BSA - 4.16% PEI, and HBP: 8.33% Hb - 8.33% BSA - 4.16% PEI)

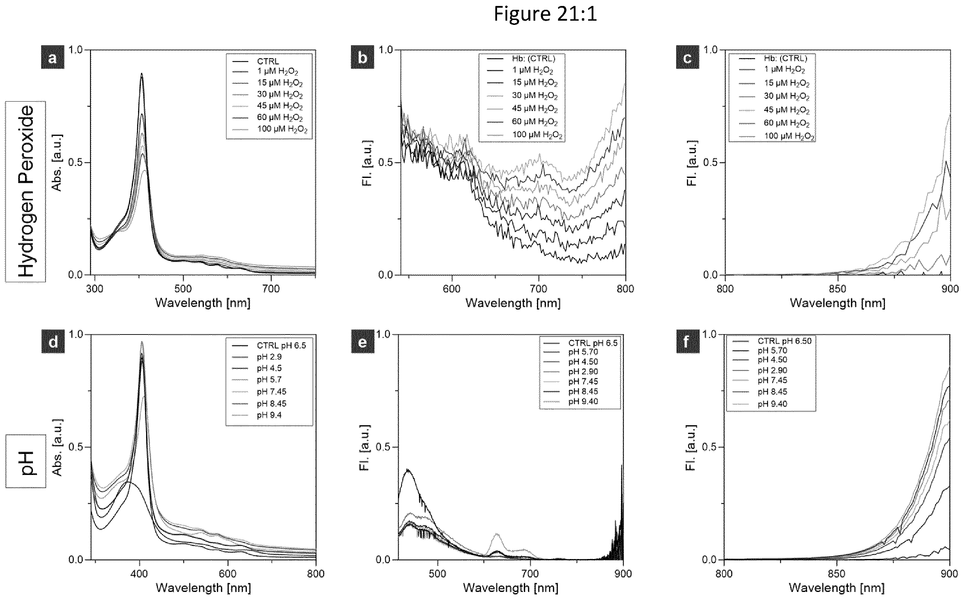

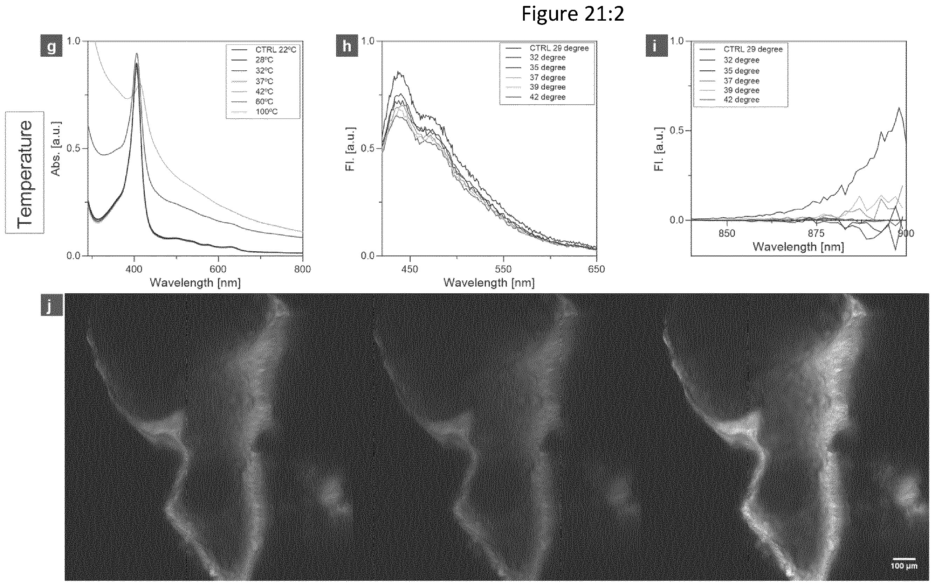

Figure 21 shows Hb. based sensing: (a) UV-Vis. Spectroscopy sensitivity of the Hb. to H2O2, (b) Fluorescence spectroscopy sensitivity of Hb. to H2O2 (ex. 400 nm), (c) Fluorescence spectroscopy sensitivity of Hb. to H2O2 (ex. 507 nm), (d) UV-Vis. Spectroscopy sensitivity of the Hb. to pH, (e) Fluorescence spectroscopy sensitivity of Hb. to pH (ex. 400 nm), (f) Fluorescence spectroscopy sensitivity of Hb. to pH (ex. 507 nm), (g) UV-Vis. Spectroscopy sensitivity of the Hb. to temperature, (h) Fluorescence spectroscopy sensitivity of Hb. to temperature (ex. 400 nm), (i) Fluorescence spectroscopy sensitivity of Hb. to temperature (ex. 507 nm), (j) Fluorescence imaging of HBP hydrogel in green and red channel emissions and their merge image (orange).

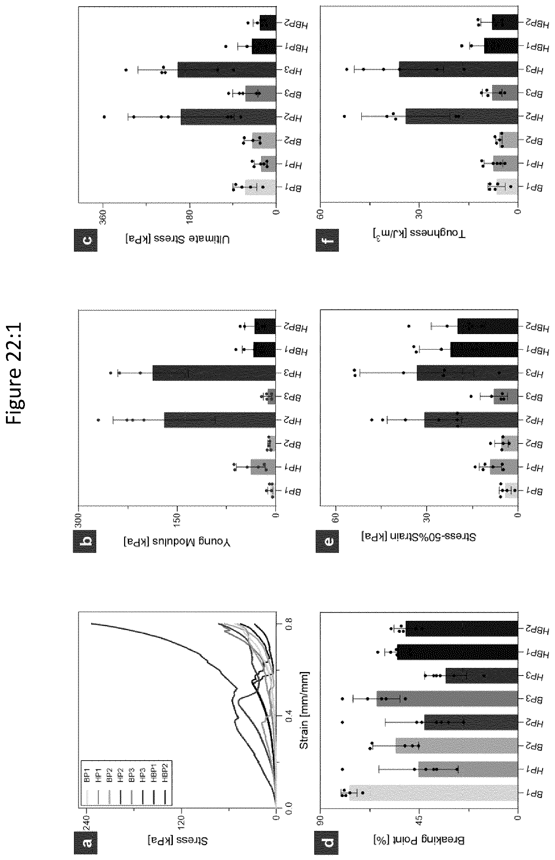

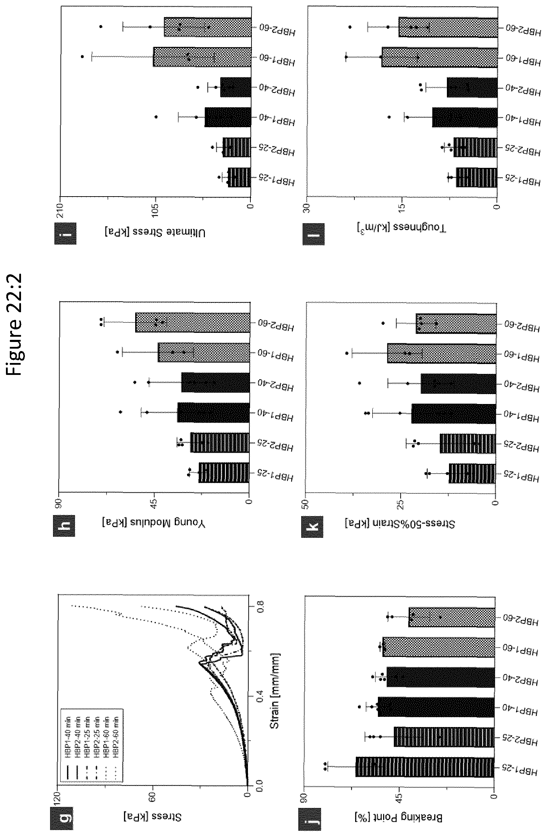

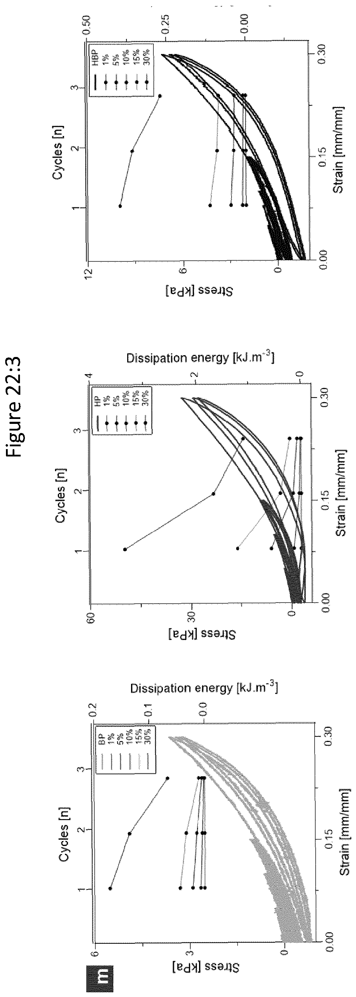

Figure 22 shows Mechanical and rheological characterization: (a) Compressive stress-strain curves for the different composites Hb.-BSA-PEI (HP: 16.66% Hb - 4.16% PEI, BP: 16.66% BSA - 4.16% PEI, and HBP: 8.33% Hb - 8.33% BSA - 4.16% PEI). Calculated (b) Young modulus, (c) Compressive strength, (d) Strain at break, (e) Stress at 50% strain and (f) Toughness values; (g) Compressive stress-strain curves for the different composites (HP: 16.66% Hb - 4.16% PEI, BP: 16.66% BSA - 4.16% PEI, and HBP: 8.33% Hb - 8.33% BSA - 4.16% PEI) in different temperatures. Calculated (h) Young modulus, (i) Compressive strength, (j) Strain at break, (k) Stress at 50% strain and (l) Toughness values. (m) Cyclic mechanical test in 1, 5, 10, 15 and 30% strain and calculated dissipated energy of the hydrogels for each cycle.

### Examples

### Example 1

### Goal of the experiment

### Materials and methods

### Preparation of DNA-BSA hydrogels

Stock solutions of deoxyribonucleic acid from fish sperm (dsDNA; Sigma-Aldrich 74782, with a typical molecular weight between 50 and 100 kDa, approximately 75-150 base pairs) and bovine serum albumin (BSA; A9647 Sigma-Aldrich, mol wt~66 kDa) were prepared at a concentration of 30% w/v and 20% w/v, respectively. This was achieved by dissolving the desired amount of DNA or BSA in deionized water. The BSA stock solution was homogenized by placing it on an orbital shaker at 40 rpm. The hydrogels were prepared with and without Protoporphyrin IX (demetallized hemin; Sigma-Aldrich). In mentioned samples, protoporphyrin IX added to the system in 0.1% w/v to the final stock solutions.

The DNA stock solution was homogenized in two different ways: The first method involved dissolving the DNA by heating the solution to 37 °C. The second method involved dissolving the DNA by adding DNA powder to a 0.5 M NaOH solution (Sodium hydroxide; Sigma-Aldrich S5881).

Three different sets of samples were prepared based on the DNA stock solution used and the final concentration:
a. Hard self-assembled C (SA-C) samples: A specific amount of DNA stock solution, which had been homogenized at 37 °C (30% w/v concentration), was added to a specific amount of BSA stock solution (20% w/v concentration) to obtain the desired weight percentages of DNA:BSA at ratios of 1:3, 1:1.85, and 1:1 (SA-C1: 1:3, SA-C2: 1:1.85, and SA-C3: 1:1 ratios).
b. Soft denatured C (D-C or C) hydrogels: The same protocol as for the C samples was followed (a above), with the difference being that the DNA stock solution was homogenized using a 0.5 M NaOH solution. After mixing the DNA with BSA, the samples were cured at 60 °C for 1 hour (C1, C2 and C3 or D-C1, D-C2 and D-C3).
c. Soft denatured C (soft C) gels: The same protocol as for the C hydrogels was followed (a above), with the difference being that the DNA stock solution was homogenized using a 0.1 M NaOH solution. After mixing the DNA with BSA, the samples were cured at 60 °C for 1 hour/ml (Soft C1, C2 and C3).

### Physicochemical, thermal and spectroscopy analysis

Functional group analysis of DNA-BSA hydrogels was performed using Fourier transform infrared (FTIR) spectroscopy. All samples were analyzed with a PerkinElmer Spectrum 100 FTIR spectrometer equipped with attenuated total reflectance (ATR) in the 4000-600 cm⁻¹ range, using 16 scans at a 1 cm⁻¹ resolution. The acquired spectra were baseline-corrected and standardized using PerkinElmer Spectrum software.

Additionally, the secondary structure of freeze-dried DNA-BSA hydrogels was determined by analyzing the absorbance spectra in the 1570-1740 cm⁻¹ region using Origin Pro 2023 software. The absorbance spectra were base-corrected and fitted with Gaussian-type peaks (average half-bandwidth of 25 cm⁻¹).

Thermogravimetric analysis (TGA) was conducted on freeze-dried samples using the TGA Q500 Thermogravimetric analyzer from TA Instruments (USA). The measurements were performed on a platinum pan, monitoring mass loss in the temperature range of 30 to 800 °C at a ramp of 10 °C/min. The analysis was carried out under a balanced nitrogen flow of 40 mL/min for the system and 60 mL/min for the sample.

Differential Scanning Calorimetry (DSC) measurements were conducted using the DSC Q200 instrument from TA Instruments. The analysis was performed on freeze-dried samples within a temperature range of -90 to 400 °C, with a ramp rate of 10 °C/min. A balanced nitrogen flow of 50 mL/min was maintained throughout the experiment.

To acquire DSC spectra, approximately 3 to 4 mg of the samples were weighed and placed in DSC CONSUMABLES sample pans and lids (Code: DSC84001 and DSC84002). The heat flow changes were then measured as a function of temperature, with reference to an empty sealed pan.

The morphology of the condensates, post freeze-drying and gold sputter coating (Quorum coater Q150T (Quorum Technologies, UK)), was analysed using a Scanning Electron Microscope (SEM). The specific SEM model used was the Quanta FEG 250 (ESEM). This analysis was conducted at an accelerating voltage of 5 kV, utilizing the Secondary Electron Detector (SED) mode. The pore sizes were subsequently analyzed using Fiji ImageJ.

The swelling properties of scaffolds were assessed by immersing samples in phosphate-buffered saline (PBS) at 37°C. Initially, we recorded the dry weight of the samples before soaking them in PBS. After a defined period of time, we measured the wet weight of the swelled samples. The swelling ratio percentage can be calculated using the following equation (Equation 1): $Swelling ratio \left(%\right) = \left(\left({W}_{s}-{W}_{d}\right)/{W}_{d}\right) \times 100$ Equation 1.

Where:
- (W*ₛ*) represents the swelled sample weight.
- (W*_{d}*) refers to the initial dry weight of the sample.

To simulate the behavior of the samples in a physiological environment, degradation experiments were conducted. Freeze-dried hydrogels were hydrated with PBS until they reached swelling equilibrium. The initial weight was established as a baseline and then the samples were immersed in PBS for varying time periods. At the end of each defined time interval, the samples were withdrawn, and their weight were measured. The degradation ratio was calculated by subtracting the remaining weight from the initial weight.

The Shimadzu UV-VIS spectrometer (UV-2600) was employed to measure the UV-Vis spectroscopy of samples with 1 nm steps. Additionally, the TECAN SPARK^{®} MULTIMODE MICROPLATE READER was utilized to measure fluorescence emission. The measurements were taken at the corresponding excitation monochromator, with the following parameters:
- Excitation bandwidth: 5 nm
- Gain (manual): 100
- Number of flashes: 30
- Automatic mirror setting: 50% Mirror
- Integration time: 40 µs
- Z-Position: 20,000 µm

The scaffolds were imaged with a LSM 900 confocal microscope (Zeiss, GmbH, Germany).

### Mechanorheological characterizations of scaffolds

The mechanical properties of the engineered constructs were assessed using an Instron model 5967 Universal Testing System (UK). Compression tests were performed with a 500 N load cell at a compression rate of 0.5 mm/min. Prior to evaluation, cylindrical scaffolds (with hydrogel dimensions approximately 4.65 mm in diameter and 4.81 mm in height, independently measured using a digital Vernier caliper) were soaked in PBS. The elastic modulus of the scaffolds was calculated from the stress-strain curve in the 0.15-0.25 mm/mm strain region, and the ultimate stress was determined as the maximum stress. Toughness was quantified by calculating the area under the stress-strain curve.

Cyclic compression testing was conducted by compressing the hydrogels to 75% strain and unloading them to 0% strain (at a constant speed rate of 0.5 mm/min for five cycles). Hysteresis energy, representing energy dissipation per unit volume, was measured by integrating the stress-strain curve between the loading and unloading hysteresis curves using MATLAB (MathWorks, Natick, MA) and OriginPro 2023 software.

Rheological assessment of DNA-BSA hydrogels was carried out using a Discovery Hybrid Rheometer HR-2 from TA Instruments. The experiments were conducted with an 8 mm parallel plate geometry and a gap distance of 500 µm at 37 °C. Shear storage modulus (G') and loss modulus (G") were determined as functions of oscillation strain and angular frequency in the following conditions:
- Frequency sweep analysis covered a strain range of 0.01-6000.00% at an angular frequency of 10 rad/s.
- Shear-rate analysis spanned 0.1-628.0 rad/s with an oscillation displacement of 0.12 rad.

To assess self-recovery ability, amplitude oscillatory strain was varied from 1% to subsequent large strains up to 1000% at an angular frequency of 10 rad/s, followed by a 300 s rest period after the large strain.

The apparent viscosity of DNA-BSA hydrogels was measured at 25 °C by varying the shear rate within the range of 0.001-100,000 s⁻¹. Additionally, dynamic rheothermo-analysis involved temperature ramps from 27 °C to 57 °C at a 50 rad/s angular frequency and 0.1% strain.

### Impedance spectroscopy and strain sensing analysis

To assess the conductivity of DNA-BSA scaffolds, the PalmSens4 instrument was utilized. Electrochemical Impedance Spectroscopy (EIS) was conducted with an AC amplitude voltage of 25 mV, spanning a frequency range from 1 MHz to 0.1 Hz. The DNA-BSA hydrogels, characterized by an average cylindrical shape (4.12 mm diameter and 2.69 mm height, independently measured using a digital Vernier caliper), were positioned between two stainless steel plates. By analyzing the Nyquist plot using built-in software, the hydrogel's resistance R was determined. The conductivity (σ) can be calculated using Equation 2: $σ = \left(1/R\right) \times \left(T/A\right)$ Equation 2.

Where:
- (T) represents the thickness of the sample.
- (A) denotes the area of the sample.

The self-healing properties of the hydrogels was further demonstrated by designing an electronic circuit. This circuit included a light-emitting diode (LED) operating at a direct voltage of 3-3.2 V, with the hydrogels serving as conductors. A 9 V battery connected via copper wires completed the circuit between the battery, hydrogel and the LED.

For monitoring body movements (such as twisting, bending, and stretching), sensors were fabricated using DNA-BSA hydrogel strips. These sensors, sized between 2-5 mm in length and 1-2 mm in thickness/wide, were connected to tinned copper sheets at both ends. To prevent water evaporation and ensure proper adhesion to the body, the sensors were sandwiched with acrylic foam adhesive tape (3M VHB). Placed on various body parts (wrist, finger, and elbow), these sensors allowed to measure resistance changes using the Agilent 4294A precision impedance analyzer.

All measurements were performed at 2 kHz using a sinusoidal AC voltage with a 0.5 V amplitude. We estimated relative impedance (ΔR/R₀), where (ΔR) represents the difference between the resistance under different measuring scenarios and the initial resistance (R₀).

### Biological characterizations

*Materials*: The following reagents were purchased from Sigma Aldrich in Spain: Cell Counting Kit-8 (CCK-8), collagenase P, p-nitrophenyl phosphate (pNPP), calf intestinal alkaline phosphatase (CIAP), dexamethasone, ascorbic acid, and β-glycerophosphate. Additionally, the mouse fibroblast L-929 cell line and culture media, including Dulbecco's Modified Eagle's Medium (DMEM 30-2002) and Eagle's Minimum Essential Medium (EMEM 30-2003), were acquired from ATCC in Spain. Furthermore, Phosphate Buffered Saline (PBS), Trypsin, fetal bovine serum (FBS), Penicillin-Streptomycin solution, the LIVE/DEAD^{®} kit, and 5-bromo-4-chloro-3-indolyl phosphate/nitro-blue tetrazolium (BCIP/NBT) were obtained from Fisher Scientific in Spain.

*Biocompatibility Assay*: L-929 mouse-derived fibroblasts were cultured in basal media composed of EMEM 30-2003 supplemented with FBS (10%, v/v) and penicillin-streptomycin (1%, v/v). The incubation of cell cultures occurred under the following conditions: 37 °C, 5% CO₂, and a humidified environment.

The biocompatibility evaluation of bioengineered systems followed ISO 10993 guidelines for "Biological Evaluation of Medical Devices: Cytotoxicity on Extracts and Cytotoxicity by Direct Contact." Cytotoxicity was assessed caused by both direct cell-scaffold interaction and non-direct toxicity using a CCK-8 colorimetric assay to measure cellular metabolic activity. 100% viability was set as the metabolic activity of cells that had no contact with the selected systems or their extracts.

*Cell Studies*: Bone marrow-derived mesenchymal stem cells (BM-MSCs) from mice were cultured under controlled conditions (37 °C and 5% CO₂). The cells were grown in DMEM media supplemented with 10% fetal bovine serum (FBS) and 1% penicillin/streptomycin.

Before seeding the engineered hydrogels with cells, all constructs were exposed to ultraviolet light for 15 minutes and then hydrated overnight with complete basal media. Subsequently, fully swollen samples were placed in wells and seeded with 105 murine BM-MSCs. After incubating the scaffolds in a CO₂-controlled environment for 1.5 hours, either complete basal medium or bone induction medium (complete basal medium supplemented with 0.5 µM dexamethasone, 200 µM ascorbic acid, and 10 mM β-glycerophosphate) was added to each well. Throughout the assays, the cell culture media was replaced every 2-3 days.

*Cell Viability and Proliferation Study*: The cell viability of seeded samples was assessed following the Live/Dead viability guidelines. At 24 hours and 8 days post-seeding, four samples per prototype (Alginate (3D Control), C1, C2, C3) were evaluated. Calcein-AM and ethidium solution were used to stain viable and non-viable cells, respectively. Microscopic fluorescence images were captured using an inverted microscope (Nikon TMS). Simultaneously, cell proliferation through metabolic activity was evaluated using the CCK-8 assay, as described by Echave MC et al⁴⁸⁴. Briefly, after washing the samples with PBS, each scaffold was incubated with a 10% CCK-8 kit reagent in basal medium for 4 hours at 37 °C. The resulting solution was measured using a Tecan Infinite M2000 microplate reader.

### Osteogenic studies

*Alkaline Phosphatase (ALP) Activity and Staining:* Cell-secreted ALP activity was assessed weekly using a spectrophotometer over a 21-day period. Intracellular ALP activity was measured at pH 9.3 through p-NPP hydrolysis. A CIAP formulation, in conjunction with 0.2% (w/v) pNPP, served as the standard curve and was incubated with the samples. Subsequently, a 3 M NaOH solution terminated the reaction, and absorbance at 405 nm was recorded. Finally, seeded cells were stained with BCIP/NBT solution. After several rinses with PBS, the samples were light-protected and incubated for 2 hours at room temperature (RT). Any excess staining was then removed from the scaffolds using PBS. Images were acquired using a Nikon AZ100 microscope.

*Alizarin Red Staining for Extracellular Calcium Deposition:* Alizarin Red S (ARED) staining was employed to assess extracellular calcium deposition. The staining solution was prepared following the method described by Erezuma et al⁴⁸⁵. Briefly, 1 g of ARED powder (Sigma-Aldrich) was dissolved in 50 mL of Milli-Q water, and the pH was adjusted to 4.2 ± 0.1. The solution was then filtered through a 0.45 µm filter.

Before staining, the seeded cells were fixed in 4% paraformaldehyde for 30 minutes, and the scaffolds were washed three times with DPBS. Subsequently, the scaffolds were submerged in the ARED staining solution for 1 hour at room temperature (RT) while being protected from light. After staining, the scaffolds were thoroughly washed with Milli-Q water and left overnight to remove any excess ARED.

Images were captured using a Nikon AZ100 microscope. For quantitative measurement, ARED extraction was performed using HCl as the extracting agent. Following ARED removal, absorbance was measured at 490 nm using a Tecan Infinite M2000 spectrophotometer, and a calibration curve was used for quantification.

### Statistical analysis

Statistical data analysis was performed using GraphPad Prism 8.0.1 software and Excel 2016. The Shapiro-Wilk test was used to assess the normal distribution of the data. For datasets following a normal distribution, a one-way ANOVA was conducted to analyze differences between the groups. Post-hoc comparisons were carried out using the Tukey test. In all cases, significance was assumed for p-values < 0.05, and these values were indicated on the graphs. Results are presented as the mean ± standard deviation.

### Results and discussion

### Engineering structure, rheomechanic, and printability

Regarding the interactions between DNA and BSA, dense-opaque self-assembled condensates were formed. However, a challenge arose: how to transform these condensates into a transparent hydrogel while simultaneously altering their crystallinity, self-assembly, and density. The ointment lay in denaturation caused by increasing the pH. To dissect the structural changes, fourier transform infrared spectroscopy (FTIR), differential scanning calorimetry (DSC), and thermogravimetric analysis (TGA-DTG) were employed.

Figure 1. (f) displays FTIR spectra for various combinations of DNA and BSA samples. Notably, as the DNA percentage in hydrogels increased, the characteristic peaks associated with DNA intensified, while those linked to BSA decreased (indicated by dashed lines). Additionally, the peak centered at 1600 cm⁻¹ shifted to higher wavenumbers with increasing DNA ratio. Conversely, the peaks at 1527 cm⁻¹ (mostly associated with BSA) and 1398 cm⁻¹ decreased compared to the peak at 1655 cm⁻¹ (associated with overlapping DNA and BSA peaks), shifting to lower wavenumbers.

Furthermore, FTIR peaks were deconvoluted centered at 1620 cm⁻¹ (intermolecular aggregate β-sheet), 1635 cm⁻¹ (intramolecular native β-sheet and short segment chains connecting α-helical segments), 1645 cm⁻¹ (disordered random coils), 1655 cm⁻¹ (α-helix), 1677.5 cm⁻¹ (β-turn), and 1685 cm⁻¹ (intermolecular antiparallel β-sheet). For simplicity, all peaks at 1620, 1635, and 1685 cm⁻¹ were associated with β-sheet-like structures. By tracking changes in secondary structures with varying ratios, we deduced that increasing the DNA ratio led to a decrease in random coils and an increase in β-sheet and β-turn (Figure 1. (g) and Figure S1). Notably, sample C2 exhibited a harmonious distribution of α-helices, random coils, and β-sheets, along with the lowest β-turn, likely contributing to its superior toughness.

The thermal behavior of various combinations of DNA-BSA hydrogels, along with neat DNA and BSA, was investigated using (DSC) and TGA-DTG. DSC measurements (Figure 1. (c) and Table 1) allowed us to quantify the heat flow into or out of the samples.

In neat DNA and BSA, the onset temperatures for the first transitions were approximately 47 °C and 37 °C, respectively (associated with hybridization and unfolding denaturation processes). The corresponding peaks occurred at 95 °C and 86 °C, representing the melting points. Notably, the hydrogels exhibited transition values intermediate between those of neat DNA and BSA. Specifically, the C2 sample displayed a higher first mean transition temperature (Tₜ₁) compared to C1 and C3.

**Table 1. Experimental data derived from DSC, TGA, and DTG**

| | MC-FS HV (%) | | MC-SS LV (%) | | FS-PT (°C) | | FS-PMC (%) | | SS-PT (°C) | | SS-PMC (%) | |
|---|---|---|---|---|---|---|---|---|---|---|---|---|
| | Mean | SD | Mean | SD | Mean | SD | Mean | SD | Mean | SD | Mean | SD |
| BSA | 7.35 | 0.21 | 72.24 | 0.38 | 64.03 | 2.45 | 5.79 | 0.62 | 289.87 | 1.77 | 38.09 | 0.25 |
| C1 | 17.27 | 1.17 | 54.56 | 1.33 | 73.26 | 2.29 | 8.85 | 0.02 | 250.39 | 1.03 | 30.07 | 0.63 |
| C2 | 20.18 | 0.17 | 49.32 | 0.22 | 81.06 | 3.00 | 9.67 | 0.19 | 251.51 | 0.05 | 30.08 | 0.56 |
| C3 | 16.36 | 1.08 | 47.48 | 3.25 | 61.09 | 7.94 | 10.57 | 1.73 | 258.13 | 0.63 | 32.36 | 1.95 |
| DNA | 5.39 | 0.57 | 52.05 | 0.15 | 54.30 | 0.95 | 5.01 | 0.22 | 239.47 | 0.59 | 26.27 | 0.16 |

| | | | | | | | | | | | | |
|---|---|---|---|---|---|---|---|---|---|---|---|---|
| MC: Mass change, FS: First step, HV: High volatility, LV: Low volatility, P: Peak, T: Temperature | | | | | | | | | | | | |

| | OSTₜ₁ (°C) | | Tₜ₁ (°C) | | I₁ (J/g) | | OSTₜ₂ (°C) | | Tₜ₂ (°C) | | I₂ (J/g) | |
|---|---|---|---|---|---|---|---|---|---|---|---|---|
| | Mean | SD | Mean | SD | Mean | SD | Mean | SD | Mean | SD | Mean | SD |
| BSA | 36.76 | 1.37 | 86.25 | 2.49 | 173.93 | 32.71 | 208.28 | 0.78 | 216.94 | 0.44 | 29.51 | 6.40 |
| C1 | 38.59 | 1.33 | 86.13 | 1.67 | 329.63 | 9.91 | 208.26 | 0.19 | 217.52 | 0.35 | 16.42 | 0.76 |
| C2 | 41.58 | 4.94 | 92.43 | 5.27 | 294.70 | 35.58 | 202.67 | 1.50 | 208.72 | 0.94 | 4.83 | 3.75 |
| C3 | 41.35 | 2.32 | 88.71 | 2.82 | 263.27 | 22.16 | 194.80 | 0.42 | 203.02 | 0.97 | 3.56 | 0.36 |
| DNA | 47.47 | 15.26 | 94.90 | 2.04 | 289.73 | 49.78 | 176.19 | 0.22 | 138.68 | 77.05 | 54.71 | 5.09 |

| | | | | | | | | | | | | |
|---|---|---|---|---|---|---|---|---|---|---|---|---|
| OSTₜ: OnSet Transition temperature, Tₜ: Transition temperature, I: Integrate peak sig horizontal | | | | | | | | | | | | |

| | OSTₜ₃ (°C) | | Tₜ₃ (°C) | | I₃ (J/g) | | OSTₜ₄ (°C) | | Tₜ₄ (°C) | | I₄ (J/g) | |
|---|---|---|---|---|---|---|---|---|---|---|---|---|
| | Mean | SD | Mean | SD | Mean | SD | Mean | SD | Mean | SD | Mean | SD |
| BSA | 247.75 | 0.52 | 292.52 | 2.07 | 19.01 | 3.74 | 301.17 | 1.94 | 311.07 | 1.36 | 83.24 | 15.28 |
| C1 | 241.68 | 2.83 | 257.20 | 0.85 | 4.05 | 0.62 | 280.83 | 0.31 | 301.85 | 0.53 | 25.13 | 1.24 |
| C2 | 233.04 | 2.44 | 245.51 | 4.78 | 6.42 | 0.34 | 291.63 | 0.91 | 319.38 | 1.71 | 28.51 | 11.32 |
| C3 | 226.23 | 3.83 | 248.66 | 1.07 | 16.84 | 0.37 | 293.29 | 0.47 | 325.12 | 2.54 | 32.68 | 1.59 |
| DNA | 208.63 | 5.70 | 215.60 | 0.80 | 19.12 | 1.35 | 241.49 | 5.95 | 259.89 | 6.53 | 40.74 | 2.19 |

| | | | | | | | | | | | | |
|---|---|---|---|---|---|---|---|---|---|---|---|---|
| OSTₜ: OnSet Transition temperature, Tₜ: Transition temperature, I: Integrate peak sig horizontal | | | | | | | | | | | | |

Furthermore, the enthalpy changes during the first transition were greater for DNA and the hydrogels than for BSA. The order of these values aligned with the rigidity of the hydrogels (C1 > C2 > C3). As for the second and third transitions in DNA, they were exothermic, likely due to isomerization and oxidation reactions. These were followed by segment decomposition in subsequent transitions.

The TG curves for various compositions are depicted in Figure 1. (d and e) and summarized in Table 1. These curves reveal a multi-step thermal decomposition pattern across all samples. The initial stage of the TG curve corresponds to the thermal denaturation of BSA and DNA, accompanied by the loss of bound water. Notably, the mass change during freeze-drying of hydrogels was threefold greater than that observed for neat DNA and BSA, indicating the pronounced humidity-absorbing capacity of these porous samples.

Furthermore, the first-step peak temperature (FS-PT (°C)) for sample C2 was the highest, surpassing the corresponding values for neat DNA and BSA. The second decomposition step likely results from the thermal degradation of BSA and DNA chains into smaller molecular segments. Finally, the gradual, continuous weight loss can be attributed to the decomposition of the DNA-BSA backbone and the final matrix.

Comparing neat BSA and DNA, the second weight loss region in BSA exhibits a steeper slope during the second degradation step. This phenomenon is attributed to the faster decomposition of amide bonds in BSA. Additionally, the different DNA-BSA ratios exhibit a comparable degradation pattern (MC-SS LV %), with the order of degradation rates being C1 > C2 > C3 relative to neat DNA and BSA.

The microstructure images of various DNA-BSA network hydrogels are depicted in Figure 1. (h). In this study, membrane porosity was examined using ImageJ software (Figure 1. (i)). The combined results revealed interconnected pore networks with potential biomedical applications, facilitating mass transport. Notably, as the DNA ratio increased in the hydrogel combinations, the average pore size significantly decreased from approximately 300 µm to 100 µm. Among these, the C3 freeze-dried hydrogel, created by stacking feathers like structures, exhibited the highest pore density.

To investigate the long-term stability of hydrogels, their swelling and degradation behavior were assessed in an aqueous PBS solution at pH 7.4 and 37°C, as depicted in Figure 1. (j and k). Notably, the hydrogels exhibited rapid swelling kinetics, reaching a final swelling capacity of approximately 600-700%. Over a two-month period, the degradation ratio fell within the range of 30-40%.

To render DNA-BSA hydrogels printable, we meticulously manipulated pH, temperature, and time, ensuring their suitability for injection, printing, and conformability (Figure 2. (e-f)). Remarkably adaptable to complex surfaces, these hydrogels are ideal for crafting complex 3D shapes and patterns. The synergy between printability, conformability, and conductivity positions DNA hydrogels as invaluable tools in flexible biomedicine, bioelectronics manufacturing, and healthcare solutions.

In Figure 2. (a, b), a slight increase in viscosity and stress was observed with rising shear rates up to 0.01 s⁻¹. Beyond this threshold, flowability escalates significantly (by a factor of 10⁴) under nearly constant stress. Elevated temperatures induce denaturation, augmenting the hydrogels' modulus (Figure 2. (c)). Notably, C3 hydrogels initially exhibit decreased modulus due to higher DNA ratios, with the DNA component playing a role in this behavior, while the BSA component shows the opposite trend.

Additionally, the self-healing and recovery behavior of DNA-BSA hydrogels were verified by cutting and re-assembling them. Whether subjected to compression loads (Figure 2. (d)) or cycled On-Off-On states of an LED lamp (Figure 4. (f)), these hydrogels maintain uniformity. Their self-healing capacity not only enhances reusability but also expands the window for scaffolds in a dynamic matrix-cell mechanotransduction signaling.

Mechanical tests were conducted on DNA-BSA hydrogels due to the critical role of mechanical cues in cell adhesion, migration, and differentiation. As depicted in Figure 3. (a-h), varying the DNA-to-BSA ratio within the hydrogel systems resulted in significant changes. Specifically, increasing the DNA content or decreasing the BSA proportion led to a nearly threefold reduction in the compressive Young's modulus (from approximately 56 to 19 kPa) and the stress at 50% strain (from 33 to 10 kPa). These findings indicate that higher DNA content renders the hydrogels softer. Notably, the 35% DNA-65% BSA combination exhibited a higher ultimate compressive stress (UCS), toughness and breaking point comparable to the observed points in the 25% DNA-75% BSA and 50% DNA-50% BSA combinations.

Additionally, cyclic compression stress (as shown in Figure 3, panels g and h) allowed the hydrogels to recover, suggesting their potential use as pressure sensors. Preliminary rheological analysis further confirmed these trends (Figure 3, panels i-k, and Figure S2). Increasing the DNA content or decreasing the BSA ratio resulted in a sixfold decrease in the storage modulus. Moreover, the hydrogels exhibited increased pseudo-plasticity, shear thinning behavior, and viscoelastic-liquid characteristics. Notably, the oscillatory shear strain percentages at the breaking point increased from ~315% (25% DNA-75% BSA) to ~1000% (50% DNA-50% BSA) with higher DNA content. Overall, our rheological studies demonstrated that DNA-BSA hydrogels possess remarkable self-recovery capabilities, achieving strains exceeding 1000%.

### Multi-channel physicochemical sensing of hybrid: conductivity to fluorescence

The conductivity and strain sensitivity of DNA-BSA hydrogels are demonstrated in Figure 4, with a high gauge factor (approximately 3.5) observed at 600% stretching strain (Figure 4. (h)).

Notably, the conductivity of these hydrogels increases with higher DNA ratios, consistent with the behavior of polyelectrolyte macromolecules (Figure 4. (e)). While there are diverse views on DNA conductivity, the consensus remains that structural changes, attached ions, and co-assembled proteins significantly impact its conductivity. In our case, the increase in conductivity may be attributed to alterations in material density and structural features (as evidenced by SEM images showing feather-like plates with smaller pore sizes at higher DNA ratios) or an elevated charge density of DNA.

Considering the versatility of hydrogel fabrication, we propose exploring their potential as mechanical sensors. Specifically, investigating DNA-DNA, DNA-BSA, and BSA-BSA interactions-both in the presence and absence of ions-could unlock valuable insights. These interactions could be harnessed for compression, tension, and various motion scenarios, catering to both hard (compressible) and soft (stretchable) hydrogels. Notably, our hydrogels exhibit nonlinear impedance changes under different pressure conditions (Figure 4. (b-d)), suggesting promise for pressure and strain sensitivity. Furthermore, DNA-BSA hydrogels respond to different human motion patterns (slow, fast, stepwise, and square), making them potential candidates for tracking musculoskeletal disorders or serving as motion sensors (Figure 4. (g) and Figure S3).

An intriguing question arises: Do DNA and BSA interactions resemble molecular cracks (MC)? As external forces change-whether through compression, tension, or bending-the interactions between condensates or self-assembled DNA-BSA stacks, along with anchored BSA to DNA, undergo modifications. These alterations create angstrom-scale domain mismatches or cracks (ASM or ASC), ultimately affecting the hydrogel's conductivity. This phenomenon likely contributes to the observed high strain sensitivity and gauge factor.

To monitor Ca2+ dynamics and biomineralization, we demetallized the hemin molecule to enhance its solubility, fluorescence intensity in the near-red region, and sensitivity toward Ca2+. We employed MALDI-TOF mass spectrometry, FTIR, and UV-Vis spectroscopy to characterize the demetallization process. These analyses confirmed the successful removal of Fe from the Hemin groups, as evidenced by changes in m/z values, free amine -N-H stretching vibrations, and the emergence of Q-bands (Figure 12. (a and b) and Figure S5 (not shown).

As depicted in Figure 5. (a-c, m) and Figure 12. (d, e), increasing Ca2+ concentration led to a decrease in UV-Vis absorbance and fluorescence emission. This information can be harnessed for real-time monitoring of biomineralization processes. Additionally, DHem exhibited sensitivity to pH, H2O2, and temperature in the red and near-infrared (NIR) regions (zones I and II). These properties enable the tracking of spatiotemporal multi-stimuli signals based on their kinetic and magnitude responses, as demonstrated by distinct calibration curves (Figure 5).

### Photo-enhanced conductivity

The impact of UV exposure on DNA and BSA was investigated. Figure 6 reveals that up to 30 minutes of exposure, an increase in absorbance was observed, accompanied by a left-shifted peak in DNA. Conversely, in BSA, absorbance increased below 350 nm but decreased at 420 nm, resulting in a right-shifted peak.

Regarding fluorescence intensity, DNA exhibited increased emission at an excitation wavelength of 240 nm and decreased emission at an excitation 380 nm with prolonged UV exposure. In contrast, BSA fluorescence initially increased and then decreased at 240 nm, while showing a higher magnitude increase at 380 nm. These spectral and intensity changes indicate structural alterations in both DNA and BSA due to prolonged UV exposure. Consequently, their conductivity may also be affected following these structural modifications.

In the investigation of impedance spectroscopy, the effects of varying UV exposure intensities were scrutinized on distinct samples: DNA, self-assembled DNA-BSA (SA-C3), and denatured DNA-BSA (D-C3) in a 1:1 weight ratio (as depicted in Figure 7). As the UV intensity increased, intriguing shifts emerged.

Firstly, all samples exhibited a leftward shift in the Nyquist plot, accompanied by decreased impedances across different frequencies. SA-C3 experienced a significant impedance drop in lower frequencies after UV exposure, whereas DNA and D-C3 maintained near-constant impedance changes levels. Furthermore, the impedance decreases in the wide frequency range for DNA followed a harmonious pattern, with consistent fitted circuit behavior before and after exposure.

In stark contrast, D-C3 and SA-C3 diverged. SA-C3 demonstrated pronounced impedance reduction at lower frequencies compared to D-C3. The fitted circuit for D-C3 necessitated a series of imperfect impedances, including the CPE (constant phase element) or Warburg impedance. Remarkably, SA-C3's circuit transformed, requiring parallel elements after relaxation from UV exposure.

Notably, the samples exhibited cyclic, reversible changes in conductivity during on-off UV exposure states. These behaviors hold promise for remote spatiotemporal control and noninvasive manipulation of electronic and biological circuits, as well as wearable photodetectors (see Figure 7, panel (d)).

### Biological functions and biomineralization capacity

The preliminary biocompatibility test demonstrates the hydrogel matrices' capacity to support cell survival, specifically L-929 fibroblasts and bone marrow mesenchymal stem cells (Figure 8. (a-d)). Cytocompatibility was assessed through both direct (toxicity resulting from direct cell-hydrogel contact) and indirect tests (toxicity induced by extracts), following established guidelines. For a device to be considered biocompatible, cell viability must exceed 70% compared to the control486,487. As depicted in Figure 8. (a, b), there were no significant differences between the 3D control and C1 sample, and C2 and C3 hydrogels showed higher viability; in fact, all formulations exhibited viability above 70% in both tests. Consequently, in accordance with ISO requirements, these systems can be considered non-cytotoxic for further in vitro studies.

The viability of mBM-MSCs was assessed eight days after seeding the cells on top of hydrogels. Cell quantification was based on metabolic activity, revealing that all designed scaffolds effectively promoted cell viability. Notably, the viability increased significantly in C2 and C3 samples (see Figure 8. (c)). Specifically, C2 samples exhibited a statistically significant rise in cell population after exposure to the scaffolds for an 8-day period. To further validate these results, Live/Dead evaluation demonstrated a high percentage of green-dyed cells (indicating live cells) after a week of contact with the scaffolds (see Figure 8. (d)).

These findings align with previous publications, which have shown that DNA-based hydrogels are non-cytotoxic and create a favorable environment for cell growth488. Consequently, it can be inferred that bioadaptable DNA-BSA based constructs have the potential to enhance cell proliferation. Future work will focus specifically on the C2 and C3 samples.

Furthermore, their osteogenic potential was assessed using an early marker-alkaline phosphatase (ALP) activity-and visualized biomineralization through Alizarin Red (ARED) staining (Figure 8. (e-h)).

The intracellular activity of ALP was measured over a span of three weeks, and optical images were captured after 21 days of seeding cells. Notably, there was a consistent upward trend in ALP activity during this early marker period (see Figure 8. (e, g). Specifically, a statistically significant increase was observed from week 1 to week 3. Notably, ALP activity increased 1.5-2-fold during week 3 compared to week 1. Interestingly, samples incubated with differentiation medium exhibited higher ALP activity, a finding further confirmed by stained samples after 21 days: those treated with differentiation medium displayed more violet dots compared to samples incubated with basal media.

During the second week of cell incubation on the scaffolds, extracellular calcium deposits formed by differentiated osteoblasts were quantified using ARED489. As illustrated in Figure 8. (f, h), cells incubated with hydrogels-particularly those treated with differentiation medium-showed elevated levels of ARED. Additionally, when cultured in specialized medium (C3), the hydrogels exhibited the highest biomineralization capacity-approximately fourfold greater than cells cultured in normal media. This enhanced biomineralization is likely associated with a higher DNA ratio and the phosphate groups' ability to capture Ca2+, initiating nucleation and subsequent growth of the mineralized deposits.

These results suggest that the osteogenic properties of the hydrogel, within an osteoinductive environment, may enhance cell differentiation toward bone cells, which play a crucial role in calcium deposition within the extracellular matrix.

### Conclusion of example 1

In the specialized field of biomedical engineering, the complexity of pathophysiological processes presents significant challenges, characterized by deviations from normative physiological states. To address this complexity, we have pioneered a novel category of self-healing hydrogels which fabricated from DNA-BSA matrices with and without demetallized hemin ligand. These bioactive (macro)molecules work together to give the hydrogels with biocompatibility, osteogenic properties, intrinsic self-healing capabilities, and sensitivity to a wide range of physiological and chemical stimuli. By strategically incorporating hemin and demetallized hemin, we have laid the groundwork for a new generation of smart, injectable, and conductive hybrid hydrogels. These innovative materials hold promise for applications in diverse biomedical and bioelectronic domains which advance therapeutic and diagnostic interventions.

### Example 2: scalable Active-Living Biomaterials (ALB) comprising DNA-Hemoglobin nanocondensates

### Materials and methods

### Preparation of DNA-Hb condensates

Stock solutions of deoxyribonucleic acid from fish sperm (dsDNA; Sigma-Aldrich 74782, with a typical molecular weight between 50,000 and 100,000 Da, approximately 75-150 base pairs) and bovine hemoglobin (Hb; Millipore 08449, Sigma-Aldrich, mol wt ~64.5 kDa) were prepared at a concentration of 25% w/v. This was achieved by dissolving the desired amount of DNA or Hb in deionized water. The Hb stock solution was homogenized by placing it on an orbital shaker at 70 rpm.

The DNA stock solution was homogenized in two different ways: The first method involved dissolving the DNA by heating the solution to 37 °C. The second method involved dissolving the DNA by adding DNA powder to a 0.5 M NaOH solution (Sodium hydroxide; Sigma-Aldrich S5881).

Three different set of samples were prepared based on the DNA stock solution used and the final concentration:
C samples: A specific amount of DNA stock solution, which had been homogenized at 37 °C (25% w/v concentration), was added to a specific amount of Hb stock solution (25% w/v concentration) to obtain the desired weight percentages of DNA:Hb at ratios of 3:1, 1:1, and 1:3 (C1: 3:1, C2: 1:1, and C3: 1:3 ratios).
C' samples: The same protocol as for the C samples was followed, with the difference being that the DNA stock solution was homogenized using a 0.5 M NaOH solution. After mixing the DNA with Hb, the samples were cured at 60 °C for 1 hour.
C" samples: The same protocol as for the C' samples was followed, with the difference being that the DNA and Hb stock solutions were diluted with a ½ dilution factor using deionized water, without curing at 60 °C. The particles were collected through centrifugation at 2500 rpm for 15 minutes.

### Physicochemical characterizations of condensates

The confirmation of samples was captured using Atomic Force Microscopy (AFM). The AFM system used was a JPK Nanowizard 4, which was equipped with pyramidal silicon cantilevers specifically designed for alternating contact mode imaging. These cantilevers had a resonance frequency of 75 kHz and a stiffness of 3 N/m (Model: MPP-21 220, Manufacturer: Bruker). After the condensates were absorbed onto the mica surface, the surface was thoroughly washed with deionized water. Following this, the surface was air-dried before proceeding with the imaging process.

The morphology of the condensates, post freeze-drying and gold sputter coating, was analysed using a Scanning Electron Microscope (SEM). The specific SEM model used was the JEOL IT100 SEM. This analysis was conducted at an accelerating voltage of 10 kV, utilizing the Secondary Electron Detector (SED) mode.

Samples of C", diluted 10 times, were used for analysis via Transmission Electron Microscopy (TEM). The specific TEM model used was the JEOL JEM-1400 FLASH TEM. This analysis was conducted at a high tension (HT) voltage of 80 kV. 5 µl droplets were drop cast onto 400 mesh carbon coated copper grids which were previously Glow Discharged (Quorum Q150T ES, settings 20 mA 30 secs). Following surface absorption, they were then left to air-dry prior to the analysis. Tif format images captured using TEM CENTER software version 1.7.26.3016 and JEOL FLASH CCD camera

X-ray Diffraction (XRD) analysis of freeze-dried samples was performed using a Malvern Panalytical Empyrean multi-purpose platform. This platform was equipped with a Cu sealed tube X-ray source, multicore iCore/dCore motorised optics, a hybrid monochromator, a focusing mirror, and a PIXcel3D detector.

The analysis was conducted under the following conditions:
X-ray Photoelectron Spectroscopy (XPS) analysis of freeze-dried samples was conducted using an AXIS Supra+ XPS surface analysis instrument. This instrument is equipped with a large 500 mm Rowland circle monochromated Al Kα X-ray source and optimised electron optics. These features ensure unambiguous chemical resolution for both insulating and conducting samples. The analysis was conducted under the following conditions:

### Biological characterizations

Cell culture: In this study, all experiments were conducted using Human Bone Marrow-Derived Mesenchymal Stem Cells (hMSCs) (PromoCell GmbH, Germany, PromoCell, C-14090). The cells were cultured in Dulbecco's Modified Eagle's Media (DMEM) (Sigma-Aldrich, D5671), which was supplemented with the following:
- 10% Fetal Bovine Serum (FBS; Sigma-Aldrich, 26010)
- 2% mixture of antibiotics (6.74 U mL-1 penicillin, 6.74 µg mL-1 streptomycin; Penicillin-Streptomycin (Sigma-Aldrich, P0781), and 0.2 µg mL-1 Amphotericin B (fungizone); Gibco, 15290-026)
- 1% Non-Essential Amino Acids Solution (MEM NEAA; Gibco, 11140-035)
- 1% L-Glutamine Solution (200 mM, Sigma-Aldrich, G7513)
- 1% Sodium Pyruvate Solution (100 mM, Sigma-Aldrich, S8636)

The cells were incubated in a humidified incubator with a 5% CO2 atmosphere at 37 °C. The culture medium was replaced every other day. For differentiation-related experiments, cells were cultured at passage 2, and for live/dead experiments, they were cultured at passage 3.

Trypsin-EDTA solution (TE; Sigma-Aldrich, T4049) was used to detach cells from culture flask Dulbecco's Phosphate Buffered Saline (DPBS), without calcium and magnesium (Gibco, 14190-094) and Trypan Blue stain (0.4%; Gibco, 15250-061), was also used during washing the cells and staining process.

Live/dead assay: Following the culturing of cells over various days, a LIVE/DEAD assay was performed. This process involved washing the cells with DPBS and then adding 4 µM of Calcein, AM (Invitrogen, C3099) and 2 µM of Ethidium Homodimer-1 (EthD-1) (Invitrogen, E1169) to the samples. The cells were then incubated at 37 °C for 15 minutes.

After another round of washing with DPBS, the samples were imaged using an Axio Observer Z1 Epifluorescence Inverted Microscope (Zeiss, GmbH, Germany) and a Zeiss LSM 980 Confocal Microscope with Calcein (Ex. 493 nm, Em. 514 nm) and Ethidium homodimer (Ex. 528 nm, Em. 617 nm) channels. The images were subsequently analyzed using Fiji ImageJ. For each replicate, ten images were recorded. This process was repeated for triplicated samples in each group.

Osteogenic differentiation and immunofluorescence: C3" nanoparticles and Bone morphogenetic protein-2 (BMP-2) releasing bacteria was used to induce osteogenic differentiation of hMSC for 3, 10 and 16 days. Four different conditions was used in triplicate repetition for osteogenic studies as follow:
- BMP-2: Media containing 100ng mL-1 BMP-2 (positive control).
- NP: Media containing C3" nanoparticles
- Bac: Media containing BMP-2 releasing bacteria (Engineered Lactococcus lactis (Engineered L. lactis NZ9020) was used in this study and all parameters related to the bacteria were the same as the reference [529])
- NP-Bac: Media containing C3" nanoparticles and BMP-2 releasing bacteria

Cells were cultured at a density of 30,000 cells per well. This was done in a 24-well cell culture plate that had not been treated. The plate was covered with UV-treated glass slips. For immunostaining529, the treated cells were initially rinsed twice with DPBS and then stabilized with a 4% paraformaldehyde (PFA) solution in DPBS at ambient temperature for a quarter of an hour. The fixative solution was subsequently discarded, and the specimens were rinsed thrice with DPBS. Following this, the cells were permeabilized with a 0.1% Triton X-100 solution in DPBS for a duration of 5 minutes. To prevent non-specific binding, the samples were blocked by incubating them in a 2% DBSA solution in DPBS for half an hour at 37 °C.

Subsequently, the treated cells were incubated with an anti-osteopontin rabbit primary polyclonal antibody (Jackson ImmunoResearch Laboratories Inc., USA, 315,165,003) at a ratio of 1:150 in a 2% BSA solution in DPBS for an hour at 37 °C. The samples were then rinsed to eliminate any unbound primary antibody with a 1% Tween 20 solution in PBS, repeated thrice for 5 minutes each.

The cells were later incubated with a goat anti-rabbit secondary antibody (Jackson ImmunoResearch Laboratories Inc., USA, 111165003) and Alexa Fluor 488 Phalloidin (Invitrogen',Thermofisher Scientific, U.K., A12379) at ratios of 1:200 and 1:100 in a 2% BSA solution in PBS, respectively, for an hour at room temperature in a light-free environment. Following this, the samples were rinsed thrice with a 1% Tween 20 solution in PBS for 5 minutes each.

The nuclei of hMSCs, bacteria, and DNA within adsorbed/permeated nanoparticles were stained using a Vectashield-DAPI mounting medium (Vector Laboratories, USA). Following this, the cells were imaged with a 20x 0.8NA lens LSM 900 confocal microscope (Zeiss, GmbH, Germany).

Different channels were used for different components:
- DAPI channel (Ex. 358 nm, Em. 463 nm) was used for the nuclei/DNA, which appear blue.
- Cy3 (Ex. 549 nm, Em. 562 nm) was used for Osteopontin (OPN), which appears orange.
- Alexa Fluor 488 (Ex. 493 nm, Em. 517 nm) was used for actin, which appears green.

The captured images were in Zeiss .czi multichannel format, taken with a 20x 0.8NA lens on an LSM 900 microscope, and analyzed using Fiji ImageJ.

### Statistical analysis

Each condition was repeated three times for biological studies and four times for chemical and physical independent samples. Statistical analyses were conducted using GraphPad Prism 10 software and Excel 2016.

### Results and discussion

### Engineering size, structure, and mechanic

Upon the initial attempt to mix DNA and Hb solutions, an exceedingly rapid condensation occurred, resulting in a dense and opaque substance. Initially, various DNA and Hb combinations were tested, revealing that an increased DNA ratio correlates with greater material density.

Subsequently, to achieve a softer gel with enhanced transparency, denaturation was incorporated into the procedure. Utilizing identical combinations but elevating the pH dramatically altered the condensates' behavior, yielding much softer, paste-like materials. The observed shifts in mechanorheological and optical properties, contingent on the ratio and extent of denaturation, necessitated an investigation using the analytical capabilities of Scanning Electron Microscopy (SEM), Atomic Force Microscopy (AFM), and Transmission Electron Microscopy (TEM).

The Atomic Force Microscopy (AFM) images of the C2 sample, with a DNA:Hb ratio of 1:1, were captured both in the absence (C2) and presence of denaturation (C2'). The C2 samples exhibited clusters of nanoparticles with an average diameter of approximately 155 nm and smaller, twisted nanocondensates measuring around 38 nm (refer to Figure 13.b). In contrast, the twisted nanoparticles in C2' displayed a more uniform size distribution, averaging about 27 nm.

For a broader perspective, SEM images of various samples were spotlighted (see Figure 13.a). In the C1 sample, particles ranging from roughly 100 nm to clusters spanning 0.5-2.0 µm were evident, characterized by a dense micro-tile texture interspersed with nanoparticles. Conversely, the C1' sample, at the microscale, revealed a starkly different structure of interconnected pores approximately 63 µm in size, composed of brick-like particles ranging from 200-500 nm or orbit-like particles with dimensions of (300, 500) nm. The anisotropic growth of condensates in both C1 and C1' samples is attributed to the higher DNA ratio, with the notable distinction that C1, compared to C1', displayed increased density and anisotropy, likely due to the denaturation of DNA in C1'.

Reducing the DNA ratio in the C2 samples resulted in roundish particles varying from about 130 nm to 1.5 µm, which formed pores approximately 1-5 µm in size. However, the particles in C2' were more uniform, with an average size of about 168 nm, constructing interconnected pores around 526 nm in size. Moreover, C2' at the microscale showcased interconnected pores with an average size of about 52 µm, whereas C2 was characterized by a denser structure with discrete planes.

As the proportion of Hb became predominant, the interconnected particles in both C3 and C3' samples achieved greater uniformity and lower densities compared to other samples, with sizes ranging from 200-700 nm and pores of 1-4 µm for C3, and approximately 130 nm diameter with pores of 0.5-3.5 µm for C3'. Additionally, C3' presented a pore size of about 58 µm in a broader view, while C3 maintained a denser composition with significantly fewer pores.

In summary, the analyzed images suggest that an increased DNA ratio augments anisotropy in the samples, possibly due to enhanced DNA stacking interactions. Conversely, denaturation promotes homogeneity and isotropy in both particle and pore sizes. Furthermore, denaturation or an increased Hb ratio appears to reduce the density of the samples and the size of the particles, presumably owing to the diminished stacking of DNA and DNA-Hb interactions, as well as the neutralization of DNA charge due to denaturation and the elevated ionic strength of the medium.

The query regarding the whereabouts of the particles, specifically those within the 20-40 nm range as depicted by AFM, warrants critical examination. To enhance visibility and mitigate the likelihood of particle co-condensation, the diluted C3' samples-designated as C3"-were subjected to TEM analysis. This investigation revealed particles with dimensions spanning from 15 to 60 nm. It is imperative to scrutinize the discrepancy in particle size distribution between the AFM and TEM observations, as well as to consider the implications of dilution on the observed particle behavior and characteristics.

The evidence gleaned from SEM and AFM images-specifically, the clustering phenomena, the disparate anisotropic behaviors, and edge effects observed across samples-coupled with the noted increase in transparency and softness in the denatured specimens, compels a more profound investigation into their structural and electronic properties. It is essential to delve into X-ray Diffraction (XRD) and X-ray Photoelectron Spectroscopy (XPS) analyses to elucidate how the crystallinity and electronic band structures are influenced by the interplay of DNA-Hb interactions, the condensation ratio, the process of denaturation, and the effects of ionic neutralization. Such an in-depth study is critical to comprehending the underlying mechanisms that govern the observed physical changes and to predict the behavior of these complex systems under varying experimental conditions.

The analysis of dsDNA powders revealed a pronounced shoulder in the full width at half maximum (FWHM) region, approximately 10.4°, with a 2θ peak at 20.8° corresponding to a d-spacing of 0.43 nm at a first-order reflection. Additionally, distinct, sharp-shorter peaks at 31.7° (d 0.28 nm), 40.8° (d 0.22 nm), and 45.6° (d 0.20 nm) emerged on a short wide shoulder commencing at 60.6° (d 0.15 nm). In contrast, Hb powders exhibited two more defined shoulders with narrower FWHMs of about 5.6° at a peak of 20.0° (d 0.44 nm) and 3.8° at a peak of 9.2° (d 0.96 nm).

For compound C1, the peak centered at 19.2° became more constricted, indicating larger crystallite sizes, and shifted to the left when compared to the peaks of DNA and Hb, suggesting an increased d-spacing. However, C1' displayed three sharp peaks at the highest intensity, yet the collective shoulders were broader than those of C1, indicating a greater polydispersity in crystallite sizes and d-spacing, as corroborated by SEM images. An increase in the Hb ratio or denaturation caused the peak at the highest intensity to split into three sub-peaks: one centered at the highest intensity DNA peak, with the other two being left and right-shifted and more defined than those of DNA and Hb. This suggests alterations in both the d-spacing and the crystallinity sizes. All denatured samples exhibited broader shoulders centered at 42.8°, which had smaller crystallite sizes, in contrast to the denser, undenatured samples. Furthermore, the denatured samples all presented a left-shifted peak centered at 8.7° with an increased intensity relative to the peak centered at 19.3°, compared to Hb and corresponding denatured samples. This is likely indicative of a reduced density of Hb in condensed states and diminished interactions with DNA.

In summary, a decrease in the DNA ratio and an increase in denaturation led to a rise in the number of peaks and their polydispersity, introducing greater entropy into the systems. Conversely, narrower shoulders and the augmented incorporation of Hb in the structures contributed to enhanced crystallinity, stronger intermolecular forces, and a higher density of condensates. This could be attributed to the more compact nature of the neat samples relative to the denatured samples, as evident in SEM and AFM imaging. The critical examination of these patterns suggests that the structural integrity and intermolecular interactions within these compounds are significantly influenced by the ratio of DNA to Hb and the degree of denaturation, with potential implications for their functional properties in biological systems.

In the comprehensive analysis of XPS spectra, as delineated in Table 2, a discernible trend emerges: a reduction in DNA ratio or an augmentation in denaturation correlates with an increase in both the standard deviation (SD) of binding energy (BE) and the full width at half maximum (FWHM) of elemental orbitals. This trend signifies an expansion in the diversity of electron densities, which aligns with the polydispersity of interactions previously inferred from XRD analyses. Notably, this polydispersity reaches its zenith in the C2 condensate, while the C1 condensate exhibits the nadir in comparison to other condensates.

The mean BE and FWHM values for Hb across all C1s, O1s, N1s, and P2p levels are consistently lower than those observed in DNA. The C1 sample, in particular, displays a BE equivalent to that of DNA, with an FWHM that straddles the values between DNA and Hb for all aforementioned levels. Conversely, the C2 and C3 samples exhibit divergent trends: the mean BE in the C2 condensate is diminished, whereas the C3 condensate's BE is elevated in comparison to both DNA and Hb. The FWHM values in these samples generally exhibit an uptick relative to the FWHM values in DNA and Hb.

Examining the denatured samples, C1' and C2' maintain a consistent trend, with the BE of N1s and O1s nestled between the corresponding BE values of DNA and Hb, while the BE of C1s and P2p surpasses that of DNA and Hb. The C3' sample stands apart, with the N1s BE exceeding that of both DNA and Hb. Across all analyzed C' samples, the FWHM trends are uniform: the FWHM values for O1s, N1s, and P2p exceed those in DNA and Hb, whereas the FWHM for C1s lies intermediate.

When juxtaposing related levels in denatured and neat condensates within the wide XPS spectra, C1' and C3' parallel the trends observed in C1 and C3 samples, respectively. Specifically, the BE of C1s and P2p exhibits an increment, while the BE of O1s and N1s demonstrates a decrement. However, all levels in C2' reveal an escalation in BE and a contraction in FWHM compared to C2. Both C1' and C3' manifest an amplification in FWHM vis-à-vis the FWHM in C1 and C3 samples. The SD of BE and FWHM unveils intriguing patterns: the SD for both parameters intensifies for C1' relative to C1, diminishes for C2' in contrast to C2, and remains approximately invariant for C3' as compared to C3.

This meticulous scrutiny of the XPS spectra suggests that alterations in the DNA ratio and the degree of denaturation or ionic concentration profoundly influence the electronic environment of the condensates, as evidenced by the variability in BE and FWHM. These changes are indicative of the underlying structural dynamics and are reflective of the complex interplay between molecular interactions and electronic properties within these systems.

**Table 2. Binding energies, full width at half-maximum (FWHM), relative sensitivity factors (RSF) and atomic concentrations of different freeze dried samples (CX'; X; 3DNA:1Hb, 2: 1DNA:1Hb, 3: 1DNA:3Hb; ' for denatured samples) in wide scan of XPS spectra.**

| **DNA** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.060 | 0.000 | 4 | 3.178 | 0.140 | 4 | 1.690 | 0.000 | 4 | 0.900 | 0.082 | 4 |
| C 1s | 285.060 | 0.000 | 4 | 4.320 | 0.014 | 4 | 0.280 | 0.000 | 4 | 58.275 | 0.126 | 4 |
| O 1s | 532.060 | 0.000 | 4 | 3.538 | 0.010 | 4 | 0.780 | 0.000 | 4 | 23.700 | 0.082 | 4 |
| N 1s | 400.060 | 0.000 | 4 | 3.085 | 0.025 | 4 | 0.480 | 0.000 | 4 | 13.375 | 0.206 | 4 |
| P 2p | 133.060 | 0.000 | 4 | 2.695 | 0.031 | 4 | 0.490 | 0.000 | 4 | 3.325 | 0.050 | 4 |
| Si 2p | 101.310 | 0.500 | 4 | 2.758 | 0.515 | 4 | 0.330 | 0.000 | 4 | 0.425 | 0.171 | 4 |

| **C1** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.060 | 0.000 | 4 | 2.620 | 0.054 | 4 | 1.690 | 0.000 | 4 | 0.550 | 0.100 | 4 |
| C 1s | 285.060 | 0.000 | 4 | 4.105 | 0.024 | 4 | 0.280 | 0.000 | 4 | 62.400 | 0.408 | 4 |
| O 1s | 532.060 | 0.000 | 4 | 3.425 | 0.026 | 4 | 0.780 | 0.000 | 4 | 21.400 | 0.245 | 4 |
| N 1s | 400.060 | 0.000 | 4 | 3.025 | 0.017 | 4 | 0.480 | 0.000 | 4 | 12.575 | 0.206 | 4 |
| P 2p | 133.060 | 0.000 | 4 | 2.715 | 0.042 | 4 | 0.490 | 0.000 | 4 | 2.450 | 0.129 | 4 |
| Si 2p | 101.060 | 0.000 | 4 | 2.573 | 0.122 | 4 | 0.330 | 0.000 | 4 | 0.675 | 0.263 | 4 |

| **C2** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| C 1s | 284.000 | 1.414 | 4 | 5.308 | 0.979 | 4 | 0.280 | 0.000 | 4 | 68.225 | 0.854 | 4 |
| O 1s | 531.000 | 1.414 | 4 | 4.855 | 1.187 | 4 | 0.780 | 0.000 | 4 | 17.325 | 1.050 | 4 |
| Si 2p | 100.750 | 1.500 | 4 | 5.873 | 2.780 | 4 | 0.330 | 0.000 | 4 | 0.950 | 0.507 | 4 |
| P 2p | 132.000 | 1.414 | 4 | 4.415 | 1.352 | 4 | 0.490 | 0.000 | 4 | 2.025 | 0.171 | 4 |
| N 1s | 398.500 | 1.732 | 4 | 4.413 | 0.786 | 4 | 0.480 | 0.000 | 4 | 11.500 | 0.374 | 4 |

| **C3** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| C 1s | 285.400 | 0.000 | 4 | 4.175 | 0.423 | 4 | 0.280 | 0.000 | 4 | 69.875 | 2.438 | 4 |
| Na 1s | 1071.900 | 0.577 | 4 | 4.008 | 1.433 | 4 | 1.690 | 0.000 | 4 | 0.425 | 0.050 | 4 |
| O 1s | 532.150 | 0.500 | 4 | 3.585 | 0.376 | 4 | 0.780 | 0.000 | 4 | 15.250 | 0.843 | 4 |
| N 1s | 400.400 | 0.000 | 4 | 3.370 | 0.335 | 4 | 0.480 | 0.000 | 4 | 10.700 | 1.598 | 4 |
| Si 2p | 102.400 | 0.000 | 4 | 4.103 | 1.760 | 4 | 0.330 | 0.000 | 4 | 1.750 | 1.502 | 4 |
| P 2p | 133.650 | 0.500 | 4 | 3.275 | 0.481 | 4 | 0.490 | 0.000 | 4 | 0.900 | 0.141 | 4 |

| **C1'** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.060 | 0.000 | 4 | 2.838 | 0.353 | 4 | 1.690 | 0.000 | 4 | 1.900 | 0.082 | 4 |
| C 1s | 285.310 | 0.500 | 4 | 4.245 | 0.344 | 4 | 0.280 | 0.000 | 4 | 61.525 | 1.050 | 4 |
| O 1s | 531.810 | 0.500 | 4 | 3.765 | 0.317 | 4 | 0.780 | 0.000 | 4 | 21.775 | 0.866 | 4 |
| N 1s | 399.310 | 0.500 | 4 | 3.418 | 0.390 | 4 | 0.480 | 0.000 | 4 | 11.800 | 0.542 | 4 |
| P 2p | 133.310 | 0.500 | 4 | 2.908 | 0.515 | 4 | 0.490 | 0.000 | 4 | 2.800 | 0.346 | 4 |
| Si 2p | 102.060 | 1.000 | 3 | 2.427 | 0.951 | 3 | 0.330 | 0.000 | 3 | 0.267 | 0.058 | 3 |

| **C2'** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.180 | 0.000 | 4 | 2.983 | 0.415 | 4 | 1.690 | 0.000 | 4 | 1.375 | 0.096 | 4 |
| C 1s | 285.180 | 0.000 | 4 | 4.210 | 0.228 | 4 | 0.280 | 0.000 | 4 | 65.825 | 0.538 | 4 |
| O 1s | 531.930 | 0.500 | 4 | 3.620 | 0.130 | 4 | 0.780 | 0.000 | 4 | 18.675 | 0.395 | 4 |
| N 1s | 399.930 | 0.500 | 4 | 3.335 | 0.219 | 4 | 0.480 | 0.000 | 4 | 11.325 | 0.900 | 4 |
| Si 2p | 101.847 | 0.577 | 3 | 2.803 | 0.121 | 3 | 0.330 | 0.000 | 3 | 0.967 | 0.379 | 3 |
| P 2p | 133.180 | 0.000 | 4 | 3.070 | 0.395 | 4 | 0.490 | 0.000 | 4 | 2.075 | 0.263 | 4 |

| **C3'** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.730 | 0.577 | 4 | 3.130 | 0.481 | 4 | 1.690 | 0.000 | 4 | 0.800 | 0.082 | 4 |
| C 1s | 285.480 | 0.500 | 4 | 4.280 | 0.470 | 4 | 0.280 | 0.000 | 4 | 70.025 | 0.512 | 4 |
| O 1s | 531.980 | 0.500 | 4 | 3.605 | 0.287 | 4 | 0.780 | 0.000 | 4 | 15.600 | 0.542 | 4 |
| N 1s | 400.230 | 0.000 | 4 | 3.390 | 0.343 | 4 | 0.480 | 0.000 | 4 | 12.050 | 0.507 | 4 |
| Si 2p | 102.230 | 1.000 | 3 | 3.020 | 0.284 | 3 | 0.330 | 0.000 | 3 | 0.667 | 0.208 | 3 |
| P 2p | 133.730 | 0.577 | 4 | 3.413 | 0.583 | 4 | 0.490 | 0.000 | 4 | 1.025 | 0.096 | 4 |

| **Hb** | | BE [eV] | | FWHM [eV] | | | RSF | | | Atomic conc. [%] | | |
|---|---|---|---|---|---|---|---|---|---|---|---|---|
| | Mean | SD | N | Mean | SD | N | Mean | SD | N | Mean | SD | N |
| Na 1s | 1071.060 | 0.000 | 4 | 2.683 | 0.259 | 4 | 1.690 | 0.000 | 4 | 0.475 | 0.050 | 4 |
| C 1s | 284.060 | 0.000 | 4 | 3.243 | 0.043 | 4 | 0.280 | 0.000 | 4 | 74.975 | 0.222 | 4 |
| O 1s | 531.060 | 0.000 | 4 | 2.908 | 0.030 | 4 | 0.780 | 0.000 | 4 | 13.150 | 0.058 | 4 |
| N 1s | 399.060 | 0.000 | 4 | 2.698 | 0.057 | 4 | 0.480 | 0.000 | 4 | 10.625 | 0.096 | 4 |
| P 2p | 133.060 | 0.000 | 4 | 2.573 | 0.381 | 4 | 0.490 | 0.000 | 4 | 0.275 | 0.096 | 4 |
| Si 2p | 101.560 | 0.577 | 4 | 2.675 | 0.240 | 4 | 0.330 | 0.000 | 4 | 0.475 | 0.206 | 4 |

Upon examining Table 2 and Figure. 17, which details the core levels of various orbitals in regional XPS spectra, the alterations in the electronic states of different condensates become markedly pronounced. For instance, a focused analysis of the P 2p and N1s spectra reveals that a decrease in the DNA ratio and an increase in denaturation-ionic strength are associated with a reduction in peak energy for P 2p and N1s. This trend is consistent across the board, with the exception of the C3' sample, where the P 2p peak energies exhibit an increase, and the N1s peak energy remains unchanged in comparison to the C3 sample.

Furthermore, the area ratio and electron densities of P 2p1/2 to P 2p3/2 undergo a reversal from a 1:2 ratio in the DNA-C1-C1'-C3' samples to a 2:1 ratio in the C2-C2'-C3 condensates. This shift may be influenced by the presence of P p orbitals from other compounds that overlap with the P 2p1/2 peak. The spin-orbit splitting of the P 2p1/2 and P 2p3/2 peaks follows a specific sequence: C1 is approximately equal to DNA, which is greater than C1', followed by C3', then C3, with C2 and C2' exhibiting the least splitting. This sequence mirrors the categorization of the area ratio.

In essence, the core level spectroscopy provides a window into the changes in electronic structure as a function of DNA content and the degree of denaturation. These shifts in peak energies and area ratios are indicative of the complex correlation between molecular composition and electronic properties, offering insights into the underlying mechanisms governing the dynamic behavior of these condensates.

**Table 3. Peak energies, full width at half-maximum (FWHM), relative sensitivity factors (RSF) and area of different element states in the components modelled of the region scans in XPS spectra of different samples (CX'; X; 3DNA:1Hb, 2: 1DNA:1Hb, 3: 1DNA:3Hb; ' for denatured samples)**

| DNA | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| P 2p 133.4 | 133.38 | 0.01 | 4 | 1.45 | 0.03 | 4 | 0.49 | 0.00 | 4 | 66.67 | 0.00 | 4 |
| P 2p 134.1 | 134.33 | 0.01 | 4 | 1.46 | 0.02 | 4 | 0.49 | 0.00 | 4 | 33.33 | 0.00 | 4 |
| N 1s 400.5 | 400.11 | 5.00e-003 | 4 | 2.28 | 0.01 | 4 | 0.48 | 0.00 | 4 | 100.00 | 0.00 | 4 |
| C 1s C-C | 284.77 | 0.01 | 4 | 1.39 | 0.01 | 4 | 0.28 | 0.00 | 4 | 43.65 | 0.35 | 4 |
| C 1s C-O | 286.39 | 5.00e-003 | 4 | 1.39 | 0.01 | 4 | 0.28 | 0.00 | 4 | 34.04 | 0.08 | 4 |
| C 1s C=O | 287.79 | 0.02 | 4 | 1.39 | 0.01 | 4 | 0.28 | 0.00 | 4 | 16.05 | 0.20 | 4 |
| C 1s O-C=O | 289.03 | 0.03 | 4 | 1.39 | 0.01 | 4 | 0.28 | 0.00 | 4 | 6.27 | 0.16 | 4 |
| O 1s C-O | 532.60 | 0.01 | 4 | 1.81 | 0.02 | 4 | 0.78 | 0.00 | 4 | 63.41 | 0.79 | 4 |
| O 1s C=O | 531.01 | 0.02 | 4 | 1.54 | 0.02 | 4 | 0.78 | 0.00 | 4 | 36.59 | 0.79 | 4 |

| C1 | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| P 2p 133.5 | 134.27 | 0.02 | 4 | 1.54 | 0.05 | 4 | 0.49 | 0.00 | 4 | 33.33 | 0.00 | 4 |
| P 2p 132.8 | 133.31 | 0.02 | 4 | 1.43 | 0.03 | 4 | 0.49 | 0.00 | 4 | 66.67 | 0.00 | 4 |
| N 1s 400.3 | 400.03 | 0.01 | 4 | 2.12 | 0.01 | 4 | 0.48 | 0.00 | 4 | 100.00 | 0.00 | 4 |
| C 1s C-C | 284.71 | 0.01 | 4 | 1.38 | 0.01 | 4 | 0.28 | 0.00 | 4 | 49.22 | 0.15 | 4 |
| C 1s C-O | 286.29 | 0.01 | 4 | 1.38 | 0.01 | 4 | 0.28 | 0.00 | 4 | 30.34 | 0.11 | 4 |
| C 1s C=O | 287.82 | 0.02 | 4 | 1.38 | 0.01 | 4 | 0.28 | 0.00 | 4 | 15.90 | 0.13 | 4 |
| C 1s O-C=O | 289.02 | 0.03 | 4 | 1.38 | 0.01 | 4 | 0.28 | 0.00 | 4 | 4.54 | 0.17 | 4 |
| O 1s C=O | 531.10 | 0.02 | 4 | 1.59 | 0.03 | 4 | 0.78 | 0.00 | 4 | 39.55 | 2.55 | 4 |
| O 1s C-O | 532.50 | 0.04 | 4 | 1.82 | 0.06 | 4 | 0.78 | 0.00 | 4 | 59.86 | 3.24 | 4 |

| **C2** | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| Phosphate P2p3/2 | 133.14 | 0.02 | 4 | 1.15 | 0.04 | 4 | 0.49 | 0.00 | 4 | 33.33 | 0.00 | 4 |
| Phosphate P2p1/2 | 133.90 | 0.02 | 4 | 1.66 | 0.06 | 4 | 0.49 | 0.00 | 4 | 66.67 | 0.00 | 4 |
| N 1s C-NH2 | 400.06 | 0.02 | 4 | 1.84 | 0.02 | 4 | 0.48 | 0.00 | 4 | 100.00 | 0.00 | 4 |
| C 1s C-C | 284.81 | 0.02 | 4 | 1.36 | 0.01 | 4 | 0.28 | 0.00 | 4 | 55.21 | 0.80 | 4 |
| C 1s C-O | 286.32 | 0.02 | 4 | 1.36 | 0.01 | 4 | 0.28 | 0.00 | 4 | 26.82 | 0.55 | 4 |
| C 1s O-C=O | 287.91 | 0.03 | 4 | 1.36 | 0.01 | 4 | 0.28 | 0.00 | 4 | 14.43 | 0.43 | 4 |
| C 1s C=O | 288.98 | 0.07 | 4 | 1.36 | 0.01 | 4 | 0.28 | 0.00 | 4 | 3.54 | 0.50 | 4 |
| O 1s C=O | 531.25 | 0.04 | 4 | 1.60 | 0.03 | 4 | 0.78 | 0.00 | 4 | 43.94 | 3.12 | 4 |
| O 1s C-O | 532.57 | 0.05 | 4 | 1.89 | 0.03 | 4 | 0.78 | 0.00 | 4 | 56.06 | 3.12 | 4 |

| C3 | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| Phosphate P 2p1/2 | 133.71 | 0.07 | 4 | 1.65 | 0.20 | 4 | 0.49 | 0.00 | 4 | 66.67 | 0.00 | 4 |
| Phosphate P 2p3/2 | 132.92 | 0.12 | 4 | 1.14 | 0.14 | 4 | 0.49 | 0.00 | 4 | 33.33 | 0.00 | 4 |
| N 1s C-NH2 | 399.86 | 0.11 | 4 | 1.67 | 0.08 | 4 | 0.48 | 0.00 | 4 | 100.00 | 0.00 | 4 |
| C 1s C-C | 284.66 | 0.08 | 4 | 1.41 | 0.03 | 4 | 0.28 | 0.00 | 4 | 60.59 | 2.73 | 4 |
| C 1s C-O | 286.16 | 0.09 | 4 | 1.41 | 0.03 | 4 | 0.28 | 0.00 | 4 | 24.12 | 1.50 | 4 |
| C 1s C=O | 287.96 | 0.10 | 4 | 1.41 | 0.03 | 4 | 0.28 | 0.00 | 4 | 15.29 | 1.23 | 4 |
| O 1s C=O | 531.40 | 0.20 | 4 | 1.75 | 0.17 | 4 | 0.78 | 0.00 | 4 | 68.75 | 9.29 | 4 |
| O 1s C-O | 532.55 | 0.14 | 4 | 1.85 | 0.09 | 4 | 0.78 | 0.00 | 4 | 31.25 | 9.29 | 4 |

| **C1'** | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| Phosphate P2p3/2 | 133.17 | 0.06 | 4 | 1.40 | 0.09 | 4 | 0.49 | 0.00 | 4 | 66.67 | 0.00 | 4 |
| Phosphate P2p1/2 | 134.10 | 0.06 | 4 | 1.48 | 0.15 | 4 | 0.49 | 0.00 | 4 | 33.33 | 0.00 | 4 |
| N 1s C-NH2 | 400.05 | 0.07 | 4 | 1.94 | 0.04 | 4 | 0.48 | 0.00 | 4 | 77.79 | 3.15 | 4 |
| N 1s NSiO2/NSi3 | 398.73 | 0.05 | 4 | 1.39 | 0.13 | 4 | 0.48 | 0.00 | 4 | 22.21 | 3.15 | 4 |
| C 1s C-C | 284.67 | 0.09 | 4 | 1.43 | 0.06 | 4 | 0.28 | 0.00 | 4 | 49.29 | 1.18 | 4 |
| C 1s C-O | 286.26 | 0.12 | 4 | 1.43 | 0.06 | 4 | 0.28 | 0.00 | 4 | 30.75 | 0.30 | 4 |
| C 1s C=O | 287.74 | 0.11 | 4 | 1.43 | 0.06 | 4 | 0.28 | 0.00 | 4 | 15.17 | 0.54 | 4 |
| C 1s O-C=O | 288.97 | 0.07 | 4 | 1.43 | 0.06 | 4 | 0.28 | 0.00 | 4 | 4.79 | 0.40 | 4 |
| O 1s C-O | 532.47 | 0.09 | 4 | 1.77 | 0.07 | 4 | 0.78 | 0.00 | 4 | 54.09 | 0.47 | 4 |
| O 1s C=O | 530.94 | 0.08 | 4 | 1.62 | 0.09 | 4 | 0.78 | 0.00 | 4 | 41.13 | 0.30 | 4 |

| **C2'** | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| Phosphate P2p3/2 | 133.12 | 0.07 | 4 | 1.18 | 0.10 | 4 | 0.49 | 0.00 | 4 | 33.33 | 0.00 | 4 |
| Phosphate P2p1/2 | 133.83 | 0.08 | 4 | 1.70 | 0.08 | 4 | 0.49 | 0.00 | 4 | 66.67 | 0.00 | 4 |
| N 1s 400.2 | 399.91 | 0.05 | 4 | 2.12 | 0.01 | 4 | 0.48 | 0.00 | 4 | 100.00 | 0.00 | 4 |
| C 1s C-C | 284.79 | 0.05 | 4 | 1.43 | 0.04 | 4 | 0.28 | 0.00 | 4 | 56.64 | 0.54 | 4 |
| C 1s C-O | 286.40 | 0.05 | 4 | 1.43 | 0.04 | 4 | 0.28 | 0.00 | 4 | 27.77 | 0.52 | 4 |
| C 1s C=O | 288.08 | 0.04 | 4 | 1.43 | 0.04 | 4 | 0.28 | 0.00 | 4 | 15.58 | 0.27 | 4 |
| O 1s C=O | 531.26 | 0.06 | 4 | 1.72 | 0.01 | 4 | 0.78 | 0.00 | 4 | 58.60 | 2.05 | 4 |
| O 1s C-O | 532.67 | 0.07 | 4 | 1.58 | 0.05 | 4 | 0.78 | 0.00 | 4 | 41.40 | 2.05 | 4 |

| **C3'** | Peak energy [eV] | | | FWHM [eV] | | | RSF | | | Area [%] | | |
|---|---|---|---|---|---|---|---|---|---|---|---|---|
| | Mean | SD | N | Mean | SD | N | Mean | SD | N | Mean | SD | N |
| Phosphate P2p3/2 | 133.19 | 0.06 | 4 | 1.42 | 0.10 | 4 | 0.49 | 0.00 | 4 | 66.67 | 0.00 | 4 |
| Phosphate P2p1/2 | 134.05 | 0.07 | 4 | 1.33 | 0.11 | 4 | 0.49 | 0.00 | 4 | 33.33 | 0.00 | 4 |
| N 1s C-NH2 | 399.86 | 0.06 | 4 | 1.84 | 0.06 | 4 | 0.48 | 0.00 | 4 | 100.00 | 0.00 | 4 |
| C 1s C-C | 284.71 | 0.04 | 4 | 1.48 | 0.06 | 4 | 0.28 | 0.00 | 4 | 57.93 | 1.06 | 4 |
| C 1s C-O | 286.21 | 0.06 | 4 | 1.48 | 0.06 | 4 | 0.28 | 0.00 | 4 | 25.32 | 0.51 | 4 |
| C 1s C=O | 287.98 | 0.07 | 4 | 1.48 | 0.06 | 4 | 0.28 | 0.00 | 4 | 16.75 | 0.56 | 4 |
| O 1s C=O | 531.29 | 0.06 | 4 | 1.74 | 0.06 | 4 | 0.78 | 0.00 | 4 | 69.18 | 4.55 | 4 |
| O 1s C-O | 532.64 | 0.04 | 4 | 1.68 | 0.14 | 4 | 0.78 | 0.00 | 4 | 30.82 | 4.55 | 4 |

| **Hb** | Peak energy [eV] | | | FWHM [eV] | | | RSF | | | Area [%] | | |
|---|---|---|---|---|---|---|---|---|---|---|---|---|
| | Mean | SD | N | Mean | SD | N ean | M | SD | N | Mean | SD | N |
| Phosphate P 2p | 133.36 | 0.03 | 4 | 1.74 | 0.05 | 4 | 0.49 | 0.00 | 4 | 100.00 | 0.00 | 4 |
| N 1s C-NH2 | 399.84 | 5.00e-003 | 4 | 1.48 | 0.02 | 4 | 0.48 | 0.00 | 4 | 96.04 | 0.42 | 4 |
| N 1s NSiO2 | 401.82 | 0.11 | 4 | 1.90 | 0.26 | 4 | 0.48 | 0.00 | 4 | 3.96 | 0.42 | 4 |
| C 1s C-C | 284.79 | 0.01 | 4 | 1.34 | 0.01 | 4 | 0.28 | 0.00 | 4 | 63.67 | 0.49 | 4 |
| C 1s C=O | 286.21 | 0.01 | 4 | 1.34 | 0.01 | 4 | 0.28 | 0.00 | 4 | 21.61 | 0.29 | 4 |
| C 1s O-C=O | 288.03 | 0.01 | 4 | 1.34 | 0.01 | 4 | 0.28 | 0.00 | 4 | 14.73 | 0.20 | 4 |
| O 1s C=O | 531.29 | 0.01 | 4 | 1.60 | 0.03 | 4 | 0.78 | 0.00 | 4 | 73.59 | 3.17 | 4 |
| O 1s C-O | 532.48 | 0.08 | 4 | 1.82 | 0.06 | 4 | 0.78 | 0.00 | 4 | 26.42 | 3.17 | 4 |

### Active living material supports differentiation and morphological changes

In addition to their modulated nanostructures and electronic dynamics, what other characteristics distinguish these condensates as unique? The use of Hb fluorescence as a probe for structural and entropic allosteric effectors is well-documented. Conversely, DNA's variable binding efficiency and the electronic properties of Hb endow it with the dynamic capacity to create an active probe ranging from the nano to the macro scale. The fundamental advantage of such materials lies in their ability to elucidate the topology of matter. As depicted in Figure 15. (a), the fluorescence of the C3' condensates reveals that micropores are more discernible when excited with wavelengths of 401 nm and 508 nm, corresponding to Alexa Fluor 405 (Ex. 401 nm, Em. 421 nm) and FM 4-64 (Ex. 508 nm, Em. 751 nm), respectively.

Furthermore, the intrinsic light-responsive properties of these condensates have spurred investigations into integrating their light communication capabilities with optically active engineered bacteria. This research aims to imbue the system with a collective intelligence and enable remote spatiotemporal control. Preliminary studies on the proliferation of this optogenetic gene expression system530, as shown in Figure 13. (b), have also shed light on the topographical landscape of the condensates. The objective is to harness the light-sensitive gene expression of these optically active bacteria in tandem with heme catalytic functions.

The viability of human mesenchymal stem cells (hMSCs) was monitored until day 7. Evidence from Figure 15. (c) indicates that as the DNA concentration increases in the C1' condensate, the cells tend to cluster. Moreover, an elevated Hb ratio in the C3' condensate correlates with enhanced cell proliferation compared to the C1' condensate.

To investigate the impact of condensate nanoparticles, both with and without BMP-2 releasing engineered bacteria, on cell morphology and differentiation, passage-2 human mesenchymal stem cells (hMSCs) were cultured under four distinct conditions: with BMP-2, C3' nanoparticles (NP), BMP-2 releasing engineered bacteria (Bac), and a combination of NP and Bac (NP-Bac), as illustrated in Figure 16.

Osteopontin (OPN) expression was observed across all conditions, even in media containing solely C3' NPs. This suggests that these condensates are not merely passive entities. A notable competition between NP and Bac was observed: NPs appeared to elongate the cells into fiber-like structures, whereas Bac promoted a more expansive cell morphology. The combined effect of these two vectors was evident in the NP-Bac samples when compared to the positive BMP-2 control.

These observations indicate that active NPs do more than merely stimulate OPN expression in hMSCs; they also alter cell morphology. Furthermore, their interaction with living bacteria imparts a greater degree of collective intelligence, enabling the precise sculpting of desired cellular patterns.

### Conclusion

Our study led to the creation of an Active-Living Biomaterial (ALB) that combines nano-engineered non-living materials with living cells. The ALB, a complex of DNA-Hemoglobin nano-condensates and engineered microbes, has demonstrated potential in modulating bone marrow stem cell behavior, which is pivotal for advancements in tissue engineering, regenerative medicine, and nanotechnology. The critical analysis of structural changes, as evidenced by SEM, AFM, WAXS, and XPS, reveals that the DNA-to-Hemoglobin ratio and the degree of denaturation are instrumental in dictating the ALS's properties. These findings underscore the ALB's capacity to influence cell morphology and osteopontin expression, thereby enhancing our ability to direct cellular architecture. The ALS shows how engineered materials can impart a higher order of control and intelligence in biological systems which open new possibilities in biomaterial design.

### Example 3: Bioadaptable woundcare bandages made from blood components for theranostic applications

### Materials and methods

### Preparation of Hb-BSA-PEI hydrogels

Stock solutions containing bovine hemoglobin (Hb; 08449 Millipore, Sigma-Aldrich), bovine serum albumin (BSA; A9647 Sigma-Aldrich), and poly(ethyleneimine) solution (PEI; 181978-Sigma-Aldrich, average Mw ~750,000) were prepared at specific concentrations. These solutions were then used to create samples with final concentrations as specified in Table 4. The preparation process involved dissolving the desired quantities of Hb, BSA, and PEI in deionized water. After thorough mixing to achieve the desired compositions (as outlined in Table 4), the samples were carefully placed in an oven at 60 °C for varying time intervals. To prevent contamination, the samples were covered with Parafilm, which consisted of three layers with small holes created by a needle.

Based on considerations related to handling stock solutions, homogeneity, mechanical properties (Figure 22), and rheological properties, we selected three compositions-HP2, BP2, and HBP1-for further investigation. The composition details and preparation methods for each are as follows:
- HP: 16.66% Hb - 4.16% PEI
- BP: 16.66% BSA - 4.16% PEI
- HBP: 8.33% Hb - 8.33% BSA - 4.16% PEI

During preparation:
- BP samples were incubated for 20 minutes without a Parafilm cover and for 1 hour with a Parafilm cover at 60 °C.
- HP and HBP samples were placed with a Parafilm cover for 40 minutes at 60 °C.

**Table 4. Different compositions of hydrogels.**

| Composition | Weight % |
|---|---|
| BP1 | 12.50 % BSA + 6.25 % PEI |
| HP1 | 12.50 % Hemoglobin + 6.25 % PEI |
| HBP1 | 8.33 % BSA + 8.33 % Hemoglobin + 4.16 % PEI |
| BP2 | 16.66 % BSA + 4.16 % PEI |
| HP2 | 16.66 % Hemoglobin + 4.16 % PEI |
| HBP2 | 6.25 % BSA + 8.31 % Hemoglobin + 5.20 % PEI |
| BP3 | 20.00 % BSA + 2.5 % PEI |
| HP3 | 20.00 % Hemoglobin + 2.5 % PEI |
| HBP3 | 10.00 % BSA + 10.00 % Hemoglobin + 2.5 % PEI |

### Spectroscopy analysis

The Shimadzu UV-VIS spectrometer (UV-2600) was used to perform UV-Vis spectroscopy on samples, with measurements taken at 1 nm intervals. Additionally, the TECAN SPARK^{®} MULTIMODE MICROPLATE READER was employed to measure fluorescence emission. The measurements were conducted at the corresponding excitation monochromator, using the following parameters:
- Excitation bandwidth: 5 nm
- Gain (manual): 100
- Number of flashes: 30
- Automatic mirror setting: 50% Mirror
- Integration time: 40 µs
- Z-Position: 20,000 µm

Furthermore, the scaffolds were imaged using an LSM 900 confocal microscope (Zeiss, GmbH, Germany).

### Mechanorheological characterizations of scaffolds

The mechanical properties of the engineered constructs were evaluated using an Instron model 5967 Universal Testing System (UK). To assess compression behavior, we applied a 500 N load using a compression rate of 0.5 mm/min. Before testing, cylindrical scaffolds (with hydrogel dimensions approximately 4.70 mm in diameter and 4-6 mm in height, as measured independently using a digital Vernier caliper) were soaked in PBS. The elastic modulus of the scaffolds was determined from the stress-strain curve within the 0.15-0.25 mm/mm strain range, and the ultimate stress was identified as the maximum stress value. Additionally, toughness was quantified by calculating the area under the stress-strain curve.

For cyclic compression testing, the hydrogels were compressed to 15-30% strain and then unloaded to 0% strain (at a constant speed rate of 0.5 mm/min for five cycles). We measured hysteresis energy, which represents energy dissipation per unit volume, by integrating the stress-strain curve between the loading and unloading hysteresis curves using MATLAB (MathWorks, Natick, MA) and OriginPro 2023 software.

Rheological assessments on Hb-BSA-PEI hydrogels were conducted using a Discovery Hybrid Rheometer HR-2 from TA Instruments. The experiments were performed with an 8 mm parallel plate geometry and a gap distance of 500 µm at 25 °C. We determined the shear storage modulus (G') and loss modulus (G") as functions of oscillation strain and angular frequency under the following conditions:
Frequency Sweep Analysis: a strain range of 0.001-1000.00% was covered at an angular frequency of 10 rad/s.
Shear-Rate Analysis: This analysis spanned 0.1-620.0 rad/s with an oscillation displacement of 0.012 rad.

To assess self-recovery ability, the amplitude oscillatory strain was varied from 1% to subsequent large strains (up to 100%) at an angular frequency of 10 rad/s, followed by a 300 s rest period after the large strain.

Additionally, the apparent viscosity of DNA-BSA hydrogels was measured at 25 °C by varying the shear rate within the range of 0.001-100,000 s⁻¹. Furthermore, dynamic rheothermo-analysis involved temperature ramps from 25 °C to 60 °C at a 10 rad/s angular frequency and 0.1% strain.

### Impedance spectroscopy and strain sensing analysis

To evaluate the conductivity of Hb-BSA-PEI scaffolds, we employed the PalmSens4 instrument. Electrochemical Impedance Spectroscopy (EIS) was performed with an AC amplitude voltage of 25 mV, covering a frequency range from 1 MHz to 0.1 Hz. The DNA-BSA hydrogels, which exhibited an average cylindrical shape (with a diameter of 4.70 mm and a height ranging from 1.50 mm to 5.00 mm, as independently measured using a digital Vernier caliper), were positioned between two stainless steel plates. By analyzing the Nyquist plot using the built-in software, we determined the hydrogel's resistance (R). The conductivity (σ) can be calculated using Equation 1: $σ = \left(1/R\right) \times \left(T/A\right)$ Equation 1.

Where:
T: Represents the thickness of the sample.
A: Denotes the area of the sample.

To demonstrate the self-healing properties of the hydrogels, an electronic circuit was designed. This circuit featured a light-emitting diode (LED) operating at a direct voltage of 3-3.2 V, with the hydrogels acting as conductors. Connecting a 9 V battery via wires completed the circuit, linking the battery, hydrogel, and the LED.

For pressure monitoring, sensors were fabricated using Hb-BSA-PEI hydrogel cylinders. These sensors, ranging in size from 3-5 mm in length and 4.7 mm in diameter, were connected to tinned copper sheets at both ends and isolated from Instron steel using an insulator. The sensors were positioned between the mechanical compression Instron (as shown in Figure 20(b)). Using the Agilent 4294A precision and PalmSens4 impedance analyzers, we measured resistance changes.

All measurements were conducted at 2 kHz using a sinusoidal AC voltage with a 0.5 V amplitude. We estimated the relative impedance (ΔR/R0), where ΔR represents the difference in resistance under different measuring scenarios compared to the initial resistance (R0).

### Results and discussion

### Engineering rheomechanic

The interaction between Hb (hemoglobin) and BSA (bovine serum albumin) with PEI (polyethyleneimine) resulted in the formation of a homogeneous viscous gel. However, to convert these soft gels into stable hydrogels, structural modifications were necessary. The key lies in denaturation induced by increasing the temperature. To analyze the property changes, both mechanical and rheological properties were investigated.

In Figure 18 (b, c), a slight increase in viscosity and a sharp rise in stress were observe with rising shear rates up to 0.01 s⁻¹. Beyond this threshold, flowability escalates significantly (by a factor of 10⁴-10⁵) under nearly constant stress. Elevated temperatures induce denaturation and strengthen the interactions, augmenting the changes of hydrogels' modulus in the order of BP > HBP > HP (as shown in Figure 18 (a)). Notably, HP hydrogels exhibit slight changes in modulus. It is hypothesized that Hb with PEI has stronger interactions compared to BSA with PEI, but further theoretical and thermochemical analysis is needed to confirm this trend.

Furthermore, the self-healing and recovery behaviour of BP hydrogels was verified through a process of cutting and re-assembling. When subjected to cycled On-Off-On states of an LED lamp (as depicted in Figure 20. (a)), these hydrogels consistently maintain uniformity. Also, rheological studies demonstrate that these hydrogels possess self-recovery capabilities, achieving strains exceeding 100% (Figure 18. (f)). Their self-healing capacity not only enhances reusability but also significantly broadens the potential applications of scaffolds within dynamic manipulation of matrix-cell mechanotransduction signalling.

An analysis of the modulus of hydrogels at different angular frequencies revealed valuable trends. While HP samples exhibit nearly stable modulus, especially at low angular frequencies, both BP and HBP hydrogels exhibit an increase in modulus at 0.1 rad/s. This observation provides further evidence of the robust nature of Hb-PEl interactions compared to BSA-PEI. Additionally, under the specific preparation conditions (60°C with a specified incubation time), the order of shear modulus follows the sequence BP > HP > HBP (as shown in Figure. 18(d)).

Examining the shear modulus in relation to scanning shear strain, the breaking point of HBP occurred at higher strains compared to the HP and BP hydrogels. This phenomenon suggests that the dual networks of soft (BSA-PEI) and hard interactions (Hb-PEI) play a crucial role in determining the material strength towards shear strain (Figure 18. (e)).

Mechanical tests were conducted on BP-HP-HBP hydrogels, recognizing the pivotal role of mechanical cues in cell adhesion, migration, and differentiation. As illustrated in Figure 19. (a-f), the hydrogel systems exhibited a diverse range of mechanical properties.

Specifically, the Young's modulus of the HP hydrogel reached its zenith at approximately 180 kPa. In stark contrast, the BP hydrogel displayed the lowest Young's modulus, hovering around a mere 10 kPa. The HBP Young's modulus fell in between, more closely aligned with that of the BP hydrogel (approximately 34 kPa). Ultimate stress, stress at 50% strain, and toughness mirrored the trends observed in the Young's modulus, while breaking points exhibited an opposing pattern.

These findings underscore the influence of higher Hb content, rendering the hydrogels stiffer and more resilient. Conversely, increasing BSA content imparts greater flexibility, particularly in terms of compression breaking points. Notably, the compression studies revealed that Hb-PEI interactions contribute to rigidity, whereas BSA-PEI interactions bestow flexibility upon both HBP and BP hydrogels.

Furthermore, cyclic compression stress (depicted in Figure 19, panels g and h) facilitated hydrogel recovery, suggesting their potential utility as pressure sensors. Additionally, by modulating the load magnitude on tissue, these hydrogels could play a role in wound pressure modulation and recording during the proliferation and remodeling phases. Dissipation energies from cyclic compression experiments revealed that the BP sample exhibited the lowest dissipation, while the HP sample demonstrated the highest dissipation, aligning with the trends mentioned above.

Multi-channel physicochemical sensing of Hb and hydrogels: conductivity to fluorescence

In Figure 20. (b-i), we present a comprehensive demonstration of the conductivity and pressure sensitivity of BP, HP, and HBP hydrogels. These materials play a crucial role in cell adhesion, migration, and differentiation due to their mechanical cues.

Firstly, let's dissect the gauge factor (GF) - a key parameter in their sensitivity towards compression strain. Remarkably, the HP sample boasts the highest GF, hovering around 2.5 (as depicted in Figure 20. (f)). This superiority is presumably linked to the hydrogel's mechanical and rheological behaviors, characterized by robust and stable interactions. Notably, when fabricated in deionized water, both HP and HBP hydrogels exhibit greater conductivity than BP. However, the conductivity of HBP hydrogels experiences a more significant boost when prepared in PBS at pH 7.4 (Figure 20 (d)) compared to BP and HP. These trends underscore the impact of structural changes, attached ions, and dual interaction networks on their overall conductivity, as evidenced by the dynamic shifts in rheomechanic behaviors.

Considering the versatility of hydrogel fabrication and the critical role of pressure in wound healing, we propose exploring their potential as pressure sensors. Our hydrogels exhibit nonlinear and linear impedance changes (with a linearity trend of GF: HBP > BP > HP). Notably, the HP hydrogels displayed heightened nonlinearity, especially during the initial cycle - a phenomenon likely tied to their higher dissipation energy compared to BP and HBP hydrogels. These observations hold promise for pressure sensitivity applications.

Furthermore, the hydrogels' conductivity responds dynamically to cyclic compressions across different strain ratios. Impressively, they maintain nearly stable responses even during high cycles, positioning them as strong contenders for tracking pressure fluctuations/manipulations (Figure 20. (i)).

To monitor wound parameters, including hydrogen peroxide (H₂O₂), pH, and temperature-each closely linked to the various phases/states of healing-UV-Vis and fluorescence spectroscopy were employed to track hemoglobin (Hb) responses (as shown in Figure 21).

In Figure 21. (a-c), we observed that increasing H2O2 concentration caused a decrease and rightward shift in the peak centered around 406 nm, along with increasing and broadening of absorbance peaks beyond 500 nm. Additionally, fluorescence emission exhibited distinct peaks in the red and near-infrared (NIR) regions (zones I and II). This valuable information can be leveraged for real-time monitoring of oxidative stress.

Furthermore, Hb demonstrated sensitivity to pH and temperature in both the visible and NIR regions (zones I and II). These properties enable tracking of spatiotemporal multi-stimuli signals based on their kinetic responses and displacements/amplitude changes. Notably, when compared to the control, different trends and shifts were observed by specifying filter variant equations (Figure 21(d-i)).

To translate the behavior of Hb into scaffolds, fluorescence imaging of hydrogels in the green and red channels was acquired as an imaging probe (Figure 21 (j)). Additionally, ongoing investigations involve Raman spectroscopy to identify more distinctive trends.

### Conclusion

Wound care has evolved significantly, thanks to wearable sensors integrated into dressing patches. These sensors, covering various technologies, monitor wound parameters such as physical, physiological, mechanical, and biochemical cues. However, challenges persist which include power sources, pollution, and miniaturization. Active biomaterials as a promising solution can combines healing and remote optical and electrical sensing capabilities. By integrating hemo-proteins, bovine serum albumin (BSA), and poly(ethylene imine) (PEI), we've developed a scalable Active Scaffold (AS) called Hb-BSA-PEI (HBP). This novel system bridges the gap between research and practical wound management which promise noninvasiveness, minimal side effects, and operational simplicity by eliminating the need for extra auxiliary wearable sensors and their associated complexity.

## Claims

1. A composition comprising at least two materials of materials A, B, and C; wherein
A is selected from the group consisting of nucleic acid, peptide nucleic acid, single-stranded deoxyribonucleic acid (ssDNA), double-stranded deoxyribonucleic acid (dsDNA), peptide nucleic acid (PNA), and ribonucleic acid (RNA);
B is selected from the group consisting of metalloprotein, ferritin, serum albumin such as bovine serum albumin (BSA), histone, lysozyme, fibrinogen, fibronectin, silk protein, transferrin, peptides comprising basic amino acids, chlorophyll-protein complexes, peridinin-chlorophyll-protein complex (PCP), chlorophyll a/b binding proteins, Rubisco (Ribulose-1,5-bisphosphate carboxylase/oxygenase), ATP Synthase, NADP+ Reductase, cytochrome, ferredoxin, plastocyanin (PC), photosystem proteins, ribosomal proteins, mitochondrial proteins, NADH Dehydrogenase, humanin, and MOTS-c; and
C is selected from the group consisting of collagen, aminated collagen, gelatin, aminated gelatin, poly-l-lysine, poly-histidine, poly-arginine, chitosan, aminated pectin, aminated polythylene glycol, aminated chitosan, poly(N-isopropylacryalamide (NIPAM), Polyaniline (PANI), amidine doped PEDOT:PSS, polyethylenimine (PEI), polyacrylamide, and peptide nucleic acid (PNA);
**characterized in that**
the composition comprises a complex comprising at least two of the materials of the at least two materials.

2. A composition according to claim 1, wherein the complex is a condensate, an aggregate, a cluster, a supramolecular assembly, a coordination compound or a network; such as a condensate or an aggregate.

3. A composition according to any one of claim 1 or 2, wherein the at least two materials are in a denatured state.

4. A composition according to any one of claims 1 to 3, wherein the composition has a pH of at least 10.

5. A composition according to any one of the preceding claims, wherein the composition is in the form of a hydrogel, an aggregate, a condensate, a paste, a nanoparticle, a microparticle or a film.

6. A composition according to any one of the preceding claims, wherein the metalloprotein is selected from the group consisting of hemoglobin, myoglobin, cytochrome C, carbonic anhydrase, and superoxide dismutase.

7. A composition according to any one of the preceding claims, wherein the peptide comprising basic amino acid comprises amino acids selected from the group consisting of Lysine, Arginine, and Histidine.

8. A composition according to any one of claims 1 to 7, wherein the composition comprises dsDNA and serum albumin, such as BSA.

9. A composition acording to any one of claims 1 to 7, wherein the composition comprises dsDNA and hemoglobin.

10. A composition according to any one of the preceding claims, wherein the composition comprises material A in the range of 3 to 30% w/v, and material B in the range of 3 to 30% w/v,
such as material A in the range of 3.5 to 25% w/v and material B in the range of 4 to 25% w/v, or
material A in the range of 4.5 to 20 % w/v and B in the range of 6 to 20% w/v.

11. A composition according to claim 1, wherein the composition comprises PEI and serum albumin such as bovine serum albumin (BSA), and/or hemoglobin.

12. A composition according to claim 11, wherein the composition comprises PEI in the range of 1 to 10% w/v, serum albumin in the range of 3 to 30% w/v and/or hemoglobin in the range of 4 to 30% w/v, such as
PEI in the range of 2% w/v, serum albumin in the range of 4 to 25% w/v and/or hemoglobin in the range of 5 to 25% w/v.

13. A composition according to any one of the preceding claims, wherein the complex is of uniform size distrubution, such as a size distribution of from about 10 nm to about 800 nm, such as from about 10 nm to about 700 nm, such as from about 10 nm to about 70 nm, such as from about 15 nm to about 60 nm, or such as from about 200 nm to about 700 nm.

14. A composition according to any one of the preceding claims, further comprising a filler selected from the group consisting of porphyrin and a porphyrin derivative, graphene, chlorophyll, tannic acid, carbon nanotube, nanozymes, metal nanoparticle, Mxene's, metal-organic framworks, metal and metal-free chromophores, covalent organic framework, metal-organic cages, nanosilicate, Laponite, Hallosite, Sumecton, Montmorillonite, and calcium orthophosphate family such as Hydroxyapatite.

15. A composition according to any one of the preceding claims, further comprising hemin, such as in the range of 0.01 - 1% w/v hemin, or in the range of 0.05 - 5% w/v hemin.

16. A method of denaturing a composition according to any one of claims 1 to 15, the method comprising the steps of:
- providing a material A according to claim 1 and adding a base, such as a NaOH solution, to reach a pH level in the range of 10 to 14,
- adding a material B according to claim 1,
- curing the composition at a temperature in the range of 40 to 80°C, such as in the range of 50 to 70°C.

17. A method of denaturing a composition according to any one of claims 1 to 15, comprising the steps of:
- providing a composition according to any one of claims 1 to 14,
- exposing the composition to UV light in the range of 100 to 400 nm, such as in the range of 150 - 250 nm, or 250 - 350 nm, or 240 nm, or 380 nm, for at least 5 minutes, such as for at least 15 minutes or at least 30 minutes.

18. Use of a composition according to any one of claims 1 to 15 in tissue engineering, in stem cell differentiation, in a flexible electronic device, in a biosensor or in a woundcare patch.
